(19) **Europäisches Patentamt European Patent Office Office européen des brevets**

(11) **EP 4 663 767 A1**

(12) **EUROPEAN PATENT APPLICATION**
published in accordance with Art. 153(4) EPC

(43) Date of publication:
17.12.2025 Bulletin 2025/51

(21) Application number: 24753464.7

(22) Date of filing: 10.02.2024

(51) International Patent Classification (IPC):
$C12P\ 21/06^{(2006.01)}$    $A23F\ 3/16^{(2006.01)}$
$A23F\ 5/24^{(2006.01)}$    $A23J\ 1/00^{(2006.01)}$
$A23J\ 3/14^{(2006.01)}$    $A23L\ 5/00^{(2016.01)}$
$A23L\ 19/00^{(2016.01)}$    $C07K\ 1/34^{(2006.01)}$
$C12M\ 1/00^{(2006.01)}$    $C12P\ 1/00^{(2006.01)}$

(52) Cooperative Patent Classification (CPC):
A23F 3/16; A23F 5/24; A23J 1/00; A23J 3/14;
A23L 5/00; A23L 5/30; A23L 19/00; C07K 1/34;
C12M 1/00; C12M 1/33; C12P 1/00; C12P 21/06

(86) International application number:
PCT/JP2024/004689

(87) International publication number:
WO 2024/167021 (15.08.2024 Gazette 2024/33)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 10.02.2023 JP 2023019517
20.09.2023 JP 2023152830

(71) Applicants:
• **NATIONAL UNIVERSITY CORPORATION SHIZUOKA UNIVERSITY**
**Shizuoka-shi, Shizuoka 422-8529 (JP)**

• **S-BRIDGES CORPORATION**
**Hamamatsu-shi Shizuoka 432-8003 (JP)**

(72) Inventors:
• **SANO Yoshihiko**
**Hamamatsu-shi Shizuoka 432-8561 (JP)**
• **NAGATO Takashi**
**Hamamatsu-shi Shizuoka 432-8003 (JP)**
• **AIBA Shinya**
**Hamamatsu-shi Shizuoka 432-8003 (JP)**
• **TERADA Hitoshi**
**Hamamatsu-shi Shizuoka 432-8003 (JP)**

(74) Representative: **Schön, Christoph**
**Dr. Schön, Neymeyr & Partner mbB**
**Bavariaring 26**
**80336 München (DE)**

(54) **METHOD FOR PRODUCING EXTRACT OF BIOLOGICAL TISSUE AND METHOD AND DEVICE FOR PROCESSING BIOLOGICAL TISSUE**

(57) An object is to provide a method for producing an extract of a biological tissue and an apparatus for processing a biological tissue, with improved extraction efficiency. Provided are a method for producing an extract of a biological tissue, including a step of decomposing extracellular matrix of the biological tissue with an enzyme; and a step of separating cells of the biological tissue and/or an extract of the biological tissue from the extracellular matrix by subjecting the biological tissue to separation, and a biological tissue processing apparatus for processing a mixed solution obtained by mixing a biological tissue and a solution containing an enzyme, including a housing container in which the mixed solution is housed and decomposition is conducted for decomposing extracellular matrix of the biological tissue in the mixed solution with the enzyme; and a separation device for separating cells of the biological tissue and/or an extract of the biological tissue from the extracellular matrix by applying a force to the biological tissue in the mixed solution.

EP 4 663 767 A1

|  | 100 times | 200 times | 500 times |
|---|---|---|---|
| Tea-leaf slurry of Example 1 | | | |
| Used-tea-leaf slurry of Example 2 | | | |
| Tea leaves of Comparative Example 1 | | | |

FIG. 12

## Description

TECHNICAL FIELD

The present disclosure relates to a method for producing an extract of a biological tissue, and an apparatus and a method for processing a biological tissue.

BACKGROUND ART

Most of proteins contained in tea leaves are slightly soluble or insoluble in water, are hardly extracted during production of tea beverages, and are difficult to ingest from tea beverages produced using an ordinary method. According to the Standard Tables of Food Composition in Japan, 2020 (Eighth Revised Edition), tea leaves, which are used as raw materials of green tea beverages, black tea beverages, and the like, contain proteins at about 20 g/100 g to 30 g/100 g. Meanwhile, tea leaf infusions such as green tea beverages and black tea beverages produced through extraction of these tea leaves serving as raw materials contain proteins only at about 0 g/100 g to 1.3 g/100 g, and a large quantity of proteins remains in tea leaves (used tea leaves) after being subjected to extraction for production of tea beverages.

As in the case of tea leaves, coffee beans, which are used as raw materials of coffee beverages, also contain proteins, but coffee beverages, which are coffee bean infusions, contain substantially no proteins, and a large quantity of proteins remains in used coffee beans after being subjected to extraction for production of coffee beverages.

In most cases, these used tea leaves and used coffee beans are discarded without being subjected to further processing operations, and are not effectively utilized.

As a method for increasing efficiency of extracting components contained in tea leaves, methods for preparing a protein decomposition product by allowing an enzyme to act on an extract obtained by adding an alkaline solution to tea leaves have been proposed (Patent Literature 1 and Patent Literature 2). The references above disclose that the methods can be used to extract water-insoluble proteins contained in tea leaves, and it is also possible to reduce production cost and to effectively use unused resources because used tea leaves resulting from the extraction step of the tea beverage production process can be utilized.

Also, as a means for effectively using components contained in food such as the above-mentioned tea leaves, used tea leaves, coffee beans, and used coffee beans, food micronization has been conducted over the years as typified by powdered tea.

Citation List

Patent Literature

Patent Literature 1: Japanese Patent No. 5645994
Patent Literature 2: Japanese Patent No. 5646035

SUMMARY OF INVENTION

Technical Problem

Food micronization has a problem of a high yield reduction rate because heat generated in the drying process and the pulverization process alters the quality of the food and, for example, causes the pulverized food to be adsorbed to an apparatus and the like or to fly in the air. In addition, from the viewpoint of an improvement in a mouth-feel of food, a technique for micronizing food to powder having a size of about 100 $\mu$m to 150 $\mu$m is required. Thus, the size of micronized food is much larger than the diameter of a plant cell, 6 $\mu$m to 25 $\mu$m, which means that the food micronization focuses on only an improvement in a mouth-feel, and the disruption of the plant cell wall is not taken into consideration. The plant cell wall is composed of cellulose, hemicellulose, lignin, and the like, and cannot be decomposed by human digestive enzymes. Accordingly, when the plant cell wall is not disrupted, the efficiency of food nourishment absorption may decrease in a human body. Furthermore, in the micronization technique, it is difficult to selectively separate nutrients because food itself is micronized. Patent Literature 1 and Patent Literature 2 disclose methods for increasing efficiency of extracting components contained in tea leaves, but even these methods cannot sufficiently increase the efficiency of extracting components contained in tea leaves.

That is to say, conventional methods cannot be used to sufficiently increase the efficiency of extracting components contained in a biological tissue.

Therefore, it is a first object of the present disclosure to provide a method for producing an extract of a biological tissue and an apparatus for processing a biological tissue, with improved extraction efficiency. In addition, it is a second object of the present disclosure to provide an apparatus and a method for processing a biological tissue with improved separation or extraction efficiency.

Solution to Problem

To achieve the first object above, a production method of the present disclosure is a method for producing an extract of a biological tissue, including:

> a step of decomposing extracellular matrix of the biological tissue with an enzyme; and
> a step of separating cells of the biological tissue and/or an extract of the biological tissue from the extracellular matrix by subjecting the biological tissue to separation.

An extraction method of the present disclosure is a method for extracting an extract of a biological tissue, including:

> a step of decomposing extracellular matrix of the biological tissue with an enzyme; and
> a step of separating cells of the biological tissue and/or an extract of the biological tissue from the extracellular matrix by subjecting the biological tissue to separation.

A first biological tissue processing apparatus of the present disclosure is an apparatus for processing a mixed solution obtained by mixing a biological tissue and a solution containing an enzyme, including:

> a housing container in which the mixed solution is housed and decomposition is conducted for decomposing extracellular matrix of the biological tissue in the mixed solution with the enzyme; and
> a separation device for separating cells of the biological tissue and/or an extract of the biological tissue from the extracellular matrix by applying a force to the biological tissue in the mixed solution.

To achieve the second object above, a second biological tissue processing apparatus of the present disclosure is a biological tissue processing apparatus, including:

> a first housing container that is capable of housing a mixed solution obtained by mixing a biological tissue and a solution containing an enzyme and is configured to be capable of decomposing extracellular matrix of the biological tissue in the mixed solution with the enzyme;
> a separation device that is configured to be capable of applying a force to the biological tissue in the mixed solution and separating cells of the biological tissue and/or an extract of the biological tissue from the extracellular matrix;
> a first measurement device that is configured to be capable of measuring a state of the mixed solution over time; and
> a first control device that is configured to be capable of controlling separation of a decomposition product of the biological tissue by the separation device based on the state of the mixed solution obtained in the measurement.

A third biological tissue processing apparatus of the present disclosure is a biological tissue processing apparatus including:

> a first housing container that is capable of housing a mixed solution obtained by mixing a biological tissue and a solution containing an enzyme and is configured to be capable of decomposing extracellular matrix of the biological tissue in the mixed solution with the enzyme;
> a separation device that is configured to be capable of applying a force to the biological tissue in the mixed solution and separating cells of the biological tissue and/or an extract of the biological tissue from the extracellular matrix;
> a second measurement device that is configured to be capable of measuring a state of the separation device over time; and
> a second control device that is configured to be capable of controlling separation of a decomposition product of the biological tissue by the separation device based on the state of the separation device obtained in the measurement.

A fourth biological tissue processing apparatus of the present disclosure is a biological tissue processing apparatus, including:

> a first housing container that is capable of housing a mixed solution obtained by mixing a biological tissue and a solution containing an enzyme and is configured to be capable of decomposing extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a channel that is configured to allow the mixed solution in the first housing container to be circulated therethrough;

a liquid feeding device that is configured to be capable of feeding the mixed solution in the first housing container via the channel;

a third measurement device that is configured to be capable of measuring a state of the mixed solution in the channel over time; and

a third control device that is configured to be capable of controlling liquid feeding by the liquid feeding device based on the state of the mixed solution in the channel obtained in the measurement.

A fifth biological tissue processing apparatus of the present disclosure is a biological tissue processing apparatus, including:

a first housing container that is capable of housing a mixed solution obtained by mixing a biological tissue and a solution containing an enzyme and is configured to be capable of decomposing extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a solid-liquid separation device that is configured to be capable of conducting solid-liquid separation of the extract;

a second housing container that is configured to be capable of housing a solid fraction obtained through the solid-liquid separation of the extract;

a fourth measurement device that is configured to be capable of measuring a state of the solid fraction in the second housing container over time; and

a fourth control device that is configured to be capable of controlling the solid-liquid separation of the extract by the solid-liquid separation device based on the state of the solid fraction obtained in the measurement.

A sixth biological tissue processing apparatus of the present disclosure is a biological tissue processing apparatus, including:

a first housing container that is capable of housing a mixed solution obtained by mixing a biological tissue and a solution containing an enzyme and is configured to be capable of decomposing extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a solid-liquid separation device that is configured to be capable of conducting solid-liquid separation of the extract;

a second housing container that is configured to be capable of housing a solid fraction obtained through the solid-liquid separation of the extract;

a channel that is configured to allow the extract to be circulated therethrough via the solid-liquid separation device;

a liquid feeding device that is configured to be capable of feeding the extract via the channel;

a fourth measurement device that is configured to be capable of measuring a state of the solid fraction in the second housing container over time; and

a fifth control device that is configured to be capable of controlling supply of the extract to the solid-liquid separation device via the channel based on the state of the solid fraction obtained in the measurement.

A processing method of the present disclosure is a method for processing a biological tissue, including:

a decomposition step of decomposing extracellular matrix of a biological tissue in a mixed solution containing the biological tissue and an enzyme;

a measurement step of measuring a state of the mixed solution containing the biological tissue and the enzyme over time in the decomposition step; and

a separation step of separating cells of the biological tissue and/or an extract of the biological tissue from a decomposition product of the biological tissue,

wherein the separation of the decomposition product of the biological tissue is conducted based on the state of the mixed solution obtained in the measurement step.

Advantageous Effects of Invention

With the present disclosure, it is possible to provide a method for producing an extract of a biological tissue and an apparatus for processing a biological tissue, with improved extraction efficiency. In addition, with the present disclosure it is possible to provide an apparatus and a method for processing a biological tissue with improved separation or extraction efficiency.

Brief Description of Drawings

[FIGs. 1A-1B] FIG. 1A is a schematic diagram illustrating a biological tissue processing apparatus 1A of an embodiment, and FIG. 1B is a schematic diagram illustrating the biological tissue processing apparatus 1A of the embodiment provided with a biological tissue supply unit 11 and the like.
[FIG. 2] FIG. 2 is a schematic diagram illustrating a biological tissue processing apparatus 1B of an embodiment.
[FIG. 3] FIG. 3 is a schematic diagram illustrating a biological tissue processing apparatus 1C of an embodiment.
[FIGs. 4A-4B] FIG. 4A is a schematic diagram illustrating a biological tissue processing apparatus 1D of an embodiment, and FIG. 4B is a schematic diagram illustrating a biological tissue processing apparatus 1E of an embodiment.
[FIGs. 5A-5B] FIG. 5A is a schematic diagram illustrating a hood member 4f as viewed from a bottom surface side, and FIG. 5B is a schematic diagram illustrating a state in which an agitation blade 4c is disposed inside the hood member 4f, as viewed from the bottom surface side.
[FIGs. 6A-6B] FIGs. 6A-6B show diagrams illustrating the agitation blade 4c alone.
[FIG. 7] FIG. 7 is a schematic diagram illustrating a state in which the agitation blade 4c disposed inside the hood member 4f is disposed inside a housing container 2, 4T and is agitating a mixed solution L.
[FIG. 8] FIG. 8 is a schematic diagram illustrating the biological tissue processing apparatus 1C of the embodiment provided with a plurality of separation tanks 4T.
[FIG. 9] FIG. 9 illustrates an example of a processing flow of a production method of the present disclosure.
[FIG. 10] FIG. 10 illustrates an example of a processing flow of a production method of the present disclosure.
[FIG. 11] FIG. 11 shows SEM images of tea leaves and used tea leaves before being subjected to decomposition and separation.
[FIG. 12] FIG. 12 shows SEM images of tea-leaf slurry of Example 1, used-tea-leaf slurry of Example 2, and tea leaves of Comparative Example 1.
[FIG. 13] FIG. 13 shows SEM images of venous cellulose fibers in the tea-leaf slurry of Example 1.
[FIGs. 14A-14B] FIGs. 14A-14B show immersed tea leaves before (A) and after (B) being homogenized under agitation in Example 3.
[FIGs. 15A-15F] FIGs. 15A-15F show a process of solid-liquid separation in Example 3.
[FIG. 16] FIG. 16 shows immersed tea leaves in Comparative Example 2.
[FIG. 17] FIG. 17 shows the results of the weights of the wet tea-leaf slurries obtained in Example 3.
[FIG. 18] FIG. 18 shows the results of the protein amounts obtained in Example 3.
[FIG. 19] FIG. 19 shows the results of the concentrations of the components obtained in Example 3 and Comparative Example 2.
[FIG. 20] FIG. 20 shows immersed tea leaves after being homogenized under agitation in Example 4.
[FIGs. 21A-21F] FIGs. 21A-21F show a process of solid-liquid separation in Example 4.
[FIG. 22] FIG. 22 shows immersed tea leaves in Comparative Example 3.
[FIG. 23] FIG. 23 shows the results of the weights of the wet tea-leaf slurries obtained in Example 4.
[FIG. 24] FIG. 24 shows the results of the protein amounts obtained in Example 4.
[FIG. 25] FIG. 25 shows the results of the concentrations of the components obtained in Example 4 and Comparative Example 3.
[FIG. 26] FIG. 26 shows immersed coffee beans after being homogenized under agitation in Example 5.
[FIGs. 27A-27D] FIGs. 27A-27D show a process of solid-liquid separation in Example 5.
[FIG. 28] FIG. 28 shows immersed coffee beans in Comparative Example 4.
[FIG. 29] FIG. 29 shows the results of the weights of the wet coffee-bean slurries obtained in Example 5.
[FIG. 30] FIG. 30 shows the results of the protein amounts obtained in Example 5.
[FIGs. 31A-31B] FIGs. 31A-31B show immersed strawberry leaves before (A) and after (B) being homogenized under agitation in Example 6.
[FIGs. 32A-32F] FIGs. 32A-32F show a process of solid-liquid separation in Example 6.
[FIG. 33] FIG. 33 shows the results of the weights of the wet strawberry-leaf slurries obtained in Example 6.

[FIG. 34] FIG. 34 shows the results of the protein amounts obtained in Example 6.

[FIGs. 35A-35B] FIGs. 35A-35B show immersed tomato leaves before (A) and after (B) being homogenized under agitation in Example 7.

[FIGs. 36A-36F] FIGs. 36A-36F show a process of solid-liquid separation in Example 7.

[FIG. 37] FIG. 37 shows the results of the weights of the wet tomato-leaf slurries obtained in Example 7.

[FIG. 38] FIG. 38 shows the results of the protein amounts obtained in Example 7.

[FIG. 39] FIG. 39 is a block diagram schematically illustrating a processing apparatus 100 of the present disclosure.

[FIG. 40] FIG. 40 is a flowchart schematically illustrating an example of a processing method conducted in the processing apparatus 100 of the present disclosure.

[FIG. 41] FIG. 41 is a block diagram schematically illustrating a processing apparatus 101 of the present disclosure.

[FIG. 42] FIG. 42 is a flowchart schematically illustrating an example of a processing method conducted in the processing apparatus 101 of the present disclosure.

[FIG. 43] FIG. 43 is a block diagram schematically illustrating a processing apparatus 102 of the present disclosure.

[FIG. 44] FIG. 44 is a flowchart schematically illustrating an example of a processing method conducted in the processing apparatus 102 of the present disclosure.

[FIG. 45] FIG. 45 is a block diagram schematically illustrating a processing apparatus 103 of the present disclosure.

[FIG. 46] FIG. 46 is a flowchart schematically illustrating an example of a processing method conducted in the processing apparatus 103 of the present disclosure.

[FIG. 47] FIG. 47 is a block diagram schematically illustrating a processing apparatus 104 of the present disclosure.

[FIG. 48] FIG. 48 is a flowchart schematically illustrating an example of a processing method conducted in the processing apparatus 104 of the present disclosure.

DESCRIPTION OF EMBODIMENTS

All of the technical terms and the scientific terms as used herein are used in the same senses as those generally understood by a person skilled in the art, unless otherwise defined herein. All patents, applications, and other publications and information that are referred to herein are fully incorporated herein by reference.

[Definitions]

The term "biological tissue" as used herein means an aggregate of cells of any one type. Examples of the biological tissue include an animal tissue, a plant tissue, and the like. The biological tissue may be a processed product obtained by processing an aggregate of cells of any one type.

The term "animal tissue" as used herein means a biological tissue of any animal. Examples of the animal tissue include biological tissues of vertebrates (e.g., mammals, reptiles, birds, amphibians, and fish) and the like. Examples of the animal tissue include an epithelial tissue, a connective tissue, a muscular tissue, a nervous tissue, and the like. Specific examples thereof include a fibrillar connective tissue, an adipose tissue, a cartilaginous tissue, and the like.

The term "plant tissue" as used herein means a biological tissue of any plant. Examples of the plant tissue include biological tissues of grass, trees, and the like. Examples of the plant tissue include an epidermal tissue, a conductive tissue, a mechanical tissue, a parenchyma, and the like. Specifically, the plant tissue may be, for example, a leaf, a stem, a flower, a seed, a root, a trunk, a skin (e.g., a bark, an epidermis, and a bast), sap, and/or a fruit, and a processed product thereof. Specific examples of the plant tissue include tissues that abound in nutritional components such as proteins and amino acids. Examples of the plant tissue include tissues of a tea plant (tea), a coffee plant (coffee), moringa, a Brussels sprout, a cabbage, a tomato, bamboo, and the like. The tissue of the tea plant may be, for example, a tea leaf, a used tea leaf, or the like. Also, the plant tissue may be, for example, a cellulose fiber.

The term "cellulose fiber" as used herein means a cellulose fiber derived from a plant tissue. Examples of the cellulose fiber include a cellulose fiber contained in a leaf vein (i.e., a venous cellulose fiber), a cellulose fiber contained in a mesophyll, and a cellulose fiber contained in a cell wall. The cellulose fiber is not limited to a fiber composed of cellulose alone, and may contain hemicellulose, lignin, and other fibers.

The term "processed product" as used herein means an aggregate of cells of any one type that has been subjected to any processing operation. Examples of the processed product include aggregates of cells of any one type that have been subjected to a physical processing operation (e.g., cutting, shearing, or homogenization), a chemical processing operation (e.g., a redox reaction, a hydrolysis reaction, or an enzyme reaction), a high/low-temperature processing operation (e.g., heating, refrigeration, or freezing), or the like.

The term "extracellular matrix" as used herein means a structure that exists outside a cell. The extracellular matrix of the above-mentioned animal may include, for example, components such as collagen, proteoglycan, hyaluronic acid, fibronectin, laminin, tenascin, elastin, and chitin. The extracellular matrix of the above-mentioned plant may include, for example, a cell wall (containing cellulose, hemicellulose, lignin, pectin, cuticle, etc.), and the like.

The term "enzyme" as used herein means any enzyme capable of decomposing a target component. When the biological tissue is derived from an animal, examples of the enzyme include enzymes that decompose the components of the extracellular matrix, such as collagen, proteoglycan, hyaluronic acid, fibronectin, laminin, tenascin, elastin, and chitin. When the biological tissue is derived from a plant, examples of the enzyme include enzymes that decompose a cell wall (containing cellulose, hemicellulose, lignin, pectin, cuticle, etc.) and the like. The enzyme may be, for example, a protease, a cellulase, a hemicellulase, and/or pectinase.

The term "microbial culture solution" as used herein means a culture solution obtained by culturing microorganisms that express a target enzyme. Examples of the microbial culture solution include a culture solution in which the microorganisms proliferate to an extent that they contribute to decomposition of a target component; a culture solution in which an enzyme secreted into a culture solution by the microorganisms accumulates to an extent that the enzyme contributes to the decomposition; and the like. In the latter case, the microbial culture solution may or may not contain the microorganisms. The microorganisms are not particularly limited as long as, for example, they express the target enzyme. There is no particular limitation on the culture solution as long as the microorganisms can be cultured therein.

The term "cell" as used herein means any cell derived from the biological tissue. For example, one or two or more cells may be used. One type of cell or a plurality of types of cells may be used. When a plurality of cells or a plurality of types of cells are used, the cells may be separate from one another or form an aggregate.

The term "extraction" as used herein means removal of a certain component from a target, and/or a state in which a certain component is removed from a target. The "extraction" can be achieved by, for example, obtaining at least one extraction process. In this specification, a substance containing a component removed from the target is referred to as an "extract". In addition, a solution containing a component removed from the target is referred to as an "extract solution". The extract or extract solution may contain a cellular component of the biological tissue. The cellular component may be, for example, a low-molecular-weight compound, a nucleic acid, an amino acid, a peptide, a protein, and/or extracellular matrix. When the extract or extract solution contains extracellular matrix such as a cell wall, the extract or extract solution may contain, for example, cellulose microfibers and/or cellulose nanofibers. The extract may be, for example, cellulose fibers. The cellulose fibers include cellulose microfibers, cellulose nanofibers, and/or hemicellulose. The cellulose microfibers, cellulose nanofibers and/or hemicellulose can be isolated by, for example, subjecting the cellulose fibers to delignification or the like. These substances can be independently trading targets as various materials. Also, the cellulose fibers themselves can be trading targets. The cellulose fibers may be cellulose fibers (i.e., venous cellulose fibers) contained in a leaf vein of any plant. Also, the cellulose fibers may be cellulose fibers contained in cellulose fibers contained in a mesophyll or cellulose fibers contained in a cell wall.

The term "purification" as used herein means identification and separation of a substance, collection of a substance from components in a natural state, a state in which a substance is identified and separated, and/or a state in which a substance is collected from components in a natural state. The "purification" can be achieved by, for example, obtaining at least one purification process. The purification can also be referred to as "isolation".

The term "low-molecular-weight compound" means a compound with a low molecular weight (lower than 900 daltons). Examples of the low-molecular-weight compound include an amino acid, a lipid, a sugar, a fatty acid, and the like. Specifically, the low-molecular-weight compound may be, for example, catechin or the like.

The term "peptide" as used herein means a polymer composed of unmodified amino acids (natural amino acids), modified amino acids, and/or artificial amino acids.

The term "protein" as used herein means a polymer composed of unmodified amino acids (natural amino acids), modified amino acids, and/or artificial amino acids. The protein is, for example, a peptide composed of 10 or more amino acids.

Although the following describes examples of the present disclosure, the present disclosure is not limited to the following examples and the like, and can be implemented with any modifications. Also, each description in the present disclosure and embodiments can be applied to other descriptions unless otherwise stated. Note that the expression "to" as used herein means that the numerical or physical values before and after "to" are included. In addition, the expression "A and/or B" as used herein encompasses "only A", "only B", and "both A and B".

[Method for Producing Extract of Biological Tissue]

An aspect of the present disclosure provides a method for producing an extract of a biological tissue with improved extraction efficiency. The production method of the present disclosure is a method for producing an extract of a biological tissue, and includes a step (decomposition step) of decomposing extracellular matrix of the biological tissue with an enzyme, and a step (separation step) of separating cells of the biological tissue and/or an extract of the biological tissue from the extracellular matrix by subjecting the biological tissue to separation. The production method of the present disclosure includes the decomposition step as well as the separation step, thus making it possible to effectively separate the extracellular matrix of the biological tissue and other components (e.g., cells or cellular components) of the biological tissue from each other. Accordingly, with the production method of the present disclosure, it is possible to improve, for example, the efficiency of extracting an extract of the biological tissue. The production method of the present disclosure can also be referred to as, for example, a "method for extracting an extract of a biological tissue".

(Decomposition Step)

The decomposition step is a step of decomposing extracellular matrix of the biological tissue with an enzyme. Accordingly, the decomposition step can also be referred to as, for example, an "enzymatic processing step". Since the extracellular matrix of the biological tissue is decomposed in the decomposition step, the extracellular matrix of the biological tissue and other components (e.g., cells or cellular components) of the biological tissue can be effectively separated from each other in the separation step, which will be described below.

The decomposition step can be conducted by, for example, bringing the biological tissue into contact with the enzyme. Specifically, in the decomposition step, for example, the biological tissue and an enzyme solution containing the enzyme are mixed, and thereby a reaction solution (mixed solution) is prepared. Next, in the decomposition step, for example, the extracellular matrix of the biological tissue is decomposed with the enzyme in the reaction solution to an extent that the extracellular matrix of the biological tissue and other components of the biological tissue can be separated from each other in the separation step, which will be described below. In the decomposition step, for example, the extracellular matrix of the biological tissue is decomposed with the enzyme while the reaction solution is being agitated.

In the decomposition step, the enzyme can be selected as appropriate in accordance with, for example, the origin of the biological tissue, that is, the extracellular matrix contained in the biological tissue. When the biological tissue is an animal tissue, examples of the enzyme include enzymes that decompose the components of the extracellular matrix, such as collagen, proteoglycan, hyaluronic acid, fibronectin, laminin, tenascin, elastin, and chitin. Specifically, the enzyme may be, for example, a collagenase, a glycosidase, a hyaluronidase, a protease (e.g., a matrix metalloprotease, a serine protease, etc.), an elastase, and/or a chitinase. When the biological tissue is a plant tissue, examples of the enzyme include enzymes that decompose a cell wall (containing cellulose, hemicellulose, lignin, pectin, cuticle, etc.) and the like. Specifically, the enzyme may be, for example, a protease, a cellulase, a hemicellulase, a lignin peroxidase, a manganese peroxidase, a laccase, an amylase, and/or pectinase.

When the biological tissue is a tissue of a tea plant (tea) or a coffee plant (coffee), the enzyme is preferably, for example, a cellulase, a hemicellulase, a pectinase, an amylase, a protease, or the like from the viewpoint of the extraction efficiency.

The enzyme need only include, for example, an enzyme capable of decomposing at least one component of the extracellular matrix contained in the biological tissue. The enzyme may be purified or partially purified, or a microorganism expressing the enzyme, a processed product of the microorganism, or a culture solution of the microorganism (microbial culture solution).

The conditions for the decomposition reaction in the decomposition step are not particularly limited, and can be set to, for example, conditions under which the extracellular matrix of the biological tissue can be decomposed with the enzyme. The pH of the reaction solution can be set as appropriate in accordance with, for example, the type of the enzyme, and is, for example, 1 to 10, 2 to 9, 3 to 8, or 4 to 7.

The temperature of the reaction solution can be set as appropriate in accordance with, for example, the type of the enzyme, and is, for example, 20°C to 70°C, 25°C to 65°C, 35°C to 60°C, 40°C to 60°C, or 45°C to 55°C.

(Separation Step)

The separation step is a step of separating cells of the biological tissue and/or an extract of the biological tissue from the extracellular matrix by subjecting the biological tissue to separation. Accordingly, the separation step can also be referred to as, for example, a "cell separation step". In the separation step, the cells of the biological tissue and/or the extract of the biological tissue is separated from the extracellular matrix. Accordingly, in the reaction solution during or after the separation step, the biological tissue and the components derived from the biological tissue on which the enzyme can act have relatively increased surface areas compared with the reaction solution before the separation step, and can thus be more efficiently decomposed with the enzyme. Moreover, due to the agitation effect along with the separation, nutrients that still remain in the cells even after disruption of the cell walls can be extracted into the solution. Therefore, in the production method of the present disclosure, the extracellular matrix of the biological tissue and other components (e.g., cells or cellular components) of the biological tissue can be effectively separated from each other due to a combination of the enzymatic decomposition in the decomposition step and the separation of the cells of the biological tissue and/or the extract of the biological tissue from the extracellular matrix in the separation step.

It is preferable to conduct the separation step on, for example, the reaction solution containing the biological tissue and the enzyme. When the separation step is conducted during or after the decomposition step, the above-mentioned separation is conducted on the reaction solution prepared in the decomposition step in the separation step. When the separation step is conducted before the decomposition step, in the separation step, the reaction solution is prepared in advance, and the above-mentioned separation is conducted on the resulting reaction solution.

In the separation step, for example, cells included in the biological tissue are mechanically separated from the biological tissue. The separation step may include a processing operation (shearing) for mechanically shearing the extracellular matrix of the biological tissue because, for example, the extracellular matrix and the other components can be more efficiently separated from each other. The mechanical shearing can be conducted using, for example, a shearing apparatus (shearing machine). It is preferable that the shearing apparatus includes a shearing blade because the extracellular matrix and the other components can be more efficiently separated from each other. Specifically, the separation step includes, for example, a processing operation for mechanically shearing the extracellular matrix of the biological tissue by bringing the biological tissue into contact with an agitation blade having a shearing blade. The separation step includes, for example, a processing operation for homogenizing the biological tissue under agitation using a homogenizer. With this processing operation, the extracellular matrix of the biological tissue and the other components (e.g., cells or cellular components) of the biological tissue can be effectively separated from each other.

In the separation step, the setting of the mechanical shearing can be determined as appropriate in accordance with, for example, the origin of the biological tissue, that is, the extracellular matrix contained in the biological tissue. When the biological tissue is an animal tissue, the processing operation for mechanically shearing the extracellular matrix of the biological tissue may be, for example, a processing operation for homogenizing the biological tissue using a Waring blender, a mixer, ultrasonic homogenization, or the like. When the biological tissue is a plant tissue, the processing operation for mechanically shearing the extracellular matrix of the biological tissue may be, for example, a processing operation for homogenizing the biological tissue under agitation using a homogenizer or the like.

When the biological tissue is a tissue of a tea plant (tea) or a coffee plant (coffee), the mechanical shearing may be shearing conducted using any device with a blade capable of effectively shearing the tissue of the tea plant (tea) or the coffee plant (coffee).

The temperature during the processing operation for mechanically shearing the extracellular matrix of the biological tissue can be set as appropriate in accordance with, for example, an enzyme used in the decomposition step. The temperature is, for example, a temperature at which the enzyme exhibits enzymatic activity, and is specifically 20°C to 70°C, 25°C to 65°C, 35°C to 60°C, 40°C to 60°C, or 45°C to 55°C.

The separation step may further include a processing operation (solid-liquid separation) for separating a liquid and a solid of the biological tissue. Due to the separation step including the processing operation for separating a liquid and a solid, for example, the extracellular matrix and the other components contained in the processed solution after the shearing can be separated from each other. Due to the separation step including the solid-liquid separation, it is possible to separate, for example, the cell wall, which is the extracellular matrix of the plant tissue, and/or cellulose and other components derived from the plant tissue in the separation step. The solid-liquid separation can be conducted using, for example, a known solid-liquid separation method, and specifically can be conducted using a filtration means such as a filter, a membrane, a mesh (e.g., stainless mesh), or a sieving net, a cyclone, a centrifuge, or the like.

The decomposition step and/or the separation step may be independently conducted once or a plurality of times. The number of times of conducting the decomposition step and the number of times of conducting the separation step may be the same or different. The decomposition step and the separation step may be conducted separately, and parts or all of the decomposition step and the separation step may be conducted simultaneously.

(Order of Steps)

The decomposition step and the separation step can be conducted in any order. For example, these steps can be conducted as follows:

(1) the degradation step and (2) the separation step; or
(1) the separation step and (2) the decomposition step.

One or more decomposition steps and one or more separation steps can also be conducted any number of times in any order. For example, these steps can be conducted as follows:

(1) the decomposition step, (2) the separation step, (3) the decomposition step, and (4) the separation step; or
(1) the separation step, (2) the decomposition step, (3) the separation step, and (4) the decomposition step.

The decomposition step and the separation step can also be independently conducted any number of times consecutively. For example, these steps can be conducted as follows:

(1) the decomposition step, (2) the decomposition step, and (3) the separation step; or
(1) the separation step, (2) the separation step, and (3) the decomposition step.

The decomposition step and the separation step can also be conducted simultaneously. A combination of the decomposition step and the separation step can also be repeated any number of times. For example, these steps can be conducted as follows:

(1) the decomposition step and the separation step, and (2) the decomposition step and the separation step; or
(1) the decomposition step and the separation step, (2) the decomposition step and the separation step, and (3) the decomposition step and the separation step.

Thus, with the production method of the present disclosure, an extract can be produced from the biological tissue. The production method of the present disclosure can be favorably conducted using, for example, a separation device of the present disclosure, which will be described below.

In the production method of the present disclosure, for example, pretreatment such as application of a physical stimulus (e.g., crumpling, crushing, or scratching) to a biological tissue, especially the cell walls in the biological tissue, may be conducted prior to the decomposition step and/or the separation step. Examples of the pretreatment include food-mill pulverization, mill-grinding pulverization, homogenization, shearing, heat treatment, rolling (crumpling process), wringing, compressing or pressing, squeezing, crushing, friction welding, and the like. Thus, in the production method of the present disclosure, the efficiency of obtaining an extract (extract solution) from the biological tissue can be improved due to, for example, the pretreatment included.

In the production method of the present disclosure, the obtained extract (extract solution) contains, for example, an enzyme derived from the biological tissue. In this case, in the production method of the present disclosure, the enzyme may be deactivated, for example, after the decomposition step and/or the separation step. The enzyme can be deactivated through a processing operation for allowing proteins or nucleic acids to be deactivated (e.g., by heating the extract (extract solution) or changing the pH of the extract (extract solution)).

In the production method of the present disclosure, the conditions of each process may be set so as to, for example, adjust the efficiency of extracting a certain component among components contained in the biological tissue. In one example, the efficiency of extracting catechin, which is contained in the tea leaves and the like, from the biological sample to a liquid fraction is improved when the decomposition step and/or the separation step is conducted at a relatively high temperature. Meanwhile, the efficiency of extracting the catechin from the biological sample to a liquid fraction is reduced when the decomposition step and/or the separation step is conducted at a relatively low temperature. In a specific example, the efficiency of extracting the catechin can be significantly improved by, for example, conducting the extraction from the biological tissue at a high temperature (e.g., 80°C) compared with the extraction from the biological tissue conducted at room temperature (e.g., 30°C). Thus, a component that is extracted from the biological tissue with efficiency varying depending on the temperature can be easily extracted from a solid fraction to a liquid fraction by, for example, exposing the biological tissue to a high-temperature liquid at any timing during the solid-liquid separation in the separation step.

[Extract of Biological Tissue]

Another aspect of the present disclosure relates to an extract of a biological tissue produced using the production method of the present disclosure.

[Food Composition Containing Extract]

Another aspect of the present disclosure relates to a food composition containing the extract.

[Method for Extracting Extract of Biological Tissue]

Another aspect of the present disclosure is a method for extracting an extract of the biological tissue, including:

a step of decomposing extracellular matrix of the biological tissue with an enzyme; and
a step of separating cells of the biological tissue and/or an extract of the biological tissue from the extracellular matrix by subjecting the biological tissue to separation.

[First Biological Tissue Processing Apparatus]

<Biological Tissue Processing Apparatus 1A of Embodiment>

As described above, when desired cells C and an extract and/or an extract solution (referred to merely as an "extract E" hereinafter) containing a desired component are extracted (produced) from a biological tissue (target) T, it is necessary to efficiently separate the cells C, the extract E, and the like from extracellular matrix M and a cell plasma membrane. The following describes a biological tissue processing apparatus 1A of this embodiment capable of efficiently separating the cells C and the extract E from the extracellular matrix M and the cell plasma membrane and extracting cells, a desired extract, or the like.

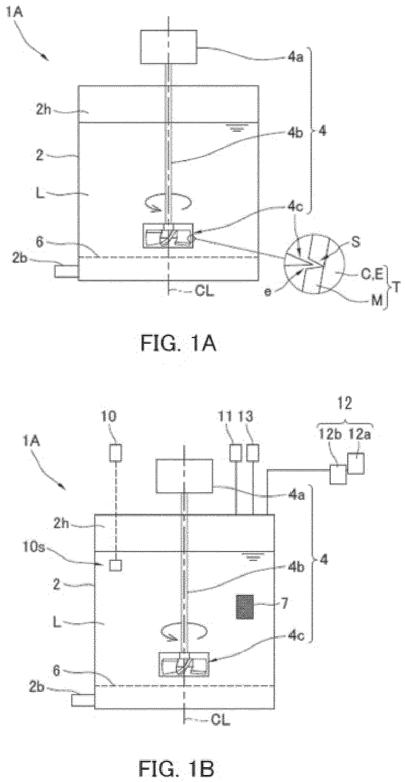

As shown in FIG. 1A, the biological tissue processing apparatus 1A of this embodiment includes a housing container 2 and a separation device 3.

(Housing Container 2)

The housing container 2 is a container for housing a mixed solution L obtained by mixing the biological tissues T (thus-obtained mixed solution L may also be referred to merely as a "mixed solution L" hereinafter) and a solution L1 containing the above-described enzyme (this solution L may also be referred to merely as a "solution L1" hereinafter). The housing container 2 is a container made of, for example, a metal such as stainless steel, steel, or a plated copper plate or a resin such as an acrylic resin, FRP, polypropylene, polyvinyl chloride (PVC), polyethylene, or ABS, and has properties of not affecting the mixed solution L, the biological tissues T, cells separated from the biological tissues T, an extract extracted from the biological tissues T, and the like. That is to say, the housing container 2 is made of a material that does not react with the solution L1 (e.g., the enzyme contained in the solution L1), the biological tissues T, cells separated from the biological tissues T, an extract extracted from the biological tissues T, and the like or is not corroded by the solution L1 (e.g., the enzyme contained in the solution L1, and the like) even when the mixed solution L and the biological tissues T are housed in a housing space 2h of the housing container 2 and then the processing operations are conducted. There is no particular limitation on the size of the housing container 2, and the housing container 2 need only have such a size that suitably enables the decomposition of the biological tissues T contained in the mixed solution L and extraction of the extract and the like.

The housing container 2 may have a structure in which the housing space 2h can be sealed from the outside in an airtight manner and a liquid-tight manner. That is to say, the housing container 2 may have a structure in which the housing space 2h can be sealed from the outside in an airtight manner and a liquid-tight manner when the mixed solution L, which will be described below, is processed. For example, the housing container 2 may be a bottomed cylindrical container with an opening in the upper portion that has a configuration in which the housing space 2h can be sealed from the outside in an airtight manner and a liquid-tight manner due to a lid attached to the opening via a packing. If the mixed solution L can be processed without problems in a state in which it is open to the atmosphere, the housing container 2 does not necessarily have a structure in which the housing space 2h can be sealed from the outside in an airtight manner and a liquid-tight manner.

(Separation Device 3)

As shown in FIGs. 1A-1B, the separation device 3 includes an agitation device 4 for agitating the mixed solution L in the housing space 2h of the housing container 2 to apply, to the biological tissues T, a decomposition force for decomposing the biological tissues T. The agitation device 4 includes a driving source 4a such as a motor, a rotating shaft 4b coupled to the main shaft of the driving source 4a, and an agitation blade 4c provided at the leading end of the rotating shaft 4b. The agitation blade 4c of the agitation device 4 is placed in the housing space 2h of the housing container 2. For example, the agitation blade 4c of the agitation device 4 is disposed in the housing space 2h of the housing container 2 such that a central axis CL of the housing space 2h of the housing container 2 coincides with the central axis of the rotating shaft 4b.

The wording "the central axis CL of the housing space 2h coincides with the central axis of the rotating shaft 4b" as used herein is a concept that encompasses not only a case where the central axis CL of the housing space 2h is coaxial with the central axis (rotation axis) of the rotating shaft 4b, but also a state in which the central axis of the rotating shaft 4b is slightly displaced in a direction intersecting the central axis CL of the housing space 2h and a state in which the central axis of the rotating shaft 4b is slightly inclined relative to the central axis CL of the housing space 2h.

The agitation blade 4c of the agitation device 4 has a shape that enables a leading edge e thereof, namely the edge placed on the front side in the rotational direction during the rotation, can apply a shearing force to the biological tissue T and the like. That is to say, the agitation device 4 has, for example, a function of a shearing apparatus (shearing machine) with a function of enabling a processing operation (shearing) for mechanically shearing the extracellular matrix M and cells of the biological tissue T. For example, the leading edge e of the agitation blade 4c has a shape that can form a scratch S such as a cut in the extracellular matrix M and the cell plasma membrane (hereinafter, the extracellular matrix M and the cell plasma membrane may be collectively referred to as "extracellular matrix M, etc.") when the rotating agitation blade 4c comes into contact with the extracellular matrix M and the cells of the biological tissue T (see FIG. 1A). Also, the leading edge e of the agitation blade 4c has a shape that enables separation of the cells C and the extract E of the biological tissues T from the extracellular matrix M when the extracellular matrix M, etc., is damaged or the extracellular matrix M, etc., is decomposed with the enzyme contained in the solution L1.

The leading edge e of the agitation blade 4c corresponds to a shearing blade as described in the claims.

(Processing of Biological Tissues T Using Biological Tissue Processing Apparatus 1)

With the biological tissue processing apparatus 1 having the structure as described above, the mixed solution L is intermittently processed, thus making it possible to achieve a state in which the extracellular matrix M, etc., and the cells C and the extract E are separated from the biological tissues T.

First, the solution L1 having a predetermined pH and a predetermined enzyme concentration is prepared, and a predetermined amount of the solution L1 is placed into the housing space 2h of the housing container 2. Then, the driving source 4a of the agitation device 4 is operated to agitate the solution L1 with the agitation blade 4c. By placing the biological tissues T into the solution L1 in the housing space 2h of the housing container 2 in this state, the mixed solution L in which the biological tissues T and the solution L1 are mixed is prepared in the housing space 2h of the housing container 2.

Here, when the biological tissues T are mixed with the solution L1 while the driving source 4a of the agitation device 4 is operated, the following situations 1) to 4) occur in the mixed solution L1 simultaneously or in a multiplex manner, thus making it possible to achieve a state in which the extracellular matrix M, etc., the cells C, and the extract E are suspended in the mixed solution L while the extracellular matrix M, etc., and the cells C and the extract E are separated from the biological tissues T. Note that when flowing out from the cells C, an extract E in a liquid form can be suspended in the mixed solution L or be mixed in the mixed solution L.

1) As mixing of the solution L1 and the biological tissues T progresses due to the rotation of the agitation blade 4c, the enzyme contained in the solution L1 comes into contact with the biological tissues T, and thus the extracellular matrix M, etc., of the biological tissues T is decomposed with the enzyme. Thus, in a portion of the biological tissues T, the cells C and the extract E of the biological tissue T are separated from the extracellular matrix M, etc., at a position at which the extracellular matrix M, etc., is decomposed, and flow out therefrom. Also, in a portion of the biological tissues T, the cells C and the extract E of the biological tissue T are not separated from the extracellular matrix M, etc. Thus, the biological tissue T in which the cells C and the extract E are linked to the extracellular matrix M, etc., and are exposed from the extracellular matrix M, etc., (such a biological tissue T may also be referred to as a "non-separate-type biological tissue T1") is suspended in the mixed solution L.

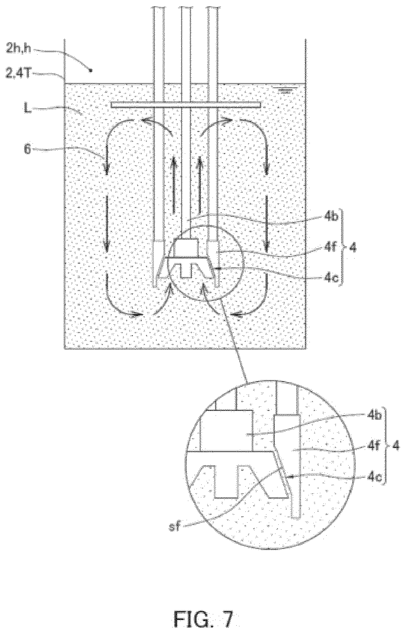

2) A stream is generated in the mixed solution L due to the rotation of the agitation blade 4c (see, for example, FIG. 7), and therefore, mixing of the biological tissues T and non-separate-type biological tissues T1 and the solution L1 progresses due to the stream. Also, a force generated by the stream of the mixed solution L is applied to the biological cells T, non-separate-type biological tissues T1, and the like. When the force generated by the stream is applied to the non-separate-type biological tissues T1, the cells C and the extract E are separated from the extracellular matrix M, etc., of a portion of the non-separate-type biological tissues T1. Note that in the case of the non-separate-type biological tissue T1 in which the cells C and the extract E are not separated from the extracellular matrix M, etc., even when the force generated by the stream is applied thereto, the non-separate-type biological tissue T1 is suspended in the mixed solution L as it is.

3) Due to the rotation of the agitation blade 4c, a situation occurs in which the agitation blade 4c comes into contact with the biological tissue T in the mixed solution L. When the agitation blade 4c comes into contact with the biological tissue T1, a cut S is generated in the extracellular matrix M, etc., in the biological cells T in which the leading edge e of the agitation blade 4c comes into contact with the extracellular matrix M, etc., at a suitable angle, out of the biological cells T with which the leading edge e comes into contact. That is to say, a shearing force is applied from the leading edge e of the agitation blade 4c to the extracellular matrix M, etc., and thus the cut S is generated. At this time, if the cut S generated is deep, the cells C and the extract E that have been covered by the extracellular matrix M, etc., will flow out from the extracellular matrix M, etc., at the position of the cut S, or the cells C and the extract E will be exposed in a state in which the cells C and the extract E are not separated from the extracellular matrix M, etc., that is, in the non-separate-type biological tissues T1. Also, even when the cut S is shallow, if the enzyme enters the extracellular matrix M, etc., through the cut S, the extracellular matrix M, etc., which cannot be decomposed without the cut S, are also decomposed with the enzyme. That is to say, the rotation of the agitation blade 4c makes it possible to promote the decomposition of the extracellular matrix M, etc.

4) When the non-separate-type biological tissues T1 are present in the mixed solution L, a situation occurs in which the agitation blade 4c comes into contact with the non-separate-type biological tissue T1 in the mixed solution L due to the rotation of the agitation blade 4c. When the agitation blade 4c comes into contact with the non-separate-type biological tissue T1, a shearing force, with which the cells C and the extract E are separated from the extracellular matrix M, etc., is applied to the non-separate-type biological tissue T1 with which the leading edge e of the agitation blade 4c comes into contact at a suitable angle, out of the non-separate-type biological tissues T1 with which the leading edge e comes into contact. Thus, the extracellular matrix M, etc., and the cells C and the extract E separated from the extracellular matrix M, etc., are suspended in the mixed solution L.

As described above, the biological tissue processing apparatus 1A of this embodiment can be used to separate the extracellular matrix M, etc., and the cells C and the extract E from the biological tissue T. In other words, the cells C and the extract E can be extracted from the biological tissue T.

(Operation Control of Biological Tissue Processing Apparatus 1A of Embodiment)

In the case of the biological tissue processing apparatus 1A of this embodiment, after confirming the state of the mixed solution L in the housing space 2h of the housing container 2, that is, the processed state of the biological tissue T, an operator can operate and stop the agitation device 4, and adjust the rotation rate of the agitation blade 4c and the like. In this case, the operator may visually confirm the state of the mixed solution L, and then adjust the rotation rate of the agitation blade 4c or stop the agitation (i.e., finish the processing).

Also, based on the results of a preparatory experiment, the processing may be completed by a timer operation or operator operation stopping the agitation by the agitation device 4 without confirming the state of the mixed solution L, on the assumption that the processing is completed when a certain period of time elapses after the agitation has been started or the biological tissue T has been placed.

Meanwhile, a sensor 10s for determining the state of the mixed solution L may be provided to adjust the rotation rate of the agitation blade 4c or stop the agitation (i.e., finish the processing) based on the signals from the sensor 10s. For example, a control unit 10 may be provided on the biological tissue processing apparatus 1A of this embodiment such that the control unit 10 controls the operation of the agitation device 4 based on the signals from the sensor 10s (see FIG. 1B). In this case, examples of the sensor for determining the state of the mixed solution L, namely the sensor 10s for determining the processed state of the biological tissue T, include a viscometer, an image sensor (using a camera for AI analysis of a separation state and colors), a pH sensor, a thermometer, and the like. Note that the signals obtained from the pH sensor, thermometer, or the like can be used as information for determining the processed state of the biological tissue T as well as information for adjusting the state of the mixed solution L when the control unit 10 controls the operation of an electrolytic solution supply device 12, an enzyme addition unit 13, a heater for adjusting the temperature of the mixed solution L, and the like, which will be described below. In order to allow the control unit 10 to control the operation state of the agitation device 4, it is desirable to provide a load sensor wattmeter for the agitation motor serving as the driving source 4a, a strain gage, a rotation rate sensor, and the like, as the sensors for determining the operation state of the agitation device 4.

(Method for Supplying Biological Tissue T)

In the description of the example above, the biological tissues T in an amount corresponding to the amount of the solution L1 in the housing space 2h of the housing container 2 are collectively supplied to the housing space 2h of the housing container 2. The biological tissues T may be supplied in small portions to the housing space 2h of the housing container 2. When supplied in small portions to the housing space 2h of the housing container 2, the biological tissues T can be easily dispersed in the solution L1, thus making it easy to adjust the state of mixing the solution L1 and the biological tissues T together, namely the state of the mixed solution L, to a favorable one.

For example, as shown in FIG. 1B, a biological tissue supply unit 11 for supplying the biological tissues T as appropriate may be provided to supply the biological tissues T in a suitable amount depending on the state of the mixed solution L. In order to allow the biological tissue supply unit 11 to supply the biological tissues T, an operator operates the biological tissue supply unit 11 based on the state of the mixed solution L (for example, based on visual confirmation and the signals from the sensor for determining the state of the mixed solution L).

The control unit 10 may be provided such that the control unit 10 controls the operation of the biological tissue supply unit 11 based on the signals from the sensor for determining the state of the mixed solution L as described above to supply the biological tissues T. In this case, it is possible to more suitably process the biological tissues T if the control unit 10 controls the operation state of the agitation device 4 in accordance with the amount of the biological tissues T supplied by the biological tissue supply unit 11.

(Method for Adjusting Solution L1)

In the description of the example above, the solution L1 that has been prepared in advance in a predetermined state is supplied to the housing space 2h of the housing container 2, but the solution L1 may be prepared in a predetermined state in the housing space 2h of the housing container 2.

For example, as shown in FIG. 1B, providing the enzyme addition unit 13 for supplying, to the housing space 2h of the housing container 2, an electrolytic solution prepared to have a predetermined pH and the electrolytic solution supply device 12 for supplying the enzyme to the housing space 2h of the housing container 2 makes it possible to prepare the solution L1 in a predetermined state in the housing space 2h of the housing container 2. In addition, it is possible to stabilize the state of the mixed solution L if the enzyme and the electrolytic solution are supplied even while the mixed solution L is processed in the biological tissue processing apparatus 1A of this embodiment. In particular, when the biological tissues T are supplied in small portions to the housing space 2h of the housing container 2, the enzyme and the electrolytic solution can be supplied in accordance with the supplied state and the processed state of the biological tissues T, thus making it possible to conduct suitable and effective processing on the biological tissues T.

In this case, it is desirable that the electrolytic solution supply device 12 is configured to include an electrolyzed water production device 12a for generating an electrolytic solution with a predetermined pH, and a reserve tank 12b for storing the electrolytic solution generated by the electrolyzed water production device 12a. With the configuration above, the electrolytic solution can be stably supplied from the reserve tank 12b to the housing space 2h of the housing container 2. Also, if the reserve tank 12b is provided with a function of preparing the state of the stored electrolytic solution, it is possible to further stabilize the state of the electrolytic solution supplied to the housing space 2h of the housing container 2. In this case, the reserve tank 12b corresponds to an adjusting unit as described in the claims.

(Method for Separating Extracellular Matrix M, Etc., and Extract E, Etc., from Each Other)

The extracellular matrix M, etc., the cells C, and the extract E (the cells C and the extract E may be collectively referred to as "extract E, etc." hereinafter) separated by the biological tissue processing apparatus 1A of this embodiment are collected from the mixed solution L. In order to efficiently collect the extracellular matrix M, etc., and the extract E, etc., it is desirable to separately collect the extracellular matrix M, etc., and the extract E, etc., from each other after separating them from each other. The separation can be conducted by passing the mixed solution L through a filtration member such as a filter or a net-like member. That is to say, the extracellular matrix M, etc., and the extract E, etc., can be separated from each other by passing the mixed solution L through a filtration member with apertures or openings through which the extracellular matrix M, etc., cannot pass but the extract E, etc., can pass together with the mixed solution L.

For example, when the biological tissues T are tea leaves, the mixed solution L processed using the biological tissue processing apparatus 1A of this embodiment is allowed to pass through a net with apertures (having, for example, a size of 1.5 to 3 mm, desirably about 2 mm) through which the cell walls of the tea leaves cannot pass but the extract (containing, for example, insoluble proteins, insoluble amino acids, and the like) can pass. Thus, the cell walls can be collected on the net, and the extract can be separated from the cell walls while being contained in the mixed solution L. Note that the extract in the mixed solution L separated from the cell walls can be collected through a technique such as concentration-separation, freezeconcentration spray drying, or evaporation treatment.

There is no particular limitation on the method for separating the extracellular matrix M, etc., and the extract E, etc., from each other by passing the mixed solution L through a filtration member, and any method may be employed. For example, a method may also be employed in which the mixed solution L is discharged through the opening of the housing container 2 to the outside by tilting the housing container 2 and is supplied to the filtration member, and then the extracellular matrix M, etc., and the extract E, etc., are separated from each other.

Also, a method may be employed in which an outlet 2b provided with a valve or the like for providing communication/blockage between the housing space 2h and the outside is provided near the bottom of the housing container 2, the mixed solution L discharged through the outlet 2b is supplied to a collection device such as a filtration member, a cyclone, a centrifuge, a mesh (net made of stainless steel or the like), or a membrane, and then the extracellular matrix M, etc., and the extract E, etc., are separated from each other.

When a filtration member is used to separate the extracellular matrix M, etc., and the extract E, etc., from each other, the operation to separate the extracellular matrix M, etc., and the extract E, etc., from each other is facilitated by providing the filtration member in the housing space 2h of the housing container 2.

For example, as shown in FIGs. 1A-1B, a filtration member 6 is provided near the bottom of the housing space 2h of the housing container 2, and the outlet 2b provided with a valve or the like for providing communication/blockage between the housing space 2h and the outside is provided below the filtration member 6. Thus, after the processing of the mixed solution L is finished, opening the outlet 2b makes it possible to allow the mixed solution L to flow out through the outlet 2b and collect the mixed solution L. At this time, the mixed solution L passes through the filtration member 6, and therefore, the filtration member 6 can catch the extracellular matrix M, etc., in the mixed solution L. That is to say, the extracellular matrix M, etc., can be separated from the extract E.

(Method for Collecting Extract E)

By providing a component collection portion 7 made of activated carbon, montmorillonite, or the like in the housing space 2h of the housing container 2 as shown in FIG. 1B, components such as caffeine and catechin in the extract E, etc., can be adsorbed by the component collection portion 7 and collected while the mixed solution L is processed in the housing space 2h of the housing container 2. Thus, by collecting the caffeine and catechin components using the component collection portion 7 while processing the mixed solution L, an advantage is obtained in that caffeine, catechin, and the like can be easily isolated and collected in a pre-extraction processing stage.

Particularly as for caffeine, removing caffeine in a pretreatment stage (i.e., a stage prior to collection of an extract) makes it easy to use the extract E, etc., in certain products and the like. For example, when the collected extract E, etc., is used in soft drinks involved in a problem of excessive intake of caffeine, or the extract E, etc., (e.g., proteins) is added to livestock feed, it is necessary to remove caffeine from the extract E, etc., before using the extract E, etc. However, if caffeine is collected using the component collection portion 7 during the processing in the housing space 2h of the housing container 2, there may be no need to separately conduct processing for removing caffeine from the extract E, etc. For example, if caffeine can be completely (or to an extent that the quality and the like of the extract E are not affected) removed using the component collection portion 7, there may be no need to separately conduct processing for removing caffeine on the collected extract E. Also, even if caffeine cannot be completely (or to an extent that the quality and the like of the extract E are not affected) removed using the component collection portion 7, when caffeine is removed using the component collection portion 7 to a certain extent, the efficiency of removing caffeine can be significantly improved when conducting processing for removing caffeine in a posttreatment stage (a stage after collection of the extract E), thus making it easy to use the extract E, etc.

<Biological Tissue Processing Apparatus 1B of Embodiment>

The above-described biological tissue processing apparatus 1A of this embodiment has a configuration in which the decomposition of the extracellular matrix M, etc., with the enzyme, and the separation of the extracellular matrix M, etc., and the extract E, etc., from each other through agitation are conducted in the same housing space 2h of the housing container 2. Meanwhile, if a period when the extracellular matrix M, etc., is decomposed with the enzyme without agitation is provided before the mixed solution L is agitated or after the mixed solution L is agitated, there is a possibility that the efficiency of separating the extracellular matrix M, etc., and the extract E, etc., can be improved.

The following description illustrates an example of an apparatus having a configuration in which a period when the extracellular matrix M, etc., is decomposed with the enzyme without agitation can be provided before the mixed solution L is agitated or after the mixed solution L is agitated.

Note that the wording "the extracellular matrix M, etc., is decomposed with the enzyme without agitation" encompasses both a state in which substantially no streams are generated in the mixed solution L, and a state in which an agitation flow or stream is generated in the mixed solution L to an extent that the above-described separation is not caused. For example, the wording "the extracellular matrix M, etc., is decomposed with the enzyme without agitation" also encompasses a case where the mixed solution L is agitated using an apparatus such as a stirrer for generating an agitation flow of a gentle stream.

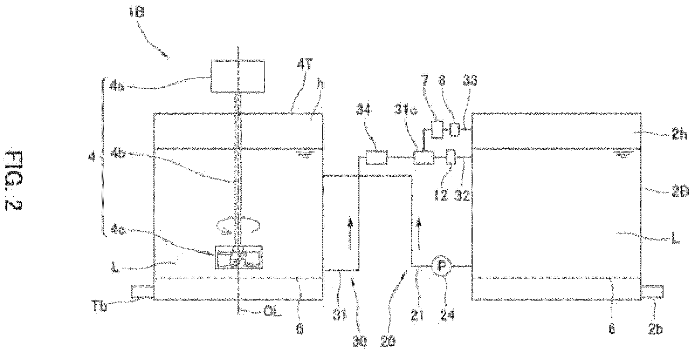

As shown in FIG. 2, a biological tissue processing apparatus 1B of this embodiment includes a housing container 2B, and a separation tank 4T that is in communication with the housing container 2B via a supply unit 20 and a return unit 30.

The housing container 2B has substantially the same configuration as that of the housing container 2 of the above-described biological tissue processing apparatus 1A of this embodiment, except that the agitation device 4 is not provided.

The separation tank 4T has substantially the same configuration as that of the housing container 2 of the above-described biological tissue processing apparatus 1A of this embodiment, and an agitation blade 4c of the agitation device 4 is provided in a separation space h of the separation tank 4T.

The supply unit 20 includes a supply channel 21 through which the housing container 2B and the separation tank 4T are in communication with each other. The supply channel 21 is a common pipe, and one end thereof is connected to a lower portion of the housing container 2B and the other end is connected to an upper portion of the separation tank 4T.

The return unit 30 includes a return channel 31 through which the housing container 2B and the separation tank 4T are in communication with each other. The return channel 31 is a common pipe, and one end thereof is connected to an upper portion of the housing container 2B and the other end is connected to a lower portion of the separation tank 4T.

Liquid feeding devices 24 and 34 such as pumps are provided at intermediate portions of the supply channel 21 and the return channel 31, respectively.

In the biological tissue processing apparatus 1B of this embodiment, due to the configuration above, the liquid feeding devices 24 and 34 can be operated to supply the mixed solution L in the lower portion of the housing container 2B to the upper portion of the separation tank 4T through the supply channel 21, and supply the mixed solution L in the lower portion of the separation tank 4T to the upper portion of housing container 2B through the return channel 31. That is to say, the mixed solution L can be circulated between the housing container 2B and the separation tank 4T via the supply channel 21 and the return channel 31.

With the configuration above, it is possible to sufficiently react the enzyme with the extracellular matrix M, etc., of the biological tissues T in the mixed solution L in the housing container 2B, thus making it possible to promote the decomposition of the extracellular matrix M, etc., with the enzyme. Thus, the non-separate-type biological tissues T1 above account for a large proportion of the biological tissues T supplied from the housing container 2B to the separation tank 4T, thus making it possible to improve the efficiency of separating and collecting the cells C and the extract E from the extracellular matrix M, etc., when agitation is conducted using the agitation blade 4c of the agitation device 4 in the separation tank 4T. Also, even when the extracellular matrix M, etc., of the biological tissues T have not been decomposed, the strength of the extracellular matrix M, etc., has decreased through the reaction with the enzyme, and therefore, the cut S is likely to be generated in the extracellular matrix M, etc., at the time of contact with the agitation blade 4c.

Meanwhile, the mixed solution L agitated in the separation tank 4T by the agitation blade 4c of the agitation device 4 is supplied to the housing container 2B, thus making it possible to sufficiently react the biological tissues T in which the cut S has been formed in the extracellular matrix M, etc., by the agitation blade 4c with the enzyme in the housing container 2B. Thus, it is possible to promote the decomposition of the extracellular matrix M, etc., with the enzyme in the housing container 2B compared with the case where the reaction with the enzyme is caused without agitation.

There is no particular limitation on a method for collecting the mixed solution L after the processing has been finished in the biological tissue processing apparatus 1B of this embodiment having the configuration above. For example, an outlet Tb and an outlet 2b for discharging the mixed solution L from the separation space h of the separation tank 4T and the housing space 2h of the housing container 2B are provided at bottom portions of the separation tank 4T and the housing container 2B. Thus, because the mixed solution L can be collected by allowing the mixed solution L to flow out to the outside when the processing of the mixed solution L is finished, it is easy to collect the mixed solution L.

In the biological tissue processing apparatus 1B of this embodiment having the configuration above, during the circulation between the separation tank 4T and the housing container 2B, the extracellular matrix M, etc., and the extract E, etc., may be separated from each other, or components such as caffeine and catechin may be collected. For example, the return channel 31 is provided with a first return channel 32 for feeding the mixed solution L fed from the separation tank 4T directly to the housing container 2B, a second return channel 33 for feeding the solution to the housing container 2B through the component collection portion 7 made of activated carbon or a solid-liquid separation device 8 such as a cyclone or a filter, and a switching mechanism 31c for switching the channel through which the mixed solution L flows between the first return channel 32 and the second return channel 33. Thus, when the mixed solution L is allowed to flow through the first return channel 32 using the switching mechanism 31c, the regular processing can be continued. Meanwhile, when the mixed solution L is allowed to flow through the second return channel 33 using the switching mechanism 31c, the extracellular matrix M, etc., can be separated from the mixed solution L from the mixed solution L, and components such as caffeine and catechin can be collected using the component collection portion 7.

The switching mechanism 31c need only have a function of switching the channel between the first return channel 32 and the second return channel 33, and the mechanism is not particularly limited. For example, a known branched valve or the like can be employed as the switching mechanism 31c.

Also, the second return channel 33 may be provided with both the component collection portion 7 and the solid-liquid separation device 8, or only one of them. For example, when the component collection portion 7 is provided in the separation tank 4T or the housing container 2B, the second return channel 33 may be provided with only the solid-liquid separation device 8. When the filtration member 6 is provided in the housing container 2B or the separation tank 4T, the second return channel 33 may be provided with only the component collection portion 7. Furthermore, the second return channel 33 may be further provided with branch channels, each of which may be provided with the component collection portion 7 and the solid-liquid separation device 8. In this case, the second return channel 33 may be provided with a switching mechanism for switching channels through which the mixed solution L flows, or the mixed solution L may be allowed to flow through both channels when the mixed solution L is supplied to the second return channel 33.

The first return channel 32 may be provided with an enzyme supply unit 12 for supplying the enzyme to the housing space 2h of the housing container 2.

<Consecutive Processing>

If the biological tissue processing apparatus 1B of this embodiment having the configuration above is provided with a plurality of housing containers 2B, and switches the housing containers 2B in order to circulate the mixed solution L between one housing container 2B and the separation tank 4T, the mixed solution L can be consecutively processed. Hereinafter, the apparatus provided with a plurality of housing containers 2B is referred to as a "biological tissue processing apparatus 1C of this embodiment".

As described above, in the biological tissue processing apparatus 1C of this embodiment, the states of the mixed solution L include a state (enzymatic processing state) in which only the enzymatic processing is conducted on the mixed solution L in the housing container 2, and a state (composite processing state) in which both the enzymatic processing and the agitation (i.e., separation) are simultaneously or consecutively conducted on the mixed solution L in the separation tank 4T. In the following description, the case where both the enzymatic processing state and the composite processing state are brought about, that is, a state in which the above-described processing is conducted by the biological tissue processing apparatus 1C of this embodiment, may be referred to simply as "processing".

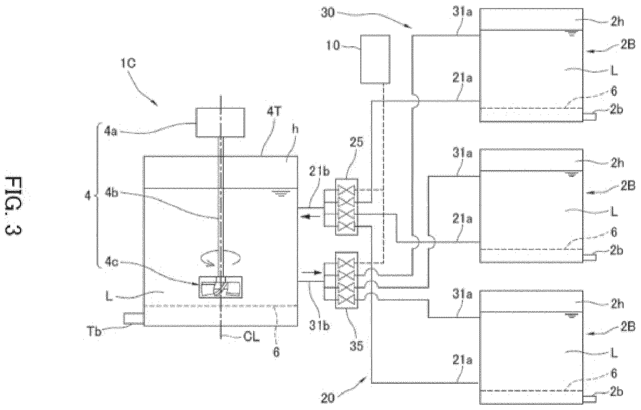

As shown in FIG. 3, the biological tissue processing apparatus 1C of this embodiment is provided with a plurality of housing containers 2B (three housing containers 2B in FIG. 3), and a plurality of processing container-side supply channels 21a that are in communication with the lower portions of the housing containers 2B as the supply units 20. Also, a separation tank-side supply channel 21b that is in communication with the upper portion of the separation tank 4T is provided. A supply-side switching mechanism 25 for switching any of the plurality of processing container-side supply channels 21a to a channel that is in communication with the separation tank-side supply channel 21b is provided between the plurality of processing container-side supply channels 21a and the separation tank-side supply channel 21b. For example, a common branched valve having a function of switching a channel for connection is provided as the supply-side switching mechanism 25.

The supply-side switching mechanism 25 may have a function of providing blockage between the plurality of processing container-side supply channels 21a and the separation tank-side supply channel 21b, that is, a function of providing complete blockage between the plurality of housing containers 2B and the separation tank 4T.

Meanwhile, a plurality of processing container-side return channels 31a that are in communication with the upper portions of the housing containers 2B are provided as the return units 30. Also, a separation tank-side return channel 31b that is in communication with the lower portion of the separation tank 4T is provided. A return-side switching mechanism 35 for switching any of the plurality of processing container-side return channels 31a to a channel that is in communication with the separation tank-side return channel 31b is provided between the plurality of processing container-side return channels 31a and the separation tank-side return channel 31b. For example, a common branched valve having a function of switching a channel for connection is provided as the return-side switching mechanism 35.

The return-side switching mechanism 35 may have a function of providing blockage between the plurality of processing container-side return channels 31a and the separation tank-side return channel 31b, that is, a function of providing complete blockage between the plurality of housing containers 2B and the separation tank 4T.

Each of the processing container-side return channels 31a may be provided with a first return channel 32a (not illustrated) and a second return channel 33a (not illustrated) similarly to the return channel 31 of the biological tissue processing apparatus 1B of this embodiment (see FIG. 2).

As described above, when the supply unit 20 and the return unit 30 have the configurations as mentioned above, switching the housing container 2B that is in communication with the separation tank 4T using the supply-side switching mechanism 25 and the return-side switching mechanism 35 makes it possible to successively switch the mixed solutions L in the plurality of housing containers 2B and process the mixed solutions L. That is to say, the mixed solution L can be processed while both the enzymatic processing state and the composite processing state are achieved substantially consecutively.

Specifically, the supply-side switching mechanism 25 provides communication between the processing container-side supply channel 21a of one housing container 2B and the separation tank-side supply channel 21b of the separation tank 4T, and the return-side switching mechanism 35 provides communication between the processing container-side return channel 31a of the same housing container 2B and the separation tank-side return channel 31b of the separation tank 4T. Thus, the mixed solution L housed in the one housing container 2B can be processed while circulated between the one housing container 2B and the separation tank 4T.

Then, when the processing of the mixed solution L housed in the one housing container 2B is finished, the supply-side switching mechanism 25 is used to switch the processing container-side supply channel 21a that is in communication with the separation tank-side supply channel 21b of the separation tank 4T from the processing container-side supply channel 21a of the one housing container 2B to the processing container-side supply channel 21a of another housing container 2B. At the same time, the return-side switching mechanism 35 is used to switch the processing container-side return channel 31a that is in communication with the separation tank-side return channel 31b of the separation tank 4T from the processing container-side return channel 31a of the one housing container 2B to the processing container-side return channel 31a of another housing container 2B. That is to say, the return-side switching mechanism 35 provides communication between the processing container-side return channel 31a of the housing container 2B whose processing container-side supply channel 21a is in communication with the separation tank-side supply channel 21b, and the separation tank-side return channel 31b of the separation tank 4T. Thus, the mixed solution L housed in the other housing container 2B can be processed while circulated between the other housing container 2B and the separation tank 4T.

Successively switching the housing container 2B that is in communication with the separation tank 4T by repeating the operation above makes it possible to successively process the mixed solutions L housed in the housing containers 2B.

Then, when the processing of the mixed solutions L housed in the plurality of housing containers 2B is all finished, an operation such as maintenance may be conducted after once discharging all the processed mixed solutions L from the plurality of housing containers 2B and stopping the processing of the mixed solution L conducted in the biological tissue processing apparatus 1B of this embodiment.

Meanwhile, if the mixed solution L is processed for a relatively long period of time, the biological tissue processing apparatus 1C of this embodiment may be operated as follows.

For example, when the processing of the mixed solution L housed in one housing container 2B is finished, the one housing container 2B is separated from the separation tank 4T, and then the mixed solution L is discharged from the outlet 2b provided in the one housing container 2B and is collected. Next, a fresh mixed solution L is supplied to the one housing container 2B from which the processed mixed solution L has been discharged. Thus, when the one housing container 2B is connected to the separation tank 4T again, the fresh mixed solution L housed in the one housing container 2B can be processed. That is to say, if the processed mixed solutions L are discharged from the plurality of housing containers 2B and then fresh mixed solutions L are supplied thereto, the mixed solution L can be consecutively processed for a long period of time by using the supply-side switching mechanism 25 and the return-side switching mechanism 35 to successively switch the housing container 2B that is connected to the separation tank 4T, even when the number of the housing containers 2B is limited.

When each of the housing containers 2B is provided with the filtration member 6, the extracellular matrix M, etc., is collected from the filtration member 6 at the time of collection of the mixed solution L.

When each of the housing containers 2B is provided with the filtration member 6, the processing container-side supply channel 21a is connected to a position above the filtration member 6. That is to say, the processing container-side supply channel 21a is provided such that the mixed solution L to be supplied from the housing container 2B to the separation tank 4T is collected from a position above the filtration member 6.

The processed mixed solution L may be discharged from an outlet Tb provided in the separation tank 4T. In this case, a fresh mixed solution L may be supplied to the separation tank 4T after the supply-side switching mechanism 25 and the return-side switching mechanism 35 are used to block communication between the separation tank 4T and all the housing containers 2B and then the processed mixed solution L is discharged from the separation tank 4T. In this case, when the separation tank 4T is provided with the filtration member 6, the separation tank-side return channel 31b is connected to a position above the filtration member 6. That is to say, the separation tank-side return channel 31b is provided such that the mixed solution L to be supplied from the separation tank 4T to the housing containers 2B is collected from a position above the filtration member 6.

The processed mixed solution L may be discharged from the separation tank 4T in a state in which the separation tank 4T and the housing container 2B remain in communication with each other. In this case, the supply-side switching mechanism 25 provides communication between the processing container-side supply channel 21a of one housing container 2B and the separation tank-side supply channel 21b of the separation tank 4T, and the return-side switching mechanism 35 provides blockage between the processing container-side return channel 31a of the one housing container 2B and the separation tank-side return channel 31b of the separation tank 4T. In this state, the processed mixed solution L is discharged from the separation tank 4T, and a fresh mixed solution L is supplied to the one housing container 2B. Thus, if all the processed mixed solution L stored in the separation tank 4T is discharged, it is possible to entirely fill the one housing container 2B with a fresh mixed solution L (or alternatively, a mixed solution containing a fresh mixed solution L for the most part, and a mixed solution L in which the enzymatic processing has progressed to some extent in a certain amount). Thereafter, using the supply-side switching mechanism 25 and the return-side switching mechanism 35 to connect the separation tank 4T and another housing container 2B to each other makes it possible to process the mixed solution L in which the enzymatic processing has progressed to some extent. Note that in this method, the separation tank 4T contains the mixed solution L containing the mixed solution L in which the enzymatic processing has progressed to some extent for the most part, and a fresh mixed solution L in a certain amount. In particular, when the capacity of the separation tank 4T is larger than the capacity of the housing container 2B, the mixed solution L in the separation tank 4T is a mixed solution L containing a fresh mixed solution L in a large amount.

If the processed mixed solution L is discharged and a fresh mixed solution L is supplied in the method above, the plurality of housing containers 2B need not be necessarily provided. That is to say, if the supply-side switching mechanism 25 provides communication between the processing container-side supply channel 21a of the housing container 2B and the separation tank-side supply channel 21b of the separation tank 4T after the entire housing container 2B is filled with a fresh mixed solution L, the circulation of the mixed solution L between the separation tank 4T and the housing container 2B can be resumed, thus making it possible to process the mixed solution L.

When the housing container 2B that is connected to the separation tank 4T is switched, an operator may switch it after confirming the processed states in the housing containers 2B and the separation tank 4T, or an operator may switch it at equal intervals. When the control unit 10 is provided, the control unit 10 may switch the housing container 2B that is connected to the separation tank 4T after confirming the processed states in the separation tank 4T and the housing containers 2B using sensors, or may switch it at equal intervals.

<Biological Tissue Processing Apparatus 1D of Embodiment>

In the description of the biological tissue processing apparatus 1B of this embodiment, the separation tank 4T in which the agitation blade 4c is installed is provided, and the mixed solution L is processed while being circulated between the separation tank 4T and the housing container 2B. If a device capable of conducting equivalent processing to the processing by the agitation blade 4c is provided, it is also possible to process the mixed solution L in the housing container 2B without providing the separation tank 4T.

The following describes a biological tissue processing apparatus 1D of this embodiment that is an apparatus having the same configuration as that of the above-described biological tissue processing apparatus 1B of this embodiment, except that the separation tank 4T is not provided.

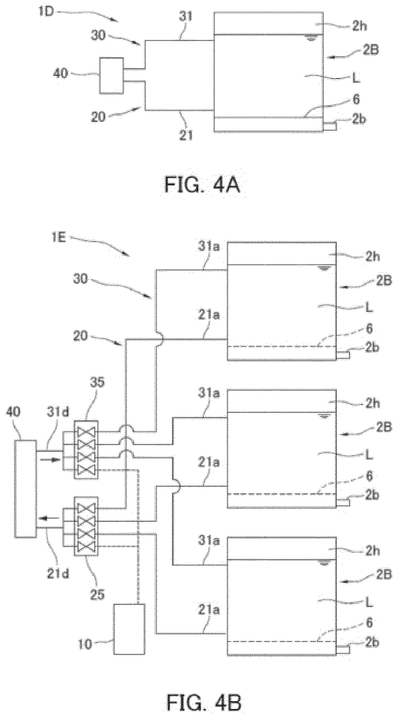

As shown in FIG. 4A, the biological tissue processing apparatus 1D of this embodiment is provided with, for example, a homogenization device 40 such as a homogenization pump instead of the separation tank 4T of the biological tissue processing apparatus 1B of this embodiment. Specifically, a supply channel 21 of the supply unit 20 is in communication with an inlet of the homogenization device 40, and a return channel 31 of the return unit 30 is in communication with an outlet of the homogenization device 40.

With the biological tissue processing apparatus 1D of this embodiment having the configuration above, if the mixed solution L is allowed to flow through the housing container 2B, the supply channel 21 of the supply unit 20, the homogenization device 40, the return channel 31 of the return unit 30, and the housing container 2B in this order while allowed to pass through the homogenization device 40, the separation in the housing container 2B and the separation by the homogenization device 40 can be consecutively conducted.

With the biological tissue processing apparatus 1D of this embodiment, a small amount of the mixed solution L can be separated using the homogenization device 40, thus making it possible to effectively apply a shearing force to the biological tissue T and the non-separate-type biological tissue T1. That is to say, the separation of the extract E, etc., from the extracellular matrix M, etc., and the generation of a cut in the extracellular matrix M, etc., can be effectively conducted.

Note that when the homogenization device 40 is a homogenization pump or the like having a pumping function, the supply channel 21 of the supply unit 20 and the return channel 31 of the return unit 30 need not be provided with a liquid feeding device. When a device having no liquid feeding function is used as the homogenization device 40, both or one of the supply channel 21 of the supply unit 20 and the return unit 30 is provided with a liquid feeding device. Also, even when the homogenization device 40 has a liquid feeding function, both or one of the supply channel 21 of the supply unit 20 and the return unit 30 may be provided with a liquid feeding device in order to stabilize the liquid feeding.

(Consecutive Processing)

Similarly to the biological tissue processing apparatus 1C of this embodiment, if the biological tissue processing apparatus 1D of this embodiment having the configuration above is also provided with a plurality of housing containers 2B, and switches the housing containers 2B to supply the mixed solution L to the homogenization device 40, the mixed solution L can be consecutively processed. Hereinafter, the apparatus provided with a plurality of housing containers 2B is referred to as a "biological tissue processing apparatus 1E of this embodiment".

As described above, in the biological tissue processing apparatus 1D of this embodiment, the states of the mixed solution L include a state (enzymatic processing state) in which only the enzymatic processing is conducted on the mixed solution L in the housing container 2, and a state (separating state) in which the separation is consecutively conducted on the mixed solution L using the homogenization device 40. In the following description, the case where both the enzymatic processing state and the separating state are brought about, that is, a state in which the above-described processing is conducted by the biological tissue processing apparatus 1D of this embodiment, may be referred to simply as "processing".

As shown in FIG. 4B, a plurality of processing container-side supply channels 21a that are in communication with the lower portions of the housing containers 2B are provided as the supply units 20, and a homogenization device-side supply channel 21d that is in communication with the inlet of the homogenization device 40 is provided. A supply-side switching mechanism 25 for switching any of the plurality of processing container-side supply channels 21a to a channel that is in communication with the homogenization device-side supply channel 21d is provided between the plurality of processing container-side supply channels 21a and the homogenization device-side supply channel 21d. For example, a common branched valve having a function of switching a channel for connection is provided as the supply-side switching mechanism 25.

The supply-side switching mechanism 25 may have a function of providing blockage between the plurality of processing container-side supply channels 21a and the homogenization device-side supply channel 21d, that is, a function of providing complete blockage between the plurality of housing containers 2B and the homogenization device 40.

Meanwhile, a plurality of processing container-side return channels 31b that are in communication with the upper portions of the housing containers 2B are provided as the return units 30. Also, a homogenization device-side return channel 31d that is in communication with the outlet of the homogenization device 40 is provided. A return-side switching mechanism 35 for switching any of the plurality of processing container-side return channels 31b to a channel that is in communication with the homogenization device-side return channel 31d is provided between the plurality of processing container-side return channels 31b and the homogenization device-side return channel 31d. For example, a common branched valve having a function of switching a channel for connection is provided as the return-side switching mechanism 35.

The return-side switching mechanism 35 may have a function of providing blockage between the plurality of processing container-side return channels 31b and the homogenization device-side return channel 31d, that is, a function of providing complete blockage between the plurality of housing containers 2B and the homogenization device 40.

Each of the processing container-side return channels 31b may be provided with a first return channel 32b (not illustrated) and a second return channel 33b (not illustrated) similarly to the return channel 31 of the biological tissue processing apparatus 1B of this embodiment (see FIG. 2).

When the configurations as mentioned above are employed, switching the housing container 2B that is in communication with the homogenization device 40 using the supply-side switching mechanism 25 and the return-side switching mechanism 35 makes it possible to successively switch the mixed solutions L in the plurality of housing containers 2B and process the mixed solutions L, similarly to the biological tissue processing apparatus 1C of this embodiment. That is to say, the mixed solution L can be processed while both the enzymatic processing state and the separating state are achieved substantially consecutively.

Specifically, the supply-side switching mechanism 25 provides communication between the processing container-side supply channel 21a of one housing container 2B and the homogenization device-side supply channel 21d, and the return-side switching mechanism 35 provides communication between the processing container-side return channel 31b of the same housing container 2B and the homogenization device-side supply channel 21d. Thus, the mixed solution L housed in the one housing container 2B can be processed while the mixed solution L is circulated between the one housing container 2B and the homogenization device 40.

Then, when the processing of the mixed solution L housed in the one housing container 2B is finished, the supply-side switching mechanism 25 is used to switch the processing container-side supply channel 21a that is in communication with the homogenization device-side supply channel 21d of the homogenization device 40 from the processing container-side supply channel 21a of the one housing container 2B to the processing container-side supply channel 21a of another housing container 2B. At the same time, the return-side switching mechanism 35 is used to switch the processing container-side return channel 31a that is in communication with the homogenization device-side return channel 31d of the homogenization device 40 from the processing container-side return channel 31a of the one housing container 2B to the processing container-side return channel 31a of another housing container 2B. That is to say, the return-side switching mechanism 35 provides communication between the processing container-side return channel 31a of the housing container 2B whose processing container-side supply channel 21a is in communication with the homogenization device-side supply channel 21d, and the homogenization device-side return channel 31d of the homogenization device 40. Thus, the mixed solution L housed in another housing container 2B can be processed while circulated between the other housing container 2B and the homogenization device 40.

Successively switching the housing container 2B that is in communication with the homogenization device 40 by repeating the operation above makes it possible to successively process the mixed solutions L housed in the housing containers 2B.

Meanwhile, if the mixed solution L is processed for a relatively long period of time, the biological tissue processing apparatus 1E of this embodiment may be operated as follows.

For example, when the processing of the mixed solution L housed in one housing container 2B is finished, the one housing container 2B is separated from the homogenization device 40, and then the mixed solution L is discharged from the outlet 2b provided in the one housing container 2B and is collected. Next, a fresh mixed solution L is supplied to the one housing container 2B from which the processed mixed solution L has been discharged. Thus, when the one housing container 2B is connected to the homogenization device 40 again, the fresh mixed solution L housed in the one housing container 2B can be processed. That is to say, if the processed mixed solutions L are discharged from the plurality of housing containers 2B and then fresh mixed solutions L are supplied thereto, the mixed solution L can be consecutively processed for a long period of time by using the supply-side switching mechanism 25 and the return-side switching mechanism 35 to successively switch the housing container 2B that is connected to the homogenization device 40, even when the number of the housing containers 2B is limited.

When each of the housing containers 2B is provided with the filtration member 6, the extracellular matrix M, etc., is collected from the filtration member 6 at the time of collection of the mixed solution L.

When each of the housing containers 2B is provided with the filtration member 6, the processing container-side supply channel 21a is connected to a position above the filtration member 6. That is to say, the processing container-side supply channel 21a is provided such that the mixed solution L to be supplied from the housing container 2B to the separation tank 4T is collected from a position above the filtration member 6.

When the housing container 2B that is connected to the homogenization device 40 is switched, an operator may switch it after confirming the processed states of the mixed solutions L in the housing containers 2B, or an operator may switch it at equal intervals. When the control unit 10 is provided, the control unit 10 may switch the housing container 2B that is connected to the homogenization device 40 after confirming the processed states in the housing containers 2B using sensors, or may switch it at equal intervals.

<Plurality of Separation Tanks 4T and Plurality of Homogenization Devices 40>

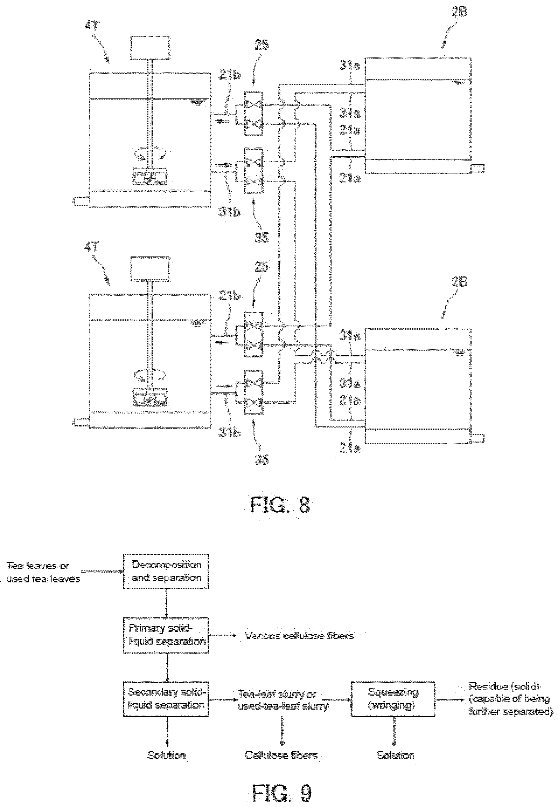

In the descriptions of the examples above, the plurality of housing containers 2B are connected to one separation tank 4T or one homogenization device 40, but a plurality of separation tanks 4T or a plurality of homogenization devices 40 may be provided and configured such that the plurality of housing containers 2B are respectively in communication with the plurality of separation tanks 4T or the plurality of homogenization devices 40. That is to say, the separation tanks 4T or the homogenization devices 40 may be configured to be switchably in communication with the plurality of housing containers 2B via the supply-side switching mechanism 25 and the return-side switching mechanism 35. For example, as shown in FIG. 8, the biological tissue processing apparatus 1C of this embodiment may have a configuration in which a plurality of (two in FIG. 8) separation tanks 4T are provided and a plurality of (two in FIG. 8) housing containers 2B are in communication therewith via the supply-side switching mechanism 25 and the return-side switching mechanism 35. In this case, if the separation tanks 4T or the homogenization devices 40 have the same agitation function, that is, the separation tanks 4T or the homogenization devices 40 are the same, it is also possible to conduct substantially the same processing on the mixed solutions L in the housing containers 2B while simultaneously circulating the mixed solutions L between the housing containers 2B and the separation tanks 4T. Meanwhile, the separation tanks 4T or the homogenization devices 40 may have different agitation functions, that is, the separation tanks 4T or the homogenization devices 40 may have a configuration in which the separation tanks 4T or the homogenization devices 40 apply different shearing forces to the biological tissues T. For example, if a plurality of separation tanks 4T are provided in which the agitation devices 4 have the agitation blades 4c with different sizes or shapes, shearing forces applied to the biological tissues T by the separation tanks 4T can be made different. Thus, by changing the separation tank 4T or the homogenization device 40, it is also possible to apply a suitable shearing force to the extracellular matrix M, etc., in accordance with the decomposition state or the like of the extracellular matrix M, etc., thus making it possible to effectively separate the extracellular matrix M, etc., and the extract E, etc., from each other.

<Device for Collecting Solid>

In the descriptions of the examples above, the extracellular matrix M, etc., is separated from the mixed solution L by providing the housing container 2 or the separation tank 4T with the filtration member 6 or providing the channel with the solid-liquid separation device 8. The housing container 2 or the separation tank 4T may be provided with a plurality of the instruments or devices for separating a solid. In this case, if the instruments or devices for separating a solid are those capable of separating different types of solids, it is possible to further classify the solids based on the size or type and collect them. For example, if a plurality of filtration members 6 are arranged in the vertical direction of the housing container 2 or the separation tank 4T, and a lower one can collect a smaller solid, it is possible to separate the solids based on the size using the plurality of filtration members 6 and collect them by merely discharging the mixed solution L from the housing container 2 or the separation tank 4T. For example, relatively large solids (e.g., long solids, solids with a large width, diameter, or the like, and the like) such as cellulose fibers can be collected in the first separation (primary solid-liquid separation), and fibers (cellulose fibers derived from tissues other than a leaf vein, and the like) smaller than cellulose fibers or the like and solids with a small width, diameter, or the like can be collected in the subsequent separation (secondary solid-liquid separation). When the housing container 2 or the separation tank 4T does not include the filtration member 6, a desired solid can be separated and collected from the mixed solution L discharged from the housing container 2 or the separation tank 4T by allowing the mixed solution L to pass through a solid-liquid separation device (e.g., a filter, a cyclone, a mesh, a membrane, or the like) capable of collecting the desired solid. Also, when the second return channel 33 shown in FIG. 2 is provided with a plurality of solid-liquid separation devices (e.g., a filter, a cyclone, a mesh, membrane, and the like) with different properties, each of the solid-liquid separation devices can separate and collect a desired solid from the mixed solution L. For example, cellulose fibers and a slurry of a solid can also be simultaneously collected during the processing process. Furthermore, when the second return channel 33 shown in FIG. 2 is provided with a plurality of solid-liquid separation devices (e.g., a filter, a cyclone, a mesh, membrane, and the like) with different properties, each of the solid-liquid separation devices can separate and collect a desired solid from the mixed solution L. For example, cellulose fibers and a slurry of a solid can also be simultaneously collected during the processing process.

(Agitation Device 4)

The agitation device 4 is not necessarily provided such that the central axis of the rotating shaft 4b thereof coincides with the central axis C of the housing space 2h of the housing container 2 or the central axis of the separation space h of the separation tank 4T.

Although FIGs. 1A-1B and the like show the case where only one agitation blade 4c of the agitation device 4 is provided in the housing space 2h of the housing container 2 or the separation space h of the separation tank 4T, two or more agitation blades 4c may be provided in the housing space 2h of the housing container 2 or the separation space h of the separation tank 4T. It is sufficient that a suitable number of agitation blades 4c are provided in accordance with the size of the housing space 2h of the housing container 2 (i.e., the depth of the housing space 2h) or the amount of the biological tissues T contained in the mixed solution L. In this case, the rotating shaft 4b of one agitation device 4 may be provided with a plurality of agitation blades 4c, or a plurality of agitation devices 4 may be provided in the housing space 2h of the housing container 2 or the separation space h of the separation tank 4T to agitate the mixed solution L using a plurality of agitation blades 4c.

When a plurality of agitation blades 4c are provided, all the agitation blades 4c may have the same shape and size, and include the same number of blades f, or the agitation blades 4c may differ in the shape, the size, the number of blades f, and the shearing force. For example, the biological tissue T and the like are likely to be precipitated at a deep position of the housing space 2h of the housing container 2 (the separation space h of the separation tank 4T), and therefore, an agitation blade 4c larger than an agitation blade 4c at a shallower position may be provided to generate more upward streams. By providing agitation blades 4c that differ in the shape or the shearing force at a deep position and a shallow position, it is also possible to more effectively separate the extracellular matrix M, etc., and the extract E, etc., from each other.

(Agitation Blade 4c)

It is sufficient that the agitation blade 4c has such a shape that the leading edge e is sharp and can apply shearing force to the extracellular matrix M, etc., of the biological tissue T when coming into contact with the extracellular matrix M, etc. That is to say, it is sufficient that the agitation blade 4c has such a shape that the leading edge e can form a scratch S such as a cut on the extracellular matrix M, etc., of the biological tissue T when coming into contact with the extracellular matrix M, etc., and can cut the linkage between the extracellular matrix M, etc., and the extract E, etc., when coming into contact with the non-separate-type biological tissue T1. For example, when the leading edge e of the agitation blade 4c has a sharp shape, that is, the surfaces forming the leading edge e of the agitation blade 4c form an acute angle, as shown in FIGs. 1A-1B, the effects as described above can be obtained.

In order to increase a shearing force applied to the extracellular matrix M, etc., of the biological tissue T, a hood member or the like is provided to cover the agitation blade 4c, and a clearance between the outer edge of the agitation blade 4c and the inner surface of the hood member is reduced. Thus, a shearing force generated between the outer edge of the agitation blade 4c and the inner surface of the hood member can also be applied to the extracellular matrix M, etc., of the biological tissue T in addition to a shearing force by the leading edge e of the agitation blade 4c, thus making it possible to enhance the effects as described above.

When the hood member is provided, it is desirable that the outer edge ef of the agitation blade 4c also has a shape that can apply a shearing force to the extracellular matrix M, etc., similarly to the leading edge e of the agitation blade 4c. Thus, a shearing force applied to the extracellular matrix M, etc., of the biological tissue T can be further increased due to a shearing force generated between the outer edge of the agitation blade 4c and the inner surface of the hood member. That is to say, a scratch S such as a cut can be effectively formed on the extracellular matrix M, etc., of the biological tissue T when the agitation blade 4c comes into contact with the extracellular matrix M, etc., and the linkage between the extracellular matrix M, etc., and the extract E, etc., can be effectively cut when the agitation blade 4c comes into contact with the non-separate-type biological tissue T1.

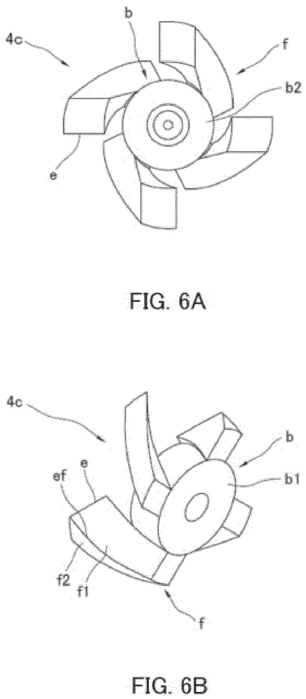

For example, assume that the agitation blade 4c is constituted by a cylindrical hub b fixed to the rotating shaft 4b, and a plurality of (four in FIGs. 6A-6B) blades f provided on the outer circumferential surface of the hub b as a rotational target manner around the rotating shaft 4b, as shown in FIGs. 6A-6B. Note that in FIGs. 6A-6B, surfaces of the hub b located at an upper position and a lower position when the agitation blade 4c is placed in the housing space 2h of the housing container 2 as shown in FIG. 7 are defined as a surface b1 and a surface b2, respectively.

An upper surface f1 of each of the blades f faces upward when the agitation blade 4c is placed in the housing space 2h of the housing container 2, and the outer edge ef formed by the upper surface f1 and an outer end surface f2 of the blade f has a sharp shape. In addition, the blade f is long to some extent in a direction in which the rotating shaft 4b extends, and the width of the upper portion (i.e., the width near the surface b1 of the hub b) is larger than the width of the lower portion (i.e., the width near the surface b2 of the hub b). That is, the agitation blade 4c has a substantially trapezoidal shape in a side view due to the shape of the blade f.

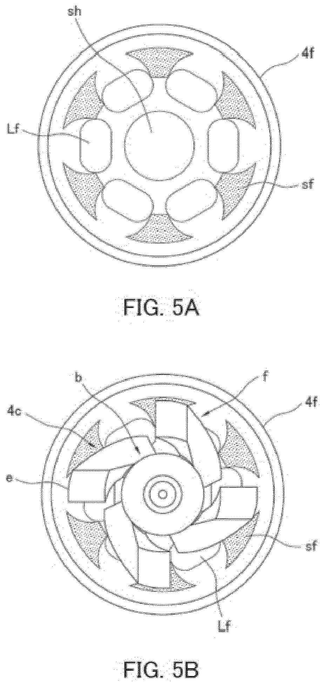

As shown in FIGs. 5A-5B, a hood member 4f for covering the agitation blade 4c (housing the agitation blade 4c thereinside) has a bottomed cylindrical shape that has a space capable of housing the agitation blade 4c thereinside, an open lower end, and a blocked upper end. Also, the upper end surface of the hood member 4f is provided with a shaft space sf through which the space and the outside are in communication with each other and the rotating shaft 4b is to be inserted, and a plurality of (six in FIGs. 5A-5B) through holes Lf through which streams of the mixed solution L generated by the agitation blade 4c can pass. Furthermore, the inner surface of the hood member 4f is provided with a shearing surface sf such that the clearance between the inner surface of the hood member 4f and the outer end surface f2 of the agitation blade 4c is reduced. The shearing surface sf has a shape that is substantially similar to the shape of the agitation blade 4c in a cross-sectional view (see FIG. 7). That is to say, the shearing surface sf is formed such that distances between the shearing surface sf and the outer end surface f2 of the agitation blade 4c at any positions corresponding to the outer end surface f2 are substantially the same. Note that in FIGs. 5A-5B, the hood member 4f and the agitation blade 4c are expressed such that the sizes on a side away from the viewpoint are reduced.

When the agitation blade 4c as described above is placed in the housing space 2h of the housing container 2 in a state of being housed in the hood member 4f, and then the agitation blade 4c is rotated, it is possible to generate a stream of the mixed solution L as shown in FIG. 7. Moreover, when the agitation blade 4c is rotated, a shearing force generated due to the biological tissue T caught between the outer edge ef and the shearing surface sf can also be applied to the biological tissue T or the non-separate-type biological tissue T1 in addition to a shearing force by the leading edge e or the outer edge ef of the agitation blade 4c alone. Thus, a scratch such as a cut can be effectively formed on the extracellular matrix M, etc., of the biological tissue T when the agitation blade 4c comes into contact with the extracellular matrix M, etc., and the linkage between the extracellular matrix M, etc., and the extract E, etc., can be effectively cut when the agitation blade 4c comes into contact with the non-separate-type biological tissue T1.

Similarly to the leading edge e of the agitation blade 4c, the outer edge ef of the agitation blade 4c also corresponds to a shearing blade as described in the claims.

[Second Biological Tissue Processing Apparatus]

An aspect of the present disclosure provides a second biological tissue processing apparatus with improved extraction efficiency. The processing apparatus of the present disclosure includes: a first housing container that is capable of housing a mixed solution obtained by mixing a biological tissue and a solution containing an enzyme and is configured to be capable of decomposing an extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a first measurement device that is configured to be capable of measuring a state of the mixed solution over time; and a first control device that is configured to be capable of controlling separation of a decomposition product of the biological tissue by the separation device based on the state of the mixed solution obtained in the measurement.

The processing apparatus of the present disclosure includes the first housing container as well as the separation device, thus making it possible to effectively separate the extracellular matrix of the biological tissue and other components (e.g., cells or cellular components) of the biological tissue from each other. Accordingly, with the processing apparatus of the present disclosure, it is possible to improve, for example, the efficiency of extracting an extract of the biological tissue. The processing apparatus of the present disclosure can also be referred to as, for example, an "apparatus for extracting an extract of a biological tissue". The processing apparatus of the present disclosure includes the first measurement device and can thereby measure the state of the mixed solution over time, thus making it possible to control the separation of the decomposition product of the biological tissue by the separation device based on the state of the mixed solution obtained through the measurement. Thus, with the processing apparatus of the present disclosure, it is possible to efficiently process a biological tissue to produce an extract of a component derived from the biological tissue.

<Embodiment>

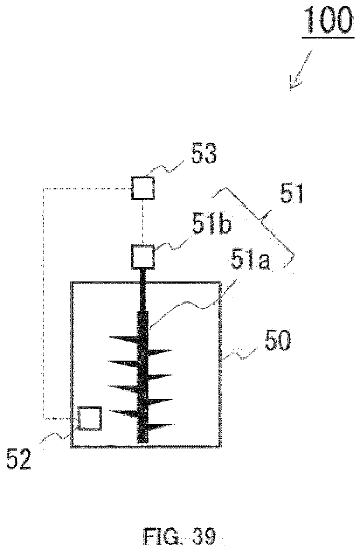

FIG. 39 is a block diagram schematically illustrating an example of a processing apparatus of this embodiment. As shown in FIG. 39, a processing apparatus 100 of this embodiment includes a first housing container 50, a separation device 51, a first measurement device 52, and a first control device 53. The separation device 51 includes an agitation blade 51a and a motor (driving unit) 51b. The agitation blade 51a of the separation device 51 is disposed in the first housing container 50. Also, the first measurement device 52 is disposed in the first housing container 50 and is electrically connected to the first control device 53. The first control device 53 is electrically connected to the motor 51b of the separation device 51 and is configured to be capable of controlling the separation by the separation device 51 by controlling the rotation of the motor 51b. With the processing apparatus 100 of this embodiment, it is possible to favorably conduct a processing method of the present disclosure, which will be described below.

(First Housing Container)

The first housing container 50 can house a mixed solution obtained by mixing a biological tissue and a solution containing an enzyme, and is configured to be capable of decomposing extracellular matrix of the biological tissue in the mixed solution with the enzyme. The first housing container 50 is, for example, a container (e.g., a tank) made of a material such as a metal, plastic, or resin. The capacity of the first housing container 50 can be determined as appropriate in accordance with the volumes of the biological tissue and the enzyme to be housed.

The first housing container 50 may include a warming device or a cooling device and be configured to be capable of warming or cooling the mixed solution, that is, adjusting the temperature of the mixed solution. Also, the first housing container 50 may include an agitation device instead of the separation device 51 and be configured to be capable of agitating a content. The agitation device may be configured to be capable of agitating a content without homogenizing it under agitation, and can be configured using, for example, an agitation blade provided with no shearing blade, which will be described below. The agitation device may be capable of, for example, shearing or homogenizing the biological tissue. In this case, the agitation device includes, for example, an agitation blade having a shearing blade.

The first housing container 50 may include, for example, a channel configured to be capable of circulating a content in the first housing container 50. For example, by providing the channel with a liquid feeding device such as a pump, the mixed solution in the first housing container 50 can be fed via the channel, and thereby the mixed solution can be circulated. The channel may be in communication with, for example, two different portions of the first housing container 50 such that the solution can pass. The channel may be, for example, a channel (e.g., a tube), etc., made of a metal, plastic, resin, or the like.

The first housing container 50 may include, for example, an ultrasonic homogenization machine. Thus, the first housing container 50 can, for example, decompose the extracellular matrix of the biological tissue with the enzyme as well as decompose the extracellular matrix of the biological tissue through ultrasonic homogenization. For example, the first housing container 50 can homogenize the extracellular matrix of the biological tissue into small fragments using the ultrasonic homogenization machine. Thus, in the reaction solution during or after the ultrasonic homogenization, the biological tissue and the components derived from the biological tissue on which the enzyme can act have relatively increased surface areas compared with the reaction solution before the ultrasonic homogenization, and can thus be more efficiently decomposed with the enzyme. The ultrasonic homogenization machine may, for example, be disposed in the first housing container 50 and be configured to be capable of ultrasonically homogenizing a content of the first housing container 50. Also, the ultrasonic homogenization machine may, for example, be disposed in the channel and be configured to be capable of ultrasonically homogenizing a content of the first housing container 50 moving through the channel.

The first housing container 50 may include, for example, an ultrasonic generator. Thus, the first housing container 50 can, for example, shake off and separate other components such as cells from the extracellular matrix of the biological tissue. Thus, in the reaction solution during or after the generation of ultrasonic waves, the biological tissue and the components derived from the biological tissue on which the enzyme can act have relatively increased surface areas compared with the reaction solution before the generation of ultrasonic waves, and can thus be more efficiently decomposed with the enzyme. The ultrasonic generator may, for example, be disposed in the first housing container 50 and be configured to be capable of conducting ultrasonication in the first housing container 50. Also, the ultrasonic generator may, for example, be disposed in the channel and be configured to be capable of conducting ultrasonication in the channel.

(Separation Device)

The separation device 51 is configured to be capable of applying a force to the biological tissue in the mixed solution to separate the cells of the biological tissue and/or the extract of the biological tissue from the extracellular matrix. The agitation blade 51a of the separation device 51 is configured to be capable of agitating the mixed solution in the first housing container 50. The motor 51b is configured to be capable of controlling the rotation of the agitation blade 51a. The separation device 51 may be, for example, a shearing machine capable of shearing a decomposition product in the first housing container 50. It is preferable that the shearing machine includes a shearing blade because the extracellular matrix and the other components can be more efficiently separated from each other. Specifically, the separation device 51 can mechanically shear the extracellular matrix of the biological tissue by, for example, bringing the biological tissue into contact with an agitation blade having a shearing blade. The separation device 51 may include a homogenization machine (homogenization device) for mechanically homogenizing the extracellular matrix of the biological tissue instead of or in addition to the shearing machine. In this case, the separation device 51 includes, for example, a homogenization machine for homogenizing a reaction solution containing the biological tissue and the enzyme under agitation using a homogenizer. The extracellular matrix of the biological tissue and the other components (e.g., cells or cellular components) of the biological tissue can be effectively separated from each other due to the separation device 51 including the shearing machine or a homogenization machine.

In the separation device 51, the shearing machine or the homogenization machine can be selected as appropriate in accordance with, for example, the origin of the biological tissue, that is, the extracellular matrix contained in the biological tissue. When the biological tissue is an animal tissue, the shearing machine or the homogenization machine may be, for example, a device for homogenizing the biological tissue using a Waring blender, a mixer, ultrasonic homogenization, or the like. When the biological tissue is a plant tissue, the shearing machine or the homogenization machine may be, for example, a device for homogenizing the biological tissue under agitation using a homogenizer or the like. Also, the shearing machine or the homogenization machine may include, for example, a homogenizer, a shear pump, an in-line mixer, a homogenization pump, or the like. The operational manner of the shearing machine or the homogenization machine can be selected as appropriate in accordance with, for example, the origin of the biological tissue, and examples thereof include those using ultrasonic homogenization, pressurization, agitation, and the like.

When the biological tissue is a plant tissue from a tea plant (tea), a coffee plant (coffee), or the like, the shearing machine may be any device with a blade for effectively shearing the plant tissue from a tea plant (tea), a coffee plant (coffee), or the like.

The shearing machine or the homogenization machine may, for example, be disposed in the first housing container 50 and be configured to be capable of shearing or homogenizing a content of the first housing container 50. Also, the shearing machine or the homogenization machine may, for example, be disposed in the channel and be configured to be capable of shearing or homogenizing a content of the first housing container 50 moving through the channel.

(First Measurement Device)

The first measurement device 52 is configured to be capable of measuring the state of the mixed solution over time. The state of the mixed solution means a state that can be evaluated based on the physical properties of the mixed solution, and examples thereof include the viscosity, pH, temperature, turbidity, color, and/or air bubble density of the mixed solution. The setting of the first measurement device 52 can be determined as appropriate in accordance with the state of the mixed solution to be measured, and the first measurement device 52 may be, for example, a viscometer, a pH meter, a thermometer, a turbidimeter, a colorimeter (color-difference meter), an air-bubble detector, or the like.

(First Control Device)

The first control device 53 is configured to be capable of controlling the separation of the decomposition product of the biological tissue by the separation device 51 based on the state of the mixed solution obtained in the measurement above. The first control device 53 can control separation of the decomposition product of the biological tissue by the separation device 51 based on, for example, a change in the state of the mixed solution over time. For example, a controller such as an analog control device (e.g., a microcomputer, an operational amplifier, or the like) or a digital control device that includes a logical circuit can be used as the first control device 53. The first control device 53 can control the separation of the decomposition product of the biological tissue by the separation device 51 by controlling the rotation rate and/or the rotational direction of the agitation blade 51a via the rotation (driving) of the motor 51b of the separation device 51.

The first control device 53 is electrically connected to the first measurement device 52, that is, connected to the first measurement device 52 via a cable, but need only be configured to be capable of acquiring the results of measurement of the state of the mixed solution by the first measurement device 52, and may be connected thereto wirelessly.

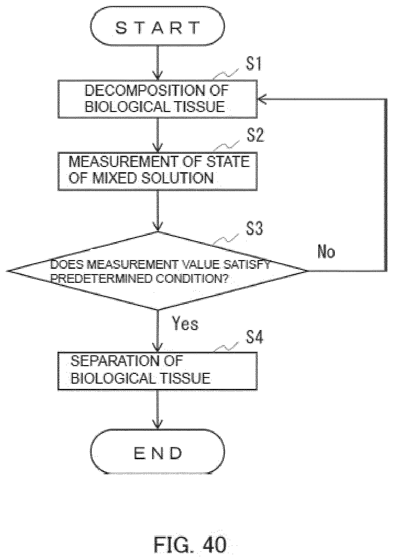

Next, an example of a processing method conducted in the processing apparatus 100 of this embodiment will be described with reference to a flowchart illustrated in FIG. 40. As shown in FIG. 40, in the processing method of this embodiment, the processing is conducted in the order of S1 (decomposition of the biological tissue), S2 (measurement of the state of the mixed solution), S3 (determination of whether or not the measurement value satisfies a predetermined condition), and S4 (separation of the biological tissue).

Prior to the process S1, a mixed solution obtained by mixing a biological tissue and a solution containing an enzyme is housed in the first housing container 50. The biological tissue and the solution containing the enzyme may be separately introduced into the first housing container 50. In the process S1, the extracellular matrix of the biological tissue in the mixed solution is decomposed with the enzyme in the first housing container 50. The conditions for the decomposition with the enzyme can be determined in accordance with the type of the enzyme.

In the process S2, the first measurement device 52 measures the state of the mixed solution over time. Thus, the measurement value of the state of the mixed solution over time is obtained. The first measurement device 52 may, for example, calculate, from the measurement value of the state over time, the rate of change in or the derivative value of the measurement value at a predetermined time.

In the process S3, it is determined whether or not the measurement value satisfies a predetermined condition. The predetermined condition may be, for example, a fixed condition such as a certain value or a variable condition calculated through a computation. When the measurement value satisfies the predetermined condition, that is, in the case of "Yes", the processing method of this embodiment proceeds to the process S4. On the other hand, when the measurement value does not satisfy the predetermined condition, that is, in the case of "No", the processing method of this embodiment returns to the process S1. Specifically, when the decomposition of the mixed solution progresses, the state of the mixed solution becomes constant. Therefore, a change in the state of the mixed solution can be evaluated based on, for example, the rate of change in or the derivative value of the measurement value. In an example in which the predetermined condition is a condition related to the rate of change in or the derivative value of the measurement value, when the rate of change or the derivative value is, for example, smaller than or equal to a certain value or substantially zero, it is determined in the process S3 that the decomposition of the biological tissue in the first housing container 50 is finished, and the processing method of this embodiment proceeds to the process S4. On the other hand, when the rate of change or the derivative value is not smaller than or equal to a certain value or substantially zero, it is determined in the process S3 that the decomposition of the biological tissue in the first housing container 50 has not been finished yet, and the processing method of this embodiment returns to the process S1.

In the process S4, the first control device 53 controls the separation device 51 to allow the motor 51b to start the rotation of the agitation blade 51a and to increase the rotation rate of the agitation blade 51a, thereby increasing the frequency of contact between the agitation blade 51a and the biological tissue in the mixture, and thus the decomposition product of the biological tissue is separated in the first housing container 50.

The processing method of this embodiment prevents the decomposition in the first housing container 50 from being continued for an unnecessarily long period of time, and the decomposition product of the biological tissue can be separated by the separation device 51 at an appropriate timing. Accordingly, with the processing method of this embodiment, it is possible to efficiently process a biological tissue to produce an extract of a component derived from the biological tissue.

In the processing method of this embodiment, the first control device 53 controls the separation device 51 to separate the decomposition product of the biological tissue in the first housing container 50 in the process S4, but the present disclosure is not limited thereto, and the first control device 53 may control the separation device 51 to finish the separation of the decomposition product of the biological tissue in the first housing container 50. When the agitation blade 51a is provided with a shearing blade, the first control device 53 may control the separation device 51 to allow the motor 51b to change the rotational direction of the agitation blade 51a in the process S4. In this case, changing the rotational direction makes it possible to bring the shearing blade into contact with the biological tissue when the agitation blade 51a is rotated.

In the processing method of this embodiment, a certain threshold may be used instead of the rate of change or the derivative value. In this case, in the processing method of this embodiment, the first control device 53 may be set such that the decomposition product of the biological tissue is separated by the separation device 51 when the measurement value of the state over time reaches a certain threshold. In the processing method of this embodiment, a total amount (integrated value) of the measurement values of the state over time may be used instead of the rate of change or the derivative value. In this case, in the processing method of this embodiment, the first control device 53 may be set such that the decomposition product of the biological tissue is separated by the separation device 51 when the total amount satisfies a predetermined condition.

When the processing apparatus 100 of this embodiment includes a warming device or a cooling device, the first control device 53 may control the warming device or the cooling device to control the temperature of the mixed solution in the first housing container 50.

The processing apparatus of the present disclosure may include, for example, any combination of various configurations included in a third biological tissue processing apparatus, a fourth biological tissue processing apparatus, a fifth biological tissue processing apparatus, and/or a sixth biological tissue processing apparatus, which will be described below.

[Third Biological Tissue Processing Apparatus]

An aspect of the present disclosure provides a third biological tissue processing apparatus with improved extraction efficiency. The biological tissue processing apparatus of the present disclosure includes: a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a second measurement device that is configured to be capable of measuring a state of the separation device over time; and a second control device that is configured to be capable of controlling separation of a decomposition product of the biological tissue by the separation device based on the state of the separation device obtained in the measurement.

The processing apparatus of the present disclosure includes the first housing container as well as the separation device, thus making it possible to effectively separate the extracellular matrix of the biological tissue and other components (e.g., cells or cellular components) of the biological tissue from each other. Accordingly, with the processing apparatus of the present disclosure, it is possible to improve, for example, the efficiency of extracting an extract of the biological tissue. The processing apparatus of the present disclosure can also be referred to as, for example, an "apparatus for extracting an extract of a biological tissue". The processing apparatus of the present disclosure includes the second measurement device and can thereby measure the state of the separation device over time, thus making it possible to control the separation of the decomposition product of the biological tissue by the separation device based on the state of the separation device obtained through the measurement. Thus, with the processing apparatus of the present disclosure, it is possible to efficiently process a biological tissue to produce an extract of a component derived from the biological tissue.

<Embodiment>

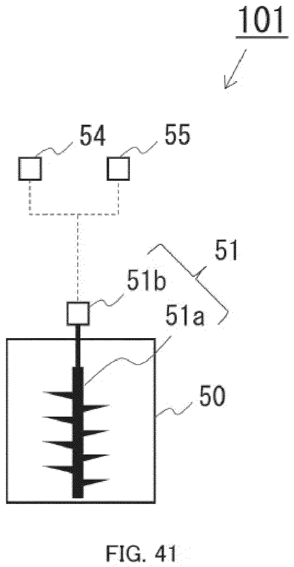

FIG. 41 is a block diagram schematically illustrating an example of a processing apparatus of this embodiment. As shown in FIG. 41, a processing apparatus 101 of this embodiment includes a first housing container 50, a separation device 51, a second measurement device 54, and a second control device 55. The separation device 51 includes an agitation blade 51a and a motor (driving unit) 51b. The agitation blade 51a of the separation device 51 is disposed in the first housing container 50. The second measurement device 54 is disposed outside the first housing container 50 and is electrically connected to the second control device 55. The second measurement device 54 is electrically connected to the motor 51b of the separation device 51 and is configured to be capable of measuring the amount of electric current used and/or the amount of power used of the motor 51b. The second control device 55 is electrically connected to the motor 51b of the separation device 51 and is configured to be capable of controlling the separation by the separation device 51 by controlling the rotation of the motor 51b.

The first housing container 50 and the separation device 51 are the same as those of the second biological tissue processing apparatus 100 above, and the descriptions thereabout are employed.

(Second Measurement Device)

The second measurement device 54 is configured to be capable of measuring the state of the separation device 51 over time. The state of the separation device 51 means a state that can be evaluated based on the physical properties of the separation device 51, and examples thereof include the amount of electric current used, the amount of power used, and the like of the motor 51b included in the separation device 51. The setting of the second measurement device 54 can be determined as appropriate in accordance with the state of the separation device 51 to be measured, and the second measurement device 54 may be, for example, an ammeter, a wattmeter, or the like.

The agitation blade 51a receives viscosity drag of the mixed solution, and the higher the viscosity is, the higher drag the agitation blade 51a receives. When the agitation blade 51a receives the drag, the motor 51b needs a larger amount of the electric current used and/or a larger amount of power used. Accordingly, the second measurement device 54 can use the amount of electric current used and/or the amount of power used as indicators for indirectly understanding the tendency of the viscosity of the mixed solution.

When the separation device 51 is another separation device that includes a homogenizer, a shear pump, an in-line mixer, a homogenization pump, or the like instead of the agitation blade 51a, the amount of electric current used, the amount of power used, or the like of a driving unit included in the separation device may be used as the state of the separation device. The setting of the second measurement device can be determined as appropriate in accordance with the state of the separation device to be measured, and the second measurement device may be, for example, an ammeter, a wattmeter, or the like.

(Second Control Device)

The second control device 55 is configured to be capable of controlling the separation of the decomposition product of the biological tissue by the separation device 51 based on the state of the separation device 51 obtained in the measurement above. For example, a controller such as an analog control device (e.g., a microcomputer, an operational amplifier, or the like) or a digital control device that includes a logical circuit can be used as the second control device 55. The second control device 55 can control separation of the decomposition product of the biological tissue by the separation device 51 based on, for example, a change in the state of the separation device 51 over time. The second control device 55 can control the separation of the decomposition product of the biological tissue by the separation device 51 by controlling the rotation rate, the rotational torque, the rotational direction, and/or the rotational pattern of the agitation blade 51a via the rotation (driving) of the motor 51b of the separation device 51. Note that the separation of the decomposition product of the biological tissue by the separation device 51 can be controlled by changing the shape and/or the number of the agitation blade 51a.

The second control device 55 is electrically connected to the second measurement device 54, that is, connected to the second measurement device 54 via a cable, but need only be configured to be capable of acquiring the results of measurement of the state of the separation device 51 by the second measurement device 54, and may be connected thereto wirelessly.

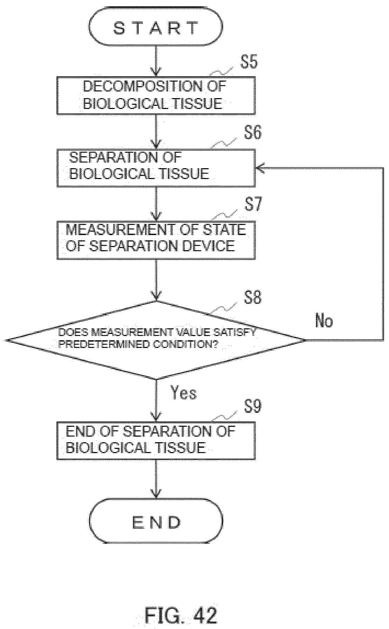

Next, an example of a processing method conducted in the processing apparatus 101 of this embodiment will be described with reference to a flowchart illustrated in FIG. 42. As shown in FIG. 42, in the processing method of this embodiment, the processing is conducted in the order of S5 (decomposition), S6 (separation of the biological tissue), S7 (measurement of the state of the separation device), S8 (determination of whether or not the measurement value satisfies a predetermined condition), and S9 (finishing of the separation of the biological tissue).

Prior to the process S5, a mixed solution obtained by mixing a biological tissue and a solution containing an enzyme is housed in the first housing container 50. The biological tissue and the solution containing the enzyme may be separately introduced into the first housing container 50. In the process S5, the extracellular matrix of the biological tissue in the mixed solution is decomposed with the enzyme by reacting the enzyme and the biological tissue in the mixed solution in the first housing container 50.

Next, in the process S6, the decomposition product of the biological tissue is separated by rotating the agitation blade 51a of the separation device 51. The conditions for the separation by the separation device 51 can be determined in accordance with the type of the biological tissue.

In the process S7, the second measurement device 54 measures the state of the separation device 51 over time. Thus, the measurement value of the state of the separation device 51 measured over time is obtained. The second measurement device 54 may, for example, calculate, from the measurement value of the state over time, the rate of change in or the derivative value of the measurement value at a predetermined time.

In the process S8, it is determined whether or not the measurement value satisfies a predetermined condition. The predetermined condition may be, for example, a fixed condition such as a certain value or a variable condition calculated through a computation. When the measurement value satisfies the predetermined condition, that is, in the case of "Yes", the processing method of this embodiment proceeds to the process S9. On the other hand, when the measurement value does not satisfy the predetermined condition, that is, in the case of "No", the processing method of this embodiment returns to the process S6. Specifically, when the separation by the separation device 51 progresses, the state of the separation device 51 becomes constant. Therefore, a change in the state of the separation device 51 can be evaluated based on, for example, the rate of change in or the derivative value of the measurement value. In an example in which the predetermined condition is a condition related to the rate of change in or the derivative value of the measurement value, when the rate of change or the derivative value is, for example, smaller than or equal to a certain value or substantially zero, it is determined in the process S8 that the separation of the biological tissue in the first housing container 50 is finished, and the processing method of this embodiment proceeds to the process S9. On the other hand, when the rate of change or the derivative value is not smaller than or equal to a certain value or substantially zero, it is determined in the process S8 that the separation of the biological tissue in the first housing container 50 has not been finished yet, and the processing method of this embodiment returns to the process S6.

In the process S9, the second control device 55 controls the separation device 51 to allow the motor 51b to finish the rotation of the agitation blade 51a and to reduce the rotation rate of the agitation blade 51a, thereby finishing the separation of the decomposition product of the biological tissue.

The processing method of this embodiment prevents the separation of the decomposition product of the biological tissue by the separation device 51 from being continued for an unnecessarily long period of time, and the separation of the decomposition product of the biological tissue by the separation device 51 can be finished at an appropriate timing. Accordingly, with the processing method of this embodiment, it is possible to efficiently process a biological tissue to produce an extract of a component derived from the biological tissue.

In this embodiment, the measurement by the second measurement device 54 and the control by the second control device 55 are used to control the finishing of the separation of the decomposition product, but the present disclosure is not limited thereto, and they may be used to control the start of the separation of the decomposition product.

The processing apparatus of the present disclosure may include, for example, any combination of various configurations included in the second biological tissue processing apparatus above, a fourth biological tissue processing apparatus, which will be described below, a fifth biological tissue processing apparatus, which will be described below, and/or a sixth biological tissue processing apparatus, which will be described below.

[Fourth Biological Tissue Processing Apparatus]

An aspect of the present disclosure provides a fourth biological tissue processing apparatus with improved extraction efficiency. The biological tissue processing apparatus of the present disclosure includes: a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a channel that is configured to allow the mixed solution in the first housing container to be circulated therethrough; a liquid feeding device that is configured to be capable of feeding the mixed solution in the first housing container via the channel; a third measurement device that is configured to be capable of measuring a state of the mixed solution in the channel over time; and a third control device that is configured to be capable of controlling liquid feeding by the liquid feeding device based on the state of the mixed solution in the channel obtained in the measurement.

The processing apparatus of the present disclosure includes the first housing container as well as the separation device, thus making it possible to effectively separate the extracellular matrix of the biological tissue and other components (e.g., cells or cellular components) of the biological tissue from each other. Accordingly, with the processing apparatus of the present disclosure, it is possible to improve, for example, the efficiency of extracting an extract of the biological tissue. The processing apparatus of the present disclosure can also be referred to as, for example, an "apparatus for extracting an extract of a biological tissue". The processing apparatus of the present disclosure includes a channel configured to be capable of circulating the mixed solution in the first housing container, and a liquid feeding device configured to be capable of feeding the mixed solution in the first housing container via the channel, thus making it possible to mix the mixed solution in the first housing container, thereby efficiently decomposing the biological tissue, and to efficiently separate the decomposition product of the biological tissue using the separation device. The processing apparatus of the present disclosure includes the third measurement device and can thereby measure the state of the mixed solution in the channel over time, thus making it possible to control the liquid feeding by the liquid feeding device based on the state of the mixed solution in the channel obtained through the measurement. Thus, with the processing apparatus of the present disclosure, it is possible to efficiently process a biological tissue to produce an extract of a component derived from the biological tissue.

<Embodiment>

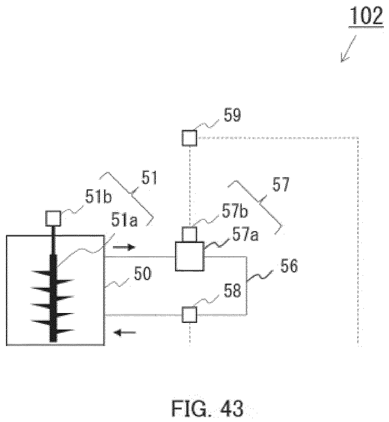

FIG. 43 is a block diagram schematically illustrating an example of a processing apparatus of this embodiment. As shown in FIG. 43, a processing apparatus 102 of this embodiment includes a first housing container 50, a separation device 51, a channel 56, a liquid feeding device 57, a third measurement device 58, and a third control device 59. The channel 56 is in communication with two different portions of the first housing container 50 such that a solution can pass. The liquid feeding device 57 and the third measurement device 58 are disposed in the channel 56. The liquid feeding device 57 includes a pump 57a and a motor (driving unit) 57b. The third measurement device 58 is electrically connected to the third control device 59. The third control device 59 is electrically connected to the motor 57b of the liquid feeding device 57 and is configured to be capable of controlling the liquid feeding by the liquid feeding device 57 by controlling the liquid feeding amount (ejection amount), the rotation rate, and/or the rotational torque of the motor 57b.
The first housing container 50 and the separation device 51 are the same as those of the second biological tissue processing apparatus above, and the descriptions thereabout are employed.

(Channel)

The channel 56 is configured to be capable of circulating the mixed solution in the first housing container 50. The channel 56 may be, for example, a channel (e.g., a tube), etc., made of a metal, plastic, resin, or the like.

(Liquid Feeding Device)

The liquid feeding device 57 is configured to be capable of feeding the mixed solution in the first housing container 50 via the channel 56. The liquid feeding device 57 includes the pump 57a and the motor (driving unit) 57b. The pump 57a may be, for example, a shear pump, a homogenization pump, or the like. Note that when the pump 57a is a shear pump, a homogenization pump, or the like, the liquid feeding device 57 may, for example, also serve as the separation device 51. In this case, the first housing container 50 may optionally include an agitation device instead of the separation device 51 and be configured to be capable of agitating a content. The agitation device need only be configured to be capable of agitating a content (mixed solution), and can be configured using, for example, an agitation blade provided with no shearing blade, which will be described below.

(Third Measurement Device)

The third measurement device 58 is configured to be capable of measuring the state of the mixed solution in the channel 56 over time. The state of the mixed solution in the channel 56 means a state that can be evaluated based on the physical properties of the mixed solution, and examples thereof include the flow rate, flow speed, viscosity, and/or turbidity of the mixed solution. The setting of the third measurement device 58 can be determined as appropriate in accordance with the state of the mixed solution in the channel 56 to be measured, and the third measurement device 58 may be, for example, a flow meter, a current meter, a viscometer, a turbidimeter, or the like.

(Third Control Device)

The third control device 59 is configured to be capable of controlling the liquid feeding by the liquid feeding device 57 based on the state of the mixed solution in the channel 56 obtained in the measurement above. For example, a controller such as an analog control device (e.g., a microcomputer, an operational amplifier, or the like) or a digital control device that includes a logical circuit can be used as the third control device 59. The third control device 59 can control the liquid feeding by the liquid feeding device 57 based on, for example, a change in the state of the mixed solution in the channel 56 over time. The third control device 59 can control the liquid feeding by the liquid feeding device 57 by controlling the liquid feeding amount (ejection amount), the rotation rate, and/or the rotational torque of the pump 57b of the liquid feeding device 57 via the rotation (driving) of the motor 57b of the liquid feeding device 57. Note that the liquid feeding by the liquid feeding device 57 may be controlled by changing the number of the liquid feeding device 57, the route of the channel 56, selection of a predetermined channel (if a plurality of channels are included), the degree of opening/closing of a valve disposed in the channel 56, and the like.

The third control device 59 is, for example, electrically connected to the third measurement device 58, that is, connected to the third measurement device 58 via a cable, but need only be configured to be capable of acquiring the results of measurement of the state of the mixed solution in the channel 56 by the third measurement device 58, and may be connected thereto wirelessly or via a cable.

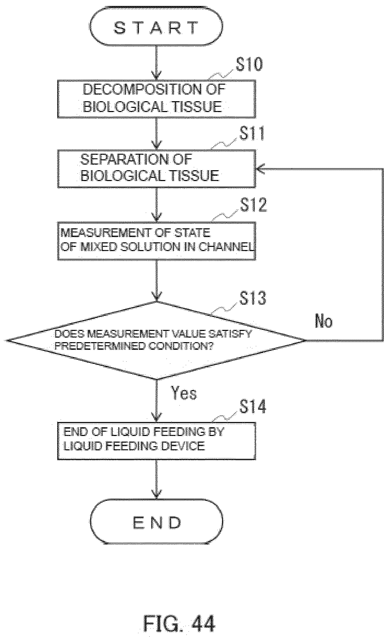

Next, an example of a processing method conducted in the processing apparatus 102 of this embodiment will be described with reference to a flowchart illustrated in FIG. 44. As shown in FIG. 44, in the processing method of this embodiment, the processing is conducted in the order of S10 (decomposition), S11 (separation of the biological tissue), S12 (measurement of the state of the mixed solution in the channel), S13 (determination of whether or not the measurement value satisfies a predetermined condition), and S14 (finishing of the liquid feeding by the liquid feeding device).

Prior to the process S10, a mixed solution obtained by mixing a biological tissue and a solution containing an enzyme is housed in the first housing container 50. The biological tissue and the solution containing the enzyme may be separately introduced into the first housing container 50. In the process S10, the extracellular matrix of the biological tissue in the mixed solution is decomposed with the enzyme by reacting the enzyme and the biological tissue in the mixed solution in the first housing container 50. The liquid feeding device 57 feeds the mixed solution in the first housing container 50 via the channel 56.

Next, in the process S11, the decomposition product of the biological tissue is separated by rotating the agitation blade 51a of the separation device 51. The conditions for the separation by the separation device 51 can be determined in accordance with the type of the biological tissue.

In the process S12, the third measurement device 58 measures the state of the mixed solution in the channel 56 over time. Thus, the measurement value of the state of the mixed solution in the channel 56 over time is obtained. The third measurement device 58 may, for example, calculate, from the measurement value of the state over time, the rate of change in or the derivative value of the measurement value at a predetermined time.

In the process S13, it is determined whether or not the measurement value satisfies a predetermined condition. The predetermined condition may be, for example, a fixed condition such as a certain value or a variable condition calculated through a computation. When the measurement value satisfies the predetermined condition, that is, in the case of "Yes", the processing method of this embodiment proceeds to the process S 14. On the other hand, when the measurement value does not satisfy the predetermined condition, that is, in the case of "No", the processing method of this embodiment returns to the process S 11. Specifically, when the separation by the separation device 51 progresses, the state of the mixed solution in the channel 56 becomes constant. Therefore, a change in the state of the mixed solution in the channel 56 can be evaluated based on, for example, the rate of change in or the derivative value of the measurement value. In an example in which the predetermined condition is a condition related to the rate of change in or the derivative value of the measurement value, when the rate of change or the derivative value is, for example, smaller than or equal to a certain value or substantially zero, it is determined in the process S13 that the separation of the biological tissue in the first housing container 50 is finished, and the processing method of this embodiment proceeds to the process S14. On the other hand, when the rate of change or the derivative value is not smaller than or equal to a certain value or substantially zero, it is determined in the process S13 that the separation of the biological tissue in the first housing container 50 has not been finished yet, and the processing method of this embodiment returns to the process S9.

In the process S14, the third control device 59 controls the liquid feeding device 57 to finish the liquid feeding by the liquid feeding device 57. The liquid feeding by the liquid feeding device 57 is finished by controlling the liquid feeding device 57 to allow the motor 57b to finish the rotation of the pump 57a and reduce the rotation rate of the pump 57a.

The processing method of this embodiment prevents the liquid feeding by the liquid feeding device 57 from being continued for an unnecessarily long period of time, and the liquid feeding by the liquid feeding device 57 can be finished at an appropriate timing. Accordingly, with the processing method of this embodiment, it is possible to efficiently process a biological tissue to produce an extract of a component derived from the biological tissue.

In this embodiment, the measurement by the third measurement device 58 and the control by the third control device 59 are used to control the finishing of the liquid feeding, but the present disclosure is not limited thereto, and they may be used to control the start of the liquid feeding.

The processing apparatus of the present disclosure may include, for example, any combination of various configurations included in the second biological tissue processing apparatus above, the third biological tissue processing apparatus above, a fifth biological tissue processing apparatus, which will be described below, and/or a sixth biological tissue processing apparatus, which will be described below.

[Fifth Biological Tissue Processing Apparatus]

An aspect of the present disclosure provides a fifth biological tissue processing apparatus with improved extraction efficiency. The biological tissue processing apparatus of the present disclosure includes: a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a solid-liquid separation device that is configured to be capable of conducting solid-liquid separation of the extract; a second housing container that is configured to be capable of housing a solid fraction obtained through the solid-liquid separation of the extract; a fourth measurement device that is configured to be capable of measuring a state of the solid fraction in the second housing container over time; and a fourth control device that is configured to be capable of controlling the solid-liquid separation of the extract by the solid-liquid separation device based on the state of the solid fraction obtained in the measurement.

The processing apparatus of the present disclosure includes the first housing container as well as the separation device, thus making it possible to effectively separate the extracellular matrix of the biological tissue and other components (e.g., cells or cellular components) of the biological tissue from each other. Accordingly, with the processing apparatus of the present disclosure, it is possible to improve, for example, the efficiency of extracting an extract of the biological tissue. The processing apparatus of the present disclosure can also be referred to as, for example, an "apparatus for extracting an extract of a biological tissue". The processing apparatus of the present disclosure includes a solid-liquid separation device configured to be capable of conducting the solid-liquid separation of the extract, and a second housing container configured to be capable of housing a solid fraction obtained through the solid-liquid separation of the extract, thus making it possible to collect the solid fraction obtained through the solid-liquid separation of the extract. Also, the processing apparatus of the present disclosure includes the fourth measurement device and can thereby measure the state of the solid fraction in the second housing container over time, thus making it possible to control the solid-liquid separation of the extract by the solid-liquid separation device based on the state of the solid fraction obtained through the measurement. Thus, with the processing apparatus of the present disclosure, it is possible to efficiently process a biological tissue to produce an extract of a component derived from the biological tissue.

<Embodiment>

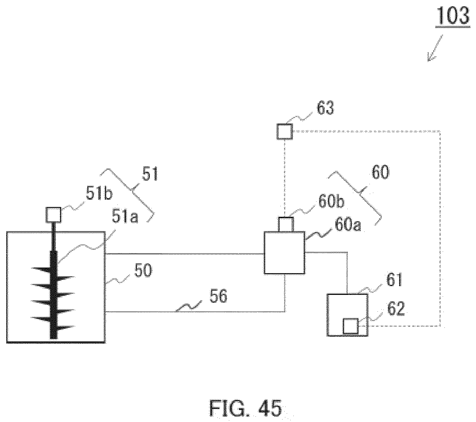

FIG. 45 is a block diagram schematically illustrating an example of a processing apparatus of this embodiment. As shown in FIG. 45, a processing apparatus 103 of this embodiment includes a first housing container 50, a separation device 51, a solid-liquid separation device 60, a second housing container 61, a fourth measurement device 62, a fourth control device 63, and a channel 56. The channel 56 is in communication with two different portions of the first housing container 50 such that a solution can pass. The solid-liquid separation device 60 is disposed in the channel 56. The solid-liquid separation device 60 includes a screw press 60a and a motor (driving unit) 60b and is configured to be capable of transferring the solid fraction separated by the screw press 60a to the second housing container 61. The fourth measurement device 62 is disposed in the second housing container 61 and is electrically connected to the fourth control device 63. The fourth control device 63 is electrically connected to the motor 60b of the solid-liquid separation device 60 and is configured to be capable of controlling the solid-liquid separation by the solid-liquid separation device 60 by controlling the rotation of the motor 60b.

The first housing container 50 and the separation device 51 are the same as those of the second biological tissue processing apparatus above, and the descriptions thereabout are employed. The channel 56 is the same as that of the fourth biological tissue processing apparatus above, and the description thereabout is employed.

(Solid-Liquid Separation Device)

The solid-liquid separation device 60 is configured to be capable of conducting the solid-liquid separation of the extract. The solid-liquid separation device 60 includes a screw press 60a and a motor (driving unit) 60b. The solid-liquid separation device 60 may include another solid-liquid separation machine, filter, or the like instead of the screw press 60a. Examples of the solid-liquid separation machine include a screw conveyor, a roller press, a belt screen, a belt press, a filter press, a cyclone, a dehydrator, a sedimentation separator, a centrifuge, an ultrasonic separator, and the like. The dehydrating machine may be, for example, a multi-disk dehydrator, a vacuum dehydrator, a centrifugal dehydrator, or the like. The sedimentation separator may be, for example, a precipitation tank, a coagulative precipitation tank, a precipitation device with a tilted plate, or the like. The centrifuge may be, for example, a liquid cyclone or the like. Examples of the filter include a tilted filter, filter paper, a sieving net, a mesh filter, a mesh screen, a stainless mesh, a punched plate, a bar screen, a wire screen, a mesh filter, a disk filter, a column filter, a yarn-wound filter, a non-woven filter, a membrane, and the like.

The tilted filter means a filter disposed in a tilted orientation relative to a horizontal direction. The tilt angle may be, for example, 45 degrees or more relative to the horizontal direction. In the solid-liquid separation device 60, a separated solid fraction can be moved along a tilted surface due to the tilted filter being employed, thus making it possible to suppress filter clogging in the tilted filter caused by the solid fraction. Accordingly, with the tilted filter, it is possible to more efficiently conduct solid-liquid separation. The tilted filter may be provided with a plurality of slits on its surface. Providing the tilted filter with a plurality of slits makes it possible to more efficiently conduct solid-liquid separation. In order to achieve favorable draining, it is preferable to use a hydrophilic tilted filter as the tilted filter. In order to achieve favorable draining and prevent blockage, it is preferable to spray the extract solution to supply minute particles thereof to the tilted filter.

(Second Housing Container)

The second housing container 61 is configured to be capable of housing a solid fraction obtained through the solid-liquid separation of the extract. The second housing container 61 is, for example, a container (e.g., a tank) made of a material such as a metal, plastic, or resin. The capacity of the second housing container 61 can be determined as appropriate in accordance with the volumes of the solid fraction to be housed.

(Fourth Measurement Device)

The fourth measurement device 62 is configured to be capable of measuring the state of the solid fraction in the second housing container 61 over time. The state of the solid fraction in the second housing container 61 means a state that can be evaluated based on the physical properties of the solid fraction, and examples thereof include the amount of a liquid contained in the solid fraction, the amount of a solid contained in the solid fraction, and/or the ratio between the liquid amount and the solid amount. The liquid amount and/or the solid amount may be, for example, the weight, the mass, the volume (capacity), and the like. The setting of the fourth measurement device 62 can be determined as appropriate in accordance with the state of the solid fraction to be measured, and the fourth measurement device 62 may be, for example, a weight meter, a mass meter, a volumeter (volumenometer), or the like.

(Fourth Control Device)

The fourth control device 63 is configured to be capable of controlling the solid-liquid separation of the extract by the solid-liquid separation device 60 based on the state of the solid fraction obtained in the measurement above. The fourth control device 63 can control the solid-liquid separation of the extract by the solid-liquid separation device 60 based on, for example, a change in the state of the solid fraction over time. The fourth control device 63 can control the solid-liquid separation of the extract by the solid-liquid separation device 60 by controlling the rotation rate of the screw press 60a via the rotation (driving) of the motor (driving unit) 60b. When the solid-liquid separation device 60 includes a roller press, a belt screen, and/or a cyclone instead of the screw conveyor 60a, the solid-liquid separation of the extract by the solid-liquid separation device 60 can be controlled by controlling the rotation rate of the roller press, the belt screen, and/or the cyclone via the rotation (driving) of the motor (driving unit) 60b.

The fourth control device 63 is electrically connected to the fourth measurement device 62, that is, connected to the fourth measurement device 62 via a cable, but need only be configured to be capable of acquiring the results of measurement of the state of the solid fraction in the second housing container 61 by the fourth measurement device 62, and may be connected thereto wirelessly.

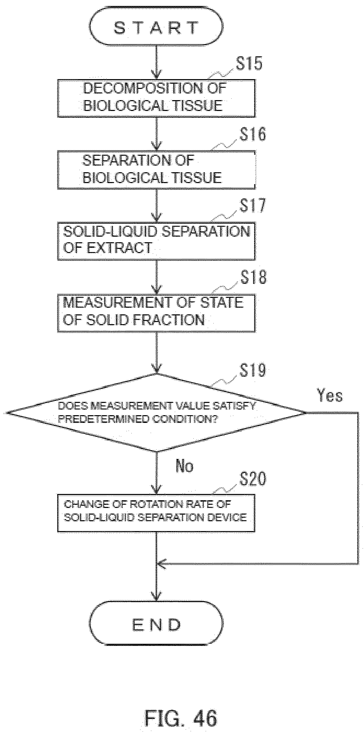

Next, an example of a processing method conducted in the processing apparatus 103 of this embodiment will be described with reference to a flowchart illustrated in FIG. 46. As shown in FIG. 46, in the processing method of this embodiment, the processing is conducted in the order of the process S15 (decomposition), the process S16 (separation), S17 (solid-liquid separation of the extract), S18 (measurement of the state of the solid fraction), S19 (determination of whether or not the measurement value satisfies a predetermined condition), and S20 (change of the rotation rate of the solid-liquid separation device).

Prior to the process S15, a mixed solution obtained by mixing a biological tissue and a solution containing an enzyme is housed in the first housing container 50. The biological tissue and the solution containing the enzyme may be separately introduced into the first housing container 50. In the process S15, the extracellular matrix of the biological tissue in the mixed solution is decomposed with the enzyme by reacting the enzyme and the biological tissue in the mixed solution in the first housing container 50.

Next, in the process S16, the decomposition product of the biological tissue is separated in the first housing container 50 using the separation device 51.

In the process S17, the extract obtained through the separation is introduced into the solid-liquid separation device 60 via the channel 56. In the process S17, the solid-liquid separation of the extract is conducted using the solid-liquid separation device 60. In the process S17, the separated solid fraction is transferred into the second housing container 61. In the process S17, the separated liquid fraction is returned to the channel 56.

In the process S18, the fourth measurement device 62 measures the state of the solid fraction in the second housing container 61. Thus, the measurement value of the state of the solid fraction in the second housing container 61 is obtained. The fourth measurement device 62 may obtain, for example, the amount of a liquid contained in the solid fraction, the amount of a solid contained in the solid fraction, and/or the ratio between the liquid amount and the solid amount.

In the process S19, it is determined whether or not the measurement value satisfies a predetermined condition. The predetermined condition may be, for example, a fixed condition such as a certain value or a variable condition calculated through a computation. When the measurement value satisfies the predetermined condition, that is, in the case of "Yes", the processing method of this embodiment is finished. On the other hand, when the measurement value does not satisfy the predetermined condition, that is, in the case of "No", the processing method of this embodiment proceeds to the process S20. In an example in which the predetermined condition is a condition related to the amount of a liquid contained in the solid fraction, when the amount of a liquid contained in the solid fraction is, for example, greater than or equal to a certain value, it is determined in the process S19 that the amount of a liquid contained in the solid fraction is excessive, and the processing method of this embodiment proceeds to the process S20. On the other hand, when the amount of a liquid contained in the solid fraction is not greater than or equal to a certain value, it is determined in the process S19 that the amount of a liquid contained in the solid fraction is appropriate, and the processing method of this embodiment is finished.

In the process S20, the fourth control device 63 controls the solid-liquid separation device 60 to allow the motor 60b to change the rotation rate of the screw press 60a. Specifically, when the amount of a liquid contained in the solid fraction is greater than or equal to a certain value, the fourth control device 63 reduces the amount of a liquid brought into the second housing container 61 by reducing the rotation rate of the motor 60b, and thus reduces the amount of a liquid contained in the solid fraction in the second housing container 61. When the amount of a liquid contained in the solid fraction is less than the certain value, the fourth control device 63 improves the processing speed of the solid-liquid separation by increasing the rotation rate of the motor 60b.

In the processing method of this embodiment, an excessive amount of a liquid does not remain in the solid fraction, thus making it possible to obtain a solid fraction containing a liquid in an appropriate amount (or containing substantially no liquid). Accordingly, with the processing method of this embodiment, it is possible to efficiently process a biological tissue to produce an extract of a component derived from the biological tissue.

The processing apparatus of the present disclosure may include, for example, any combination of various configurations included in the second biological tissue processing apparatus above, the third biological tissue processing apparatus above, the fourth biological tissue processing apparatus above, and/or a sixth biological tissue processing apparatus, which will be described below.

[Sixth Biological Tissue Processing Apparatus]

An aspect of the present disclosure provides a sixth biological tissue processing apparatus with improved extraction efficiency. The biological tissue processing apparatus of the present disclosure includes: a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a solid-liquid separation device that is configured to be capable of conducting solid-liquid separation of the extract; a second housing container that is configured to be capable of housing a solid fraction obtained through the solid-liquid separation of the extract; a channel that is configured to allow the extract to be circulated therethrough via the solid-liquid separation device; a liquid feeding device that is configured to be capable of feeding the extract via the channel; a fourth measurement device that is configured to be capable of measuring a state of the solid fraction in the second housing container over time; and a fifth control device that is configured to be capable of controlling supply of the extract to the solid-liquid separation device via the channel based on the state of the solid fraction obtained in the measurement.

The processing apparatus of the present disclosure includes the first housing container 50 as well as the separation device 51, thus making it possible to effectively separate the extracellular matrix of the biological tissue and other components (e.g., cells or cellular components) of the biological tissue from each other. Accordingly, with the processing apparatus of the present disclosure, it is possible to improve, for example, the efficiency of extracting an extract of the biological tissue. The processing apparatus of the present disclosure can also be referred to as, for example, an "apparatus for extracting an extract of a biological tissue". The processing apparatus of the present disclosure includes a solid-liquid separation device configured to be capable of conducting the solid-liquid separation of the extract, and a second housing container configured to be capable of housing a solid fraction obtained through the solid-liquid separation of the extract, thus making it possible to collect the solid fraction obtained through the solid-liquid separation of the extract. Also, the processing apparatus of the present disclosure includes the fourth measurement device and can thereby measure the state of the solid fraction in the second housing container over time, thus making it possible to control the supply of the extract to the solid-liquid separation device via the channel based on the state of the solid fraction obtained through the measurement. Thus, with the processing apparatus of the present disclosure, it is possible to efficiently process a biological tissue to produce an extract of a component derived from the biological tissue.

<Embodiment>

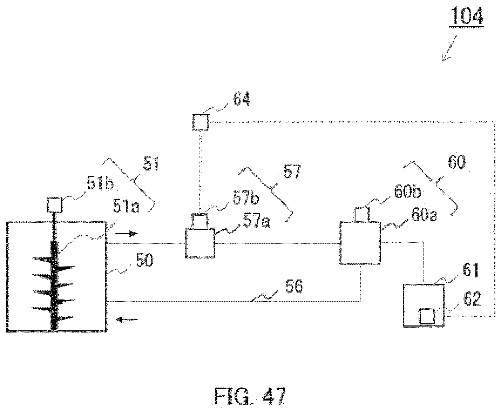

FIG. 47 is a block diagram schematically illustrating an example of a processing apparatus of this embodiment. In this example, a processing apparatus 104 of this embodiment includes a first housing container 50, a separation device 51, a solid-liquid separation device 60, a second housing container 61, a channel 56, a liquid feeding device 57, a fourth measurement device 62, and a fifth control device 64. The channel 56 is in communication with two different portions of the first housing container 50 such that a solution can pass. The solid-liquid separation device 60 and the liquid feeding device 57 are disposed in the channel 56. The fourth measurement device 62 is electrically connected to the fifth control device 64. The fifth control device 64 is electrically connected to a motor 57b of the liquid feeding device 57 and is configured to be capable of controlling the supply of the extract to the solid-liquid separation device 60 via the channel 56 by controlling the rotation of the motor 57b.

The first housing container 50 and the separation device 51 are the same as those of the second biological tissue processing apparatus above, and the descriptions thereabout are employed. The channel 56 and the liquid feeding device 57 are the same as those of the fourth biological tissue processing apparatus above, and the descriptions thereabout are employed. The solid-liquid separation device 60, the second housing container 61, and the fourth measurement device 62 are the same as those of the fifth biological tissue processing apparatus above, and the descriptions thereabout are employed.

(Fifth Control Device)

The fifth control device 64 is configured to be capable of controlling the supply of the extract to the solid-liquid separation device 60 via the channel 56 based on the state of the solid fraction obtained in the measurement above. The fifth control device 64 can control the supply of the extract to the solid-liquid separation device 60 via the channel 56 based on, for example, a change in the state of the solid fraction over time. The fifth control device 64 can control the supply of the extract to the solid-liquid separation device 60 by controlling the liquid feeding amount (ejection amount), the rotation rate, and/or the rotational torque of the pump 57b of the liquid feeding device 57 via the rotation (driving) of the motor 57b of the liquid feeding device 57. Note that the liquid feeding by the liquid feeding device 57 may be controlled by changing the number of the liquid feeding device 57, the route of the channel 56, the degree of opening/closing of a valve disposed in the channel 56, and the like.

The fifth control device 64 is electrically connected to the fourth measurement device 62, that is, connected to the fourth measurement device 62 via a cable, but need only be configured to be capable of acquiring the results of measurement of the state of the solid fraction in the second housing container 61 by the fourth measurement device 62, and may be connected thereto wirelessly.

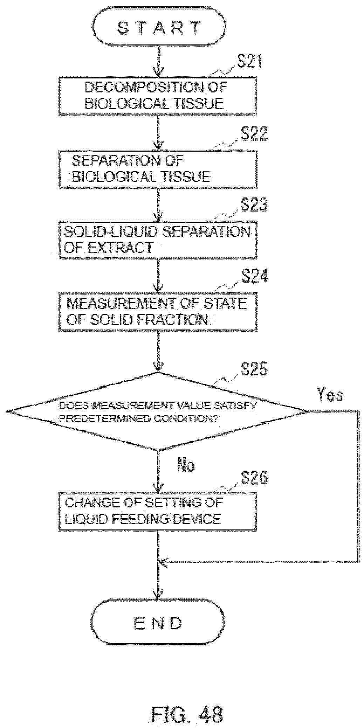

Next, an example of a processing method conducted in the processing apparatus 104 of this embodiment will be described with reference to a flowchart illustrated in FIG. 48. As shown in FIG. 48, in the processing method of this embodiment, the processing is conducted in the order of the process S21 (decomposition), the process S22 (separation), S23 (solid-liquid separation of the extract), S24 (measurement of the state of the solid fraction), S25 (determination of whether or not the measurement value satisfies a predetermined condition), and S26 (change in the setting of the liquid feeding device).

Prior to the process S21, a mixed solution obtained by mixing a biological tissue and a solution containing an enzyme is housed in the first housing container 50. The biological tissue and the solution containing the enzyme may be separately introduced into the first housing container 50. In the process S21, the extracellular matrix of the biological tissue in the mixed solution is decomposed with the enzyme by reacting the enzyme and the biological tissue in the mixed solution in the first housing container 50.

Next, in the process S22, the decomposition product of the biological tissue is separated in the first housing container 50 using the separation device 51.

In the process S23, the extract obtained through the separation is introduced into the solid-liquid separation device 60 via the channel 56. In the process S23, the solid-liquid separation device 60 is used to conduct the solid-liquid separation of the extract. In the process S23, the separated solid fraction is transferred into the second housing container 61. In the process S23, the separated liquid fraction is returned to the channel 56.

In the process S24, the fourth measurement device 62 measures the state of the solid fraction in the second housing container 61. Thus, the measurement value of the state of the solid fraction in the second housing container 61 is obtained. The fourth measurement device 62 may obtain, for example, the amount of a liquid contained in the solid fraction, the amount of a solid contained in the solid fraction, and/or the ratio between the liquid amount and the solid amount.

In the process S25, it is determined whether or not the measurement value satisfies a predetermined condition. The predetermined condition may be, for example, a fixed condition such as a certain value or a variable condition calculated through a computation. When the measurement value satisfies the predetermined condition, that is, in the case of "Yes", the processing method of this embodiment is finished. On the other hand, when the measurement value does not satisfy the predetermined condition, that is, in the case of "No", the processing method of this embodiment proceeds to the process S26. In an example in which the predetermined condition is a condition related to the amount of a liquid contained in the solid fraction, when the amount of a liquid contained in the solid fraction is, for example, greater than or equal to a certain value, it is determined in the process S25 that the amount of a liquid contained in the solid fraction is excessive, and the processing method of this embodiment proceeds to the process S26. On the other hand, when the amount of a liquid contained in the solid fraction is not greater than or equal to a certain value, it is determined in the process S25 that the amount of a liquid contained in the solid fraction is appropriate, and the processing method of this embodiment is finished.

In the process S26, the fifth control device 64 controls the liquid feeding device 57 to allow the motor 57b to change the setting of the pump 57a. Specifically, when the amount of a liquid contained in the solid fraction is greater than or equal to a certain value, the fifth control device 64 reduces the amount of a liquid brought into the solid-liquid separation device 60 by reducing the liquid feeding amount (ejection amount), the rotation rate, and/or the rotational torque of the motor 57b, and thus reduces the amount of a liquid contained in the solid fraction in the second housing container 61. When the amount of a liquid contained in the solid fraction is less than the certain value, the fifth control device 64 increases the amount of a liquid introduced into the solid-liquid separation device 60 by increasing the rotation rate of the motor 57b, and thus improves the processing speed of the solid-liquid separation.

In the processing method of this embodiment, an excessive amount of a liquid does not remain in the solid fraction, thus making it possible to obtain a solid fraction containing a liquid in an appropriate amount (or containing substantially no liquid). Accordingly, with the processing method of this embodiment, it is possible to efficiently process a biological tissue to produce an extract of a component derived from the biological tissue.

All of the above-described second biological tissue processing apparatus, third biological tissue processing apparatus, fourth biological tissue processing apparatus, fifth biological tissue processing apparatus, and sixth biological tissue processing apparatus may include a measurement device configured to be capable of measuring the temperature and/or the humidity of the environment in which the processing apparatus is disposed.

[Method for Processing Biological Tissue]

An aspect of the present disclosure provides a method for processing a biological tissue with improved extraction efficiency. The processing method of the present disclosure includes: a decomposition step of decomposing extracellular matrix of a biological tissue in a mixed solution containing the biological tissue and an enzyme; a measurement step of measuring a state of the mixed solution containing the biological tissue and the enzyme over time in the decomposition step; and a separation step of separating cells of the biological tissue and/or an extract of the biological tissue from a decomposition product of the biological tissue, wherein the separation of the decomposition product of the biological tissue is conducted based on the state of the mixed solution obtained in the measurement step.

The processing method of the present disclosure includes the decomposition step as well as the separation step, thus making it possible to effectively separate the extracellular matrix of the biological tissue and other components (e.g., cells or cellular components) of the biological tissue from each other. Accordingly, with the processing method of the present disclosure, it is possible to improve, for example, the efficiency of extracting an extract of the biological tissue. The processing method of the present disclosure can also be referred to as, for example, a "method for extracting an extract of a biological tissue". The processing method of the present disclosure includes the measurement step, thus making it possible to measure the state of the mixed solution over time as well as conduct the separation of the decomposition product of the biological tissue based on the state of the mixed solution obtained in the measurement step. Thus, with the processing method of the present disclosure, it is possible to efficiently process a biological tissue to produce an extract of a component derived from the biological tissue.

(Decomposition Step)

The decomposition step is a step of decomposing extracellular matrix of the biological tissue with an enzyme in the coexistence of the biological tissue and the enzyme. Accordingly, the decomposition step can also be referred to as, for example, an "enzymatic processing step". Since the extracellular matrix of the biological tissue is decomposed in the decomposition step, the extracellular matrix of the biological tissue and other components (e.g., cells or cellular components) of the biological tissue can be effectively separated from each other in the separation step, which will be described below.

The decomposition step can be conducted in the same manner as, for example, the decomposition step in the method for producing an extract of a biological tissue.

The decomposition step may include, for example, the decomposition of the extracellular matrix of the biological tissue through ultrasonic homogenization in addition to the decomposition of the extracellular matrix of the biological tissue with the enzyme. In the decomposition step, for example, the extracellular matrix of the biological tissue can be homogenized into small fragments through the ultrasonic homogenization. Thus, in the reaction solution during or after the ultrasonic homogenization, the biological tissue and the components derived from the biological tissue on which the enzyme can act have relatively increased surface areas compared with the reaction solution before the ultrasonic homogenization, and can thus be more efficiently decomposed with the enzyme.

The decomposition through ultrasonic homogenization may be conducted before or after the enzymatic decomposition, or simultaneously with all or a part of the enzymatic decomposition. Each of the enzymatic decomposition and the decomposition through ultrasonic homogenization can be conducted once or two or more times.

(Separation Step)

The separation step is a step of separating cells of the biological tissue and/or an extract of the biological tissue from the decomposition product of the biological tissue. It is preferable to conduct the separation step on, for example, the reaction solution containing the biological tissue and the enzyme. When the separation step is conducted during or after the decomposition step, the above-mentioned extraction is conducted on the reaction solution prepared in the decomposition step in the separation step.

When the separation step is conducted on the reaction solution containing the biological tissue and the enzyme, the enzymatic decomposition may be conducted, for example, in combination with shearing or homogenization, which will be described below. For example, when the reaction solution containing the biological tissue and the enzyme is subjected to shearing or homogenization, which will be described below, the biological tissue is sheared or homogenized, and the biological tissue and the components derived from the biological tissue on which the enzyme can act have relatively increased surface areas, resulting in more efficient enzymatic decomposition.

In the separation step, for example, cells included in the biological tissue can be mechanically separated from the biological tissue. The separation step may include a processing operation (shearing) for mechanically shearing the extracellular matrix of the biological tissue or a processing operation (homogenization) for mechanically homogenizing it because, for example, the extracellular matrix and the other components can be more efficiently separated from each other. The mechanical shearing or homogenization can be conducted using, for example, a shearing apparatus (shearing machine) or a homogenization machine (homogenization device). It is preferable that the shearing apparatus includes a shearing blade because the extracellular matrix and the other components can be more efficiently separated from each other. Specifically, the separation step includes, for example, a processing operation for mechanically shearing the extracellular matrix of the biological tissue by bringing the biological tissue into contact with an agitation blade having a shearing blade. The separation step includes, for example, a processing operation for homogenizing a reaction solution containing the biological tissue and an enzyme under agitation using a homogenizer. With this processing operation, the extracellular matrix of the biological tissue and the other components (e.g., cells or cellular components) of the biological tissue can be effectively separated from each other.

In the separation step, the setting of the mechanical shearing or homogenization can be determined as appropriate in accordance with, for example, the origin of the biological tissue, that is, the extracellular matrix contained in the biological tissue. When the biological tissue is an animal tissue, the processing operation for mechanically shearing the extracellular matrix of the biological tissue may be, for example, a processing operation for homogenizing the biological tissue using a Waring blender, a mixer, ultrasonic homogenization, or the like. When the biological tissue is a plant tissue, the processing operation for mechanically shearing the extracellular matrix of the biological tissue may be, for example, a processing operation for homogenizing the biological tissue under agitation using a homogenizer or the like. Also, the shearing machine or the homogenization machine may include, for example, a homogenizer, a shear pump, an in-line mixer homogenization pump, or the like. The operational manner of the shearing machine or the homogenization machine can be selected as appropriate in accordance with, for example, the origin of the biological tissue, and examples thereof include those using ultrasonic homogenization, pressurization, agitation, and the like.

When the biological tissue is a tissue of a tea plant (tea) or a coffee plant (coffee), the mechanical shearing may be shearing conducted using any device with a blade capable of effectively shearing the tissue of the tea plant (tea) or the coffee plant (coffee).

The temperature during the processing operation for mechanically shearing or homogenizing the extracellular matrix of the biological tissue can be set as appropriate in accordance with, for example, an enzyme used in the decomposition step. The temperature is, for example, a temperature at which the enzyme exhibits enzymatic activity, and is specifically 20°C to 70°C, 25°C to 65°C, 35°C to 60°C, 40°C to 60°C, or 45°C to 55°C.

The decomposition step and/or the separation step may be independently conducted once or a plurality of times. The number of times of conducting the decomposition step and the number of times of conducting the separation step may be the same or different. The decomposition step and the separation step may be conducted separately, and parts or all of the decomposition step and the separation step may be conducted simultaneously.

(Measurement Step)

The measurement step is a step of measuring a state of the mixed solution containing the biological tissue and the enzyme over time in the decomposition step. The state of the mixed solution may be, for example, the viscosity, pH, temperature, turbidity, color, and/or air bubble density of the mixed solution. In the measurement step, the setting of the measurement device can be selected as appropriate in accordance with the state of the mixed solution to be measured. The measurement device may be, for example, a viscometer, a pH meter, a thermometer, a turbidimeter, a colorimeter (color-difference meter), an air-bubble detector, or the like.

The separation of the decomposition product of the biological tissue is conducted based on the state of the mixed solution obtained in the measurement step. When the shearing machine or homogenization machine includes an agitation blade configured to be capable of agitating the mixed solution, the separation can be conducted by controlling the rotation rate and/or the rotational direction of the agitation blade.

When the extracellular matrix of the biological tissue in the mixed solution is decomposed with the enzyme in the decomposition step, the state of the mixed solution changes over time. The measurement value of the state of the mixed solution over time can be obtained by measuring the state of the mixed solution over time in the measurement step. For example, it is possible to obtain, from the measurement value of the state over time, the rate of change in or the derivative value of the measurement value at a predetermined time. When the rate of change or the derivative value satisfies a predetermined condition, the decomposition product of the biological tissue can be separated using the separation device 51. For example, when the rate of change or the derivative value is substantially zero, the decomposition step can be considered as being finished, and it is possible to set to conduct the separation step. This prevents the decomposition step from being continued for an unnecessarily long period of time, and the separation step can be conducted at an appropriate timing, thus making it possible to efficiently process a biological tissue to produce an extract of a component derived from the biological tissue.

With the method for producing an extract of a biological tissue, the biological tissue processing apparatus, and the method for processing a biological tissue of the present disclosure, it is possible to rapidly process wastes (e.g., wastes containing water such as used tea leaves) generated in the production process of various products (e.g., food) in the same production site (e.g., the same factory) and obtain an extract thereof. Accordingly, it is possible to reduce wastes, which would have been discarded hitherto, as well as utilize an extract obtained from wastes in various products (e.g., food). For example, when used tea leaves or the like containing water are utilized in a product instead of being discarded, it has been conventionally necessary to temporarily store the used tea leaves or the like containing water or once transport them to a place outside a factory, but there is a risk that the used tea leaves and the like containing water decay. In order to prevent the decay, the used tea leaves or the like containing water need to be dried, but a lot of energy and cost are required to dry the used tea leaves or the like containing water. Also, it is very costly to transport the used tea leaves or the like containing water to a place outside a factory. In contrast, by installing the biological tissue processing apparatus of the present disclosure in a site of producing products, used tea leaves or the like containing water generated in the process for producing the products can be processed as they are, thus making it possible to reduce the cost.

Examples

[Outline of Processing Flow]

A processing flow illustrative of the production method of the present disclosure is shown in FIG. 9. First, tea leaves or used tea leaves are subjected to decomposition or separation. Next, the processed product obtained through the processing above is subjected to primary solid-liquid separation using a sieving net, a cyclone, a filter, or the like. Venous cellulose fibers (including cellulose microfibers and/or cellulose nanofibers) can be obtained through this separation. Next, a processed product obtained through the primary solid-liquid separation can be further subjected to secondary solid-liquid separation using a sieving net, a cyclone, a filter, or the like to separate it into a tea-leaf slurry or a used-tea-leaf slurry and a solution. Then, the separated tea-leaf slurry or used-tea-leaf slurry can be subjected to squeezing (wringing) to separate it into a residue (solid) and a solution. Cellulose fibers can be further obtained from the tea-leaf slurry or used-tea-leaf slurry. For example, venous cellulose fibers can be mainly obtained through the primary solid-liquid separation, whereas cellulose fibers other than venous cellulose fibers (i.e., cellulose fibers finer than venous cellulose fibers) can be mainly obtained through the secondary solid-liquid separation. Note that the residue (solid) can be further separated.

The processed product separated through the primary solid-liquid separation and the processed product separated through the secondary solid-liquid separation may have different sizes. For example, the processed product (e.g., venous cellulose fibers) separated through the primary solid-liquid separation may be larger than the processed product separated through the secondary solid-liquid separation. For this reason, in the primary solid-liquid separation, a sieving net or the like having larger meshes than that used in the secondary solid-liquid separation may be used.

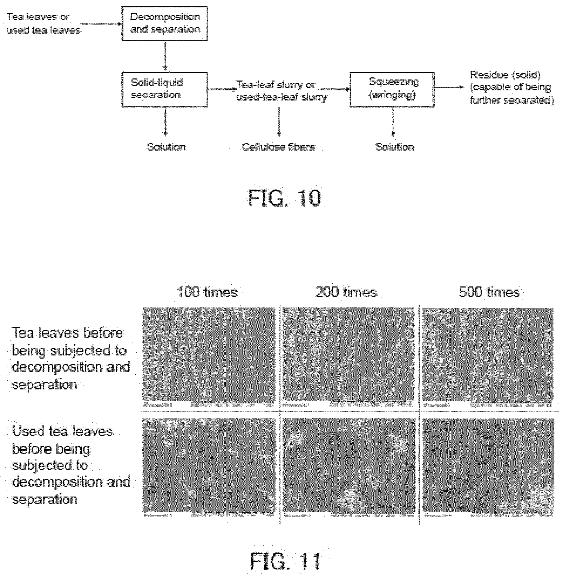

The following examples were implemented in accordance with a simplified processing flow (FIG. 10) that is based on the process flow above. First, tea leaves or used tea leaves were subjected to decomposition or separation. Next, a processed product obtained through the processing above was subjected to solid-liquid separation using a sieving net and was thus separated into a tea-leaf slurry or used-tea-leaf slurry and a solution. Then, the separated tea-leaf slurry or used-tea-leaf slurry was subjected to squeezing (wringing) to separate a solution. Cellulose fibers (also including venous cellulose fibers) were obtained from the separated tea-leaf slurry or used-tea-leaf slurry.

[Example 1]

(Decomposition and Separation of Tea Leaves)

40 L of water was placed in a container with a capacity of 50 L, and the water was heated to a temperature of 45°C using a liquid heating heater (Model Number: WPS-110, Kashima Co., Ltd.). 120 ml (corresponding to 0.3% of 40 L, the volume of the water) of cellulase was added to and mixed with the water. Next, 2 kg of the tea leaves were added to the water and were immersed therein for 24 hours (decomposition). The pH of the thus prepared reaction solution was set to 6.64. The tea leaves used were prepared as follows: raw leaves just after being plucked from a tea tree were subjected to enzyme deactivation for 60 seconds by supplying steam at 100°C thereto under agitation.

The immersed tea leaves were homogenized under agitation at 4,500 rpm for 45 minutes using a homogenizer (Auto Mixer Model 40, PRIMIX Corporation) (separation). The temperature of the water was kept at 45°C by heating the water using the liquid heating heater. Thus, a processed product of the tea leaves was obtained.

(Obtainment of Extract of Tea Leaves)

The processed product of the tea leaves was poured onto a commercially available sieving net (mesh: about 1.5 mm square). A tea-leaf slurry remained on the net, and a tea-leaf solution passed through the net and fell into a receiving container. Thus, the tea-leaf slurry and the tea-leaf solution were separated and obtained. Then, the tea-leaf slurry was squeezed using an oil press and was then filtered, and thus a squeezed solution of the tea-leaf slurry was obtained. That is to say, the tea-leaf solution, the tea-leaf slurry, and the squeezed solution of the tea-leaf slurry were obtained as extracts of the tea leaves.

[Example 2]

(Decomposition and Separation of Used Tea Leaves)

Used tea leaves obtained in the production of a common tea beverage were dried again and were then subjected to decomposition and separation in the same manner as in Example 1. This example was different from Example 1 only in that used tea leaves were used instead of tea leaves.

(Obtainment of Extract of Used Tea Leaves)

Similarly to Example 1, a used-tea-leaf solution, a used-tea-leaf slurry, and a squeezed solution of the used-tea-leaf slurry were obtained from the processed product of the used tea leaves as extracts of the used tea leaves. This example was different from Example 1 only in that a processed product of used tea leaves was used instead of a processed product of tea leaves.

[Comparative Example 1]

(Decomposition of Tea Leaves)

The tea leaves were subjected to decomposition in the same manner as in Example 1. This example was different from Example 1 only in that the separation was not conducted.

(Obtainment of Extract of Tea Leaves)

A processed product of the tea leaves was poured onto a commercially available sieving net (mesh: about 1.5 mm square). The tea leaves remained on the net, and a tea-leaf solution passed through the net and fell into a receiving container. Thus, the tea leaves and the tea-leaf solution were separated and obtained. Then, the tea leaves were squeezed using an oil press and were then filtered, and thus a squeezed solution of the tea leaves was obtained. That is to say, the tea-leaf solution, the tea leaves, and the squeezed solution of the tea leaves were obtained as extracts of the tea leaves.

[Measurement of Protein Content in Each Extract]

The protein content and the amino acid content in each of the extracts obtained in Example 1, Example 2, and Comparative Example 1 were measured. The measurement was entrusted to Bureau Veritas FEC Co., Ltd., which is an authorized inspection agency of the Ministry of Health, Labour and Welfare. Specifically, the protein content was measured using the Kjeldahl method, the amino acid content (excluding tryptophan) was measured using the PicoTag amino acid analysis method, and the tryptophan content was measured using high-performance liquid chromatography (the same applies hereinafter).
The protein transfer ratio (%) was calculated using the following equation.

(Protein transfer ratio (%)) = (protein content in each extract)/(total protein content) × 100

In the equation, the "protein content in each extract" refers to a value calculated by multiplying the weight of each of the extracts of Examples 1 and 2 and Comparative Example 1 by the protein concentration (wt%) obtained through the Kjeldahl method. The "total protein content" refers to a value calculated by adding up the protein contents in the extracts of Example 1 or 2 or Comparative Example 1. For example, in Example 1, the calculated value refers to a value calculated by adding up the protein contents in the tea-leaf solution, the tea-leaf slurry, and the squeezed solution of the tea-leaf slurry.
As a result, compared with the tea-leaf solution of Comparative Example 1, the protein content, the amino acid content, and the protein transfer ratio were generally higher in the tea-leaf solution of Example 1 and the tea-leaf solution of Example 2 (Tables 1 and 2 below). In particular, the tea-leaf solution of Example 1 and the tea-leaf solution of Example 2 contained a large amount of tryptophan (Table 2 below).
Also, compared with the squeezed solution of the tea leaves of Comparative Example 1, the squeezed solution of the tea leaves of Example 1 and the squeezed solution of the used tea leaves of Example 2 indicated a similar tendency in part (Tables 1 and 2 below).
Meanwhile, compared with the tea leaves of Comparative Example 1, the protein content and the amino acid content were generally lower in the tea-leaf slurry of Example 1 and the used-tea-leaf slurry of Example 2 (Tables 1 and 2 below).
Accordingly, it was found that large amounts of proteins and amino acids transferred from the tea leaves or the used tea leaves into the water by conducting both the decomposition and the separation. In particular, it was found that a large amount of tryptophan transferred from the tea leaves or the used tea leaves into the water.
Compared with the total protein content (the sum of the protein contents in the tea-leaf solution, the tea leaves, and the squeezed solution of the tea leaves) of Comparative Example 1, the total protein content (the sum of the protein contents in the tea-leaf solution, the tea-leaf slurry, and the squeezed solution of the tea-leaf slurry) of Example 1 and the total protein content (the sum of the protein contents in the used-tea-leaf solution, the used-tea-leaf slurry, and the squeezed solution of the used-tea-leaf slurry) of Example 2 were smaller. It is considered that the reason for this is that in Examples 1 and 2, for example, a part of the tea leaves or used tea leaves is lost when the tea leaves or used tea leaves are transferred, for example, from the container to the homogenizer, or from the homogenizer to the sieving net, or a part of the tea leaves or used tea leaves is attached and remains inside the homogenizer.
Compared with the tea-leaf slurry of Example 1, the protein content was higher in the used-tea-leaf slurry of Example 2. It is considered that the reason for this is that since the used tea leaves were prepared by drying tea leaves after the aqueous solution had been extracted from the tea leaves, the total amount of the tea leaves that were substantially contained in the used-tea-leaf slurry of Example 2 was higher, compared with the tea-leaf slurry of Example 1, that is, the amount was larger than 2 kg.

[Table 1]

Table 1. Measurement of protein contents in extracts

| | Ex. 1 (Decomposition and separation of tea leaves) | | | Ex. 2 (Decomposition and separation of used tea leaves) | | | Comp. Ex. 1 (Decomposition of tea leaves) | | |
|---|---|---|---|---|---|---|---|---|---|
| | Tea-leaf solution | Tea-leaf slurry | Squeezed solution of tea-leaf slurry | Used-tea-leaf solution | Used-tea-leaf slurry | Squeezed solution of used-tea-leaf slurry | Tea-leaf solution | Tea leaves | Squeezed solution of tea leaves |
| Protein content [g / 100 g extract] | 1.2 | 1.9 | 0.9 | 1.2 | 1.7 | 0.8 | 0.5 | 7.2 | 0.7 |
| Protein content [tea leaves / used tea leaves total amount 2 kg] | 434.4 | 66.5 | 16.2 | 390 | 120.7 | 25.6 | 174 | 446.4 | 17.5 |
| Protein transfer ratio [%] | 84 | 13 | 3 | 73 | 23 | 5 | 27 | 70 | 3 |

[Table 2]

Table 2. Measurement of amino acid contents in extracts

| | Ex. 1 (Decomposition and separation of tea leaves) | | | Ex. 2 (Decomposition and separation of used tea leaves) | | | Comp. Ex. 1 (Decomposition of tea leaves) | | |
|---|---|---|---|---|---|---|---|---|---|
| | Tea-leaf solution | Tea-leaf slurry | Squeezed solution of tea-leaf slurry | Used-tea-leaf solution | Used-tea-leaf slurry | Squeezed solution of used-tea-leaf slurry | Tea-leaf solution | Tea leaves | Squeezed solution of tea leaves |
| Isoleucine content [g / 100 g extract] | 44 | 35 | 41 | 42 | 45 | 50 | 33 | 49 | 48 |
| Leucine content [g / 100 g extract] | 51 | 42 | 49 | 48 | 49 | 57 | 34 | 60 | 56 |
| Tryptophan content [g / 100 g extract] | 333 | 254 | 296 | 333 | 294 | 333 | 233 | 431 | 310 |
| Lysine content [g / 100 g extract] | 33 | 37 | 40 | 33 | 46 | 56 | 31 | 50 | 51 |

(continued)

| Table 2. Measurement of amino acid contents in extracts | | | | | | | | | |
|---|---|---|---|---|---|---|---|---|---|
| | Ex. 1 (Decomposition and separation of tea leaves) | | | Ex. 2 (Decomposition and separation of used tea leaves) | | | Comp. Ex. 1 (Decomposition of tea leaves) | | |
| | Tea-leaf solution | Tea-leaf slurry | Squeezed solution of tea-leaf slurry | Used-tea-leaf solution | Used-tea-leaf slurry | Squeezed solution of used-tea-leaf slurry | Tea-leaf solution | Tea leaves | Squeezed solution of tea leaves |
| Histidine content [g / 100 g extract] | 44 | 49 | 52 | 50 | 43 | 67 | 40 | 49 | 48 |
| Valine content [g / 100 g extract] | 43 | 36 | 43 | 41 | 44 | 48 | 36 | 48 | 48 |
| Threonine content [g / 100 g extract] | 69 | 71 | 87 | 69 | 66 | 103 | 0 | 74 | 99 |
| Sulfur-containing amino acid[*1] content [g / 100 g extract] | 27 | 17 | 20 | 19 | 19 | 23 | 27 | 27 | 32 |
| Aromatic amino acid[*2] content [g / 100 g extract] | 94 | 69 | 85 | 86 | 77 | 102 | 63 | 91 | 94 |
| (*1) methionine and cysteine (*2) phenylalanine and tyrosine | | | | | | | | | |

It was found from the results above that conducting both the decomposition and the separation makes it possible to increase the protein content and the amino acid content in each of the extracts obtained from tea leaves or used tea leaves, and thus a method for producing an extract of a biological tissue and a biological tissue processing apparatus with improved extraction efficiency can be provided.

[SEM Observation of Each Extract]

SEM observation using a scanning electron microscope (Model Number: TM3030Plus, Hitachi High-Tech Corporation) was conducted on the tea leaves and used tea leaves before being subjected to the decomposition and the separation, the tea-leaf slurry of Example 1, the used-tea-leaf slurry of Example 2, and the tea leaves of Comparative Example 1.

As a result, it was found that the tea leaves and used tea leaves before being subjected to the decomposition and the separation both maintained stomata and had similar surface structures (FIG. 11). It is considered that since the cell walls were hardly disrupted in the used tea leaves, most of the intracellular components (e.g., proteins, amino acids, catechin, and the like) remained in the cells.

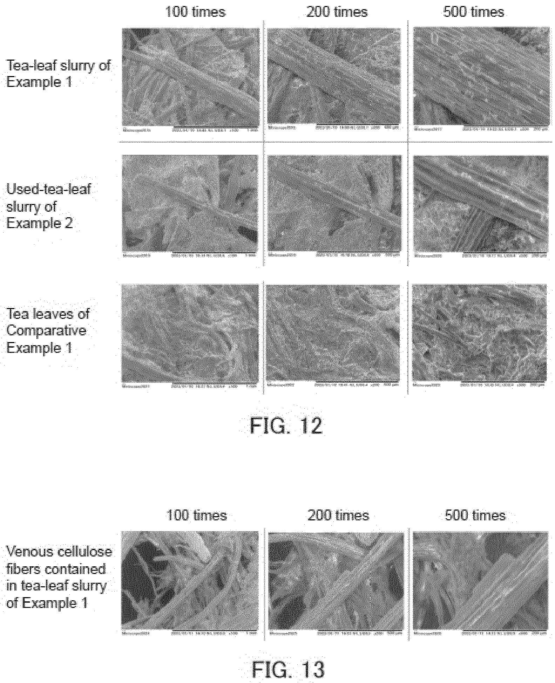

It was found that in the tea-leaf slurry of Example 1, the cell walls were significantly disrupted through the decomposition and separation, and the intracellular components were released to the outside of the cells (FIG. 12). Also, it was found that most of the components, which had adhered to each other in the tea leaves, were separated into individual components. In particular, it was found that the mesophyll and the vein were separated through the decomposition and the separation, and venous cellulose fibers that had adhered to other components such as cells were separated into individual venous cellulose fibers (FIG. 13). Cellulose and hemicellulose can be easily isolated from the separated venous cellulose fibers by subjecting them to further separation (fiber loosening, delignification, and the like). The same applies to the used-tea-leaf slurry of Example 2 (FIG. 12).

Meanwhile, it was found that the cell walls in the tea leaves of Comparative Example 1 were disrupted to some extent through the decomposition, and the intracellular components were released to the outside of the cells, but most of the components, which had adhered to each other in the tea leaves, were not separated into individual components (FIG. 12). In particular, it was found that in Comparative Example 1, the mesophyll and the veins still adhered to each other, and venous cellulose fibers remained adhering to other components such as cells. It is considered that the reason for this is that in Comparative Example 1, the components, which had adhered to each other in the tea leaves, could not be sufficiently separated into individual components through only the decomposition of the tea leaves. Also, in Comparative Example 1, venous cellulose fibers remained adhering to other components such as cells, leading to difficulty in isolating venous cellulose fibers.

It is assumed that the venous cellulose fibers of Example 1 have a thickness of 10 $\mu$m to 300 $\mu$m. The venous cellulose fibers are formed from a bundle of cellulose fibers of the order of a nanometer that is optionally branched. Cellulose microfibers and cellulose nanofibers can be obtained by subjecting the venous cellulose fibers to the fiber loosening. Cellulose microfibers and cellulose nanofibers are rich in industrial applicability and can be used in various applications.

Cellulose microfibers and cellulose nanofibers are light and strong and are thus suitable for industrial applications including additives for various organic solvents, thickeners for oils and fats, reinforcements for resin and rubber, and the like. Food such as tea leaves is used as a raw material, and it is thus considered that the venous cellulose fibers themselves are also suitable for applications including food additives and the like such as dietary fibers. For example, when venous cellulose fibers are mixed with ingredients of Western-style confectionery or Japanese-style confectionery as they are, and then Western-style confectionery or Japanese-style confectionery is cooked, it has a novel enjoyable eating texture, and dietary fibers serving as a nutrient can be ingested. For example, when a chiffon cake was cooked using ingredients mixed with venous cellulose fibers, the cake had a shape that was not easily lost and a firm eating texture with a very fluffy feel. Also, venous cellulose fibers can be added to ice cream, bread, and the like. With the applications above, it is expected to lower the environmental load because resources from the global environment are used in line with SDGs without being wasted and thus a sustainable society can be aimed for.

The following descriptions show the results obtained from tea leaves (secondary green tea and raw leaves) different from the tea leaves used in Example 1, coffee beans, strawberry leaves, and tomato leaves that were subjected to the decomposition and the separation and then the extraction of fibers, proteins, solutions, and the like in the same manner as in Example 1.

[Example 3]

(Decomposition and Separation of Tea Leaves)

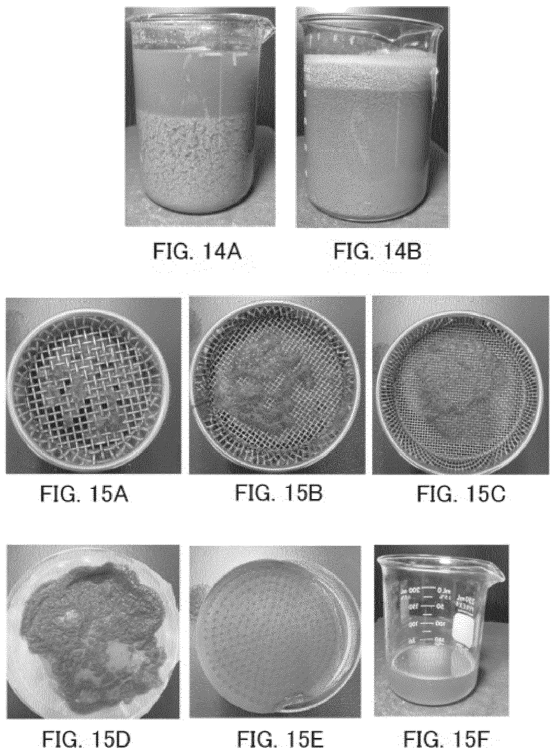

Tea leaves (secondary green tea) were subjected to the decomposition and the separation in the same manner as in Example 1. This example was different from Example 1 in that the tea leaves (25 g) were immersed in 500 ml of water (30°C, pH 4.0) containing an enzyme (cellulase, 3 g) for 24 hours, and were then homogenized under agitation at 4,000 rpm for 1 hour using the biological tissue processing apparatus 1A shown in FIGs. 1A-1B. FIGS. 14A and 14B show the immersed tea leaves before and after being homogenized under agitation. Note that the protein amount and the protein content rate in the tea leaves (25 g) were 6.35 g and 25.40%, respectively. The protein amount was measured using the Kjeldahl method in the same manner as in Example 1 (the same applies hereinafter). Note that crude tea leaves obtained by processing raw leaves plucked from a tea tree through a crude tea-producing process were used as the tea leaves and were subjected to the processing above.

(Solid-Liquid Separation (1))

A processed product of the tea leaves was poured onto a commercially available sieving net (mesh: 3.35 mm square). A tea-leaf slurry remained on the net (FIG. 15A), and a tea-leaf extract solution passed through the net and fell into a receiving container. Thus, the tea-leaf slurry (wet) and the tea-leaf extract solution were separated and obtained. The weight of the tea-leaf slurry (wet) was 13.35 g, and was 4.00 g in terms of the weight of the tea-leaf slurry (dry). The protein amount in the tea-leaf slurry was 0.10 g. The protein content rates in the wet tea-leaf slurry and the dry tea-leaf slurry were 0.76% and 2.53%, respectively.

(Solid-Liquid Separation (2))

The tea-leaf extract solution obtained through the solid-liquid separation (1) was poured onto a commercially available sieving net (mesh: 1.70 mm square). A tea-leaf slurry remained on the net (FIG. 15B), and a tea-leaf extract solution passed through the net and fell into a receiving container. Thus, the tea-leaf slurry (wet) and the tea-leaf extract solution were separated and obtained. The weight of the tea-leaf slurry (wet) was 26.35 g, and was 7.90 g in terms of the weight of the tea-leaf slurry (dry). The protein amount in the tea-leaf slurry was 0.26 g. The protein content rates in the wet tea-leaf slurry and the dry tea-leaf slurry were 0.99% and 3.29%, respectively.

(Solid-Liquid Separation (3))

The tea-leaf extract solution obtained through the solid-liquid separation (2) was poured onto a commercially available sieving net (mesh: 1.00 mm square). A tea-leaf slurry remained on the net (FIG. 15C), and a tea-leaf extract solution passed through the net and fell into a receiving container. Thus, the tea-leaf slurry (wet) and the tea-leaf extract solution were separated and obtained. The weight of the tea-leaf slurry (wet) was 15.71 g, and was 4.71 g in terms of the weight of the tea-leaf slurry (dry). The protein amount in the tea-leaf slurry was 0.42 g. The protein content rates in the wet tea-leaf slurry and the dry tea-leaf slurry were 2.69% and 8.96%, respectively.

(Solid-Liquid Separation (4))

The tea-leaf extract solution obtained through the solid-liquid separation (3) was poured onto a commercially available filter (aperture: 100 $\mu$m). A tea-leaf slurry remained on the filter (FIG. 15D), and a tea-leaf extract solution passed through the filter and fell into a receiving container. Thus, the tea-leaf slurry (wet) and the tea-leaf extract solution were separated and obtained. The weight of the tea-leaf slurry (wet) was 14.36 g, and was 4.31 g in terms of the weight of the tea-leaf slurry (dry). The protein amount in the tea-leaf slurry was 0.44 g. The protein content rates in the wet tea-leaf slurry and the dry tea-leaf slurry were 3.08% and 10.27%, respectively.

(Solid-Liquid Separation (5))

The tea-leaf extract solution obtained through the solid-liquid separation (4) was poured onto commercially available filter paper (aperture: 1.00 $\mu$m). A tea-leaf slurry remained on the filter paper (FIG. 15E), and a tea-leaf extract solution passed through the filter paper and fell into a receiving container (FIG. 15F). Thus, by separating the tea-leaf slurry (wet) and the tea-leaf extract solution from each other, the tea-leaf slurry and the tea-leaf extract solution were obtained. The weight of the tea-leaf slurry (wet) was 13.62 g, and was 4.08 g in terms of the weight of the tea-leaf slurry (dry). The protein amount in the tea-leaf slurry was 1.86 g. The protein content rates in the wet tea-leaf slurry and the dry tea-leaf slurry were 13.64% and 45.50%, respectively. The weight of the tea-leaf extract solution obtained after the solid-liquid separation (5) was 416.62 g. The protein amount in the tea-leaf extract solution was 3.26 g. The protein content rate in the tea-leaf extract solution was 0.78%. The catechin amount and the caffeine amount in the tea-leaf extract solution were 512.66 mg and 1868.62 mg, respectively. Note that the components contained in the tea-leaf extract solution, such as catechin and caffeine, were measured using high-performance liquid chromatography (the same applies hereinafter).

[Comparative Example 2]

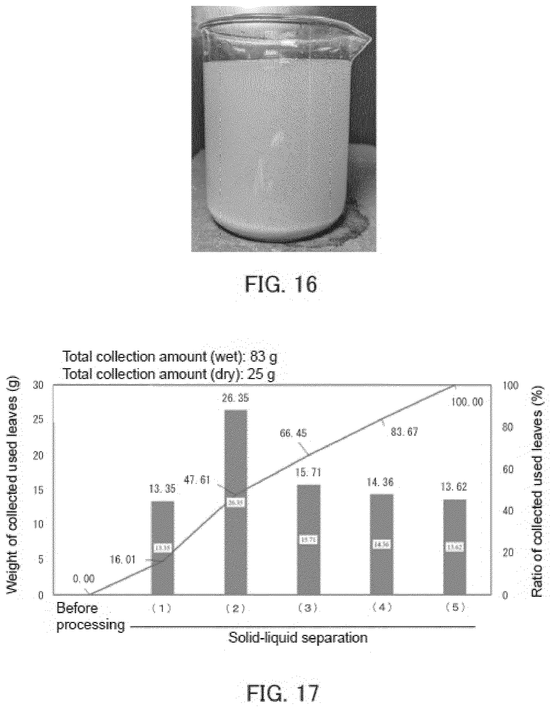

Common tea extraction was conducted using the tea leaves used in Example 3. Specifically, the tea leaves (25 g) were immersed in 500 ml of water (80°C) for 5 minutes. FIG. 16 shows the immersed tea leaves. Note that the protein amount, the protein content rate, the catechin amount, and the caffeine amount in the tea leaves (328 g) were 1.23 g, 0.376%, 1523.84 mg, and 377.23 mg, respectively.

(Results and Discussion)

FIG. 17 shows the results of the weights of the wet tea-leaf slurries obtained in Example 3. In FIG. 17, the horizontal axis indicates the solid-liquid separation, and the vertical axes indicate the weight of the wet tea-leaf slurry obtained in the solid-liquid separation (weight of collected used leaves, bar graph, left axis) and the ratio thereof to the total weight (ratio of collected used leaves, line graph, right axis). As shown in FIG. 17, the weight of the wet tea-leaf slurry obtained through the solid-liquid separation (1) was 13.35 g. Meanwhile, the total weight of the wet tea-leaf slurries obtained through all the solid-liquid separation (1) to (5) was 83 g. Accordingly, the ratio of the weight of the wet tea-leaf slurry obtained through the solid-liquid separation (1) to the total weight was 16.01%. Similarly, the ratios as described above for the solid-liquid separation (2) to (5) are also shown in FIG. 17.

The total amount of the tea-leaf slurries obtained through the solid-liquid separation (1) to (4) was 69.77 g, and the total amount of the tea-leaf slurries obtained through the solid-liquid separation (1) to (5) was 83.39 g. Accordingly, the total amount of the tea-leaf slurries obtained through the solid-liquid separation (1) to (4) made up 83.67% of the total amount of the tea-leaf slurries obtained through the solid-liquid separation (1) to (5). Therefore, it was found that most of the tea-leaf slurry could be collected from the tea-leaf slurry obtained from the entire tea leaves by conducting the solid-liquid separation (1) to (4).

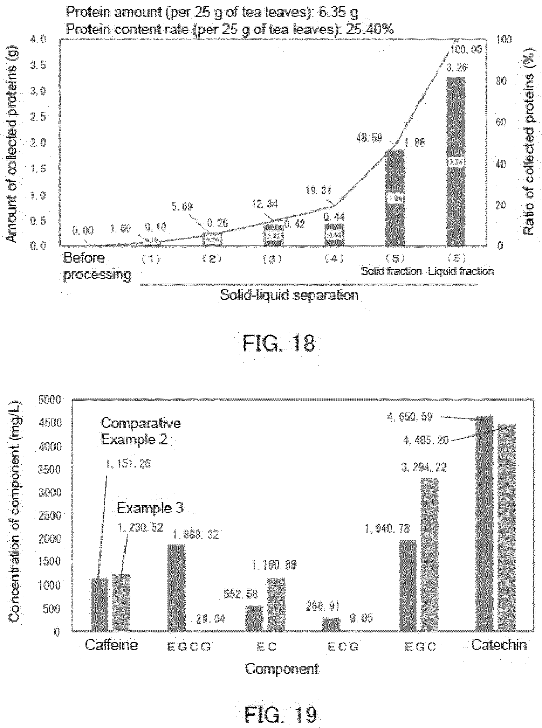

Next, FIG. 18 shows the results of the protein amounts obtained in Example 3. In FIG. 18, the horizontal axis indicates the solid-liquid separation, and the vertical axes indicate the protein amount obtained in the solid-liquid separation (total collection amount, bar graph, left axis) and the ratio thereof to the total protein amount (total collection ratio, line graph, right axis). As shown in FIG. 18, the protein amount obtained through the solid-liquid separation (1) was 0.10 g. Meanwhile, the total protein amount obtained through all the solid-liquid separation (1) to (5) was 6.35 g. Accordingly, the ratio of the protein amount obtained through the solid-liquid separation (1) to the total protein amount was 1.60%.

The total protein amount in the tea-leaf slurry and the tea-leaf extract solution obtained through the solid-liquid separation (5) was 5.12 g. Meanwhile, the protein amount in the tea leaves (25 g) used in this example was 6.35 g. Accordingly, it was found that 80.69% of the proteins obtained from the entire tea leaves could be collected by conducting the solid-liquid separation (5). It was also found that most of the proteins contained in the tea leaves could be collected using filters with apertures in a range of about 1.00 μm to 100 μm, and most of the other components such as fibers and dietary fibers could be removed using a filter with apertures of 100 μm or greater.

The total protein amount in the tea-leaf slurries obtained through the solid-liquid separation (1) to (4) was 1.22 g, and the protein amount in the tea-leaf slurry obtained through the solid-liquid separation (5) was 1.86 g. Meanwhile, the protein amount in the tea leaves (25 g) used in this example was 6.35 g. Accordingly, relative to the protein amount in the tea leaves (25 g) used in this example, the total protein amount in the tea-leaf slurries obtained through the solid-liquid separation (1) to (4) was 19.31%, and the protein amount in the tea-leaf slurry obtained through the solid-liquid separation (5) was 48.59%. Accordingly, it was found that 29.28% of the proteins obtained from the entire tea leaves could be collected by conducting the solid-liquid separation (5) subsequently to the solid-liquid separation (1) to (4).

The protein content rate in the dry tea-leaf slurry obtained through the solid-liquid separation (5) was 45.50%. A dry powder solid in which proteins are concentrated is obtained by drying the dry tea-leaf slurry shown in FIG. 15E using a hot-air dryer. Therefore, it was found that a dry solid in which proteins are concentrated can be obtained through the solid-liquid separation as described above.

FIG. 19 shows the results of the concentrations of the components obtained in Example 3 and Comparative Example 2. In FIG. 19, the horizontal axis indicates the components, and the vertical axis indicates the concentrations of the components. The catechin amount and the caffeine amount obtained in Example 3 were 1868.62 mg and 512.66 mg, respectively. The amount of the tea-leaf extract solution obtained in Example 3 was 416.62 g, and therefore, the concentrations of the catechin amount and the caffeine amount obtained in Example 3 were 4485.20 mg/L and 1230.52 mg/L, respectively.

Meanwhile, the catechin amount and the caffeine amount obtained in Comparative Example 2 were 1523.84 mg and 377.23 mg, respectively. The amount of the tea obtained in Comparative Example 2 was 328 g, and therefore, the concentrations of the catechin amount and the caffeine amount obtained in Comparative Example 2 were 4650.59 mg/L and 1151.26 mg/L, respectively. Accordingly, it was found that the concentrations of the caffeine and the catechin obtained in Example 3 were similar to those in Comparative Example 2.

It was found from the results above that fibers derived from tea leaves, dietary fibers, proteins derived from tea leaves, a solid containing components derived from tea leaves, an extract solution, and the like can be efficiently extracted from tea leaves (secondary green tea) different from the tea leaves used in Example 1 by subjecting the tea leaves to the decomposition and the separation in the same manner as in Example 1. Also, the extract solution obtained through the solid-liquid separation (5) contained proteins at a much higher concentration than Comparative Example 2, and it was thus found that a tea beverage containing proteins at a high concentration can be produced by processing the extract solution directly into a tea beverage in subsequent processes. Also, active components such as proteins and catechin can be separated and extracted from the extract solution in subsequent processes using techniques such as filtration, concentration-separation, freeze-concentration spray drying, and evaporation treatment.

[Example 4]

(Decomposition and Separation of Tea Leaves)

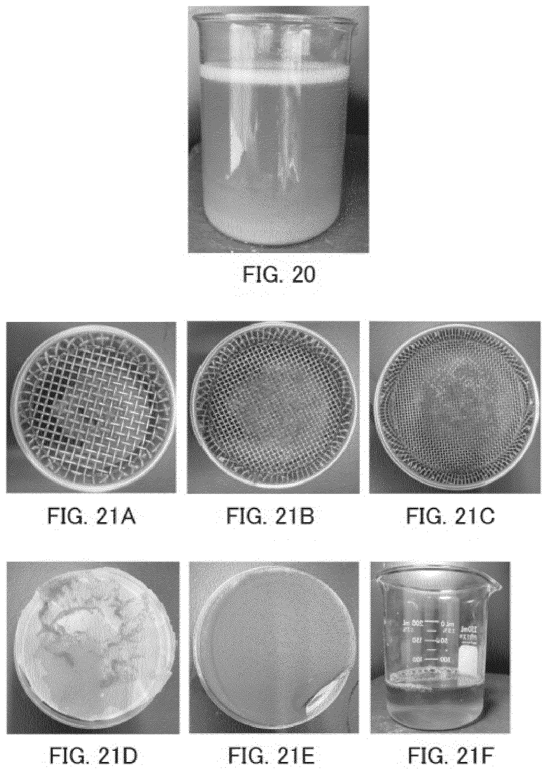

Tea leaves (raw leaves) were subjected to the decomposition and the separation in the same manner as in Example 1. This example was different from Example 1 in that the tea leaves (25 g) were immersed in 500 ml of water (30°C, pH 4.0) containing an enzyme (cellulase, 3 g) for 24 hours and were then homogenized under agitation at 4,000 rpm for 1 hour using the biological tissue processing apparatus 1A shown in FIGs. 1A-1B, and the tea leaves before being subjected to the decomposition were subjected to drying. Furthermore, while crude tea leaves were used in Example 3, raw leaves were used in this example, and therefore, this example was also different from Example 1 in that the raw leaves were subjected to pretreatment (physical disruption of cell walls (homogenization using a food mill or the like, shearing, or the like)) for the decomposition and the separation. FIG. 20 shows the tea leaves immersed after being homogenized under agitation. Note that the protein amount and the protein content rate in the tea leaves (25 g) (wet) were 1.62 g and 6.48%, respectively, and the protein content rate in the tea leaves (6.5 g) (dry) was 24.92%. Note that raw leaves plucked from a tea tree were used as the tea leaves and were subjected to the processing above.

(Solid-Liquid Separation (1))

The processed product of the tea leaves was poured onto a commercially available sieving net (mesh: 3.35 mm square). A tea-leaf slurry remained on the net (FIG. 21A), and a tea-leaf extract solution passed through the net and fell into a receiving container. Thus, the tea-leaf slurry (wet) and the tea-leaf extract solution were separated and obtained. The weight of the tea-leaf slurry (wet) was 2.85 g, and was 0.44 g in terms of the weight of the tea-leaf slurry (dry). The protein amount in the tea-leaf slurry was 0 g. The protein content rates in the wet tea-leaf slurry and the dry tea-leaf slurry were both 0%.

(Solid-Liquid Separation (2))

The tea-leaf extract solution obtained through the solid-liquid separation (1) was poured onto a commercially available sieving net (mesh: 1.70 mm square). A tea-leaf slurry remained on the net (FIG. 21B), and a tea-leaf extract solution passed through the net and fell into a receiving container. Thus, the tea-leaf slurry (wet) and the tea-leaf extract solution were separated and obtained. The weight of the tea-leaf slurry (wet) was 12.99 g, and was 2.00 g in terms of the weight of the tea-leaf slurry (dry). The protein amount in the tea-leaf slurry was 0 g. The protein content rates in the wet tea-leaf slurry and the dry tea-leaf slurry were both 0%.

(Solid-Liquid Separation (3))

The tea-leaf extract solution obtained through the solid-liquid separation (2) was poured onto a commercially available sieving net (mesh: 1.00 mm square). A tea-leaf slurry remained on the net (FIG. 21C), and a tea-leaf extract solution passed through the net and fell into a receiving container. Thus, the tea-leaf slurry (wet) and the tea-leaf extract solution were separated and obtained. The weight of the tea-leaf slurry (wet) was 12.93 g, and was 1.99 g in terms of the weight of the tea-leaf slurry (dry). The protein amount in the tea-leaf slurry was 0.14 g. The protein content rates in the wet tea-leaf slurry and the dry tea-leaf slurry were 1.11% and 7.23%, respectively.

(Solid-Liquid Separation (4))

The tea-leaf extract solution obtained through the solid-liquid separation (3) was poured onto a commercially available filter (aperture: 100 μm). A tea-leaf slurry remained on the filter (FIG. 21D), and a tea-leaf extract solution passed through the filter and fell into a receiving container. Thus, the tea-leaf slurry (wet) and the tea-leaf extract solution were separated and obtained. The weight of the tea-leaf slurry (wet) was 4.99 g, and was 0.77 g in terms of the weight of the tea-leaf slurry (dry). The protein amount in the tea-leaf slurry was 0 g. The protein content rates in the wet tea-leaf slurry and the dry tea-leaf slurry were both 0%.

(Solid-Liquid Separation (5))

The tea-leaf extract solution obtained through the solid-liquid separation (4) was poured onto commercially available filter paper (aperture: 1.00 μm). A tea-leaf slurry remained on the filter paper (FIG. 21E), and a tea-leaf extract solution passed through the filter paper and fell into a receiving container (FIG. 21F). Thus, the tea-leaf slurry (wet) and the tea-leaf extract solution were separated and obtained. The weight of the tea-leaf slurry (wet) was 8.49 g, and was 1.31 g in terms of the weight of the tea-leaf slurry (dry). The protein amount in the tea-leaf slurry was 0.79 g. The protein content rates in the wet tea-leaf slurry and the dry tea-leaf slurry were 9.26% and 60.25%, respectively. The weight of the tea-leaf extract solution obtained after the solid-liquid separation (5) was 457.75 g. The protein amount in the tea-leaf extract solution was 0.69 g. The protein content rate in the tea-leaf extract solution was 0.15%. The catechin amount and the caffeine amount in the tea-leaf extract solution were 23.61 mg and 147.69 mg, respectively.

[Comparative Example 3]

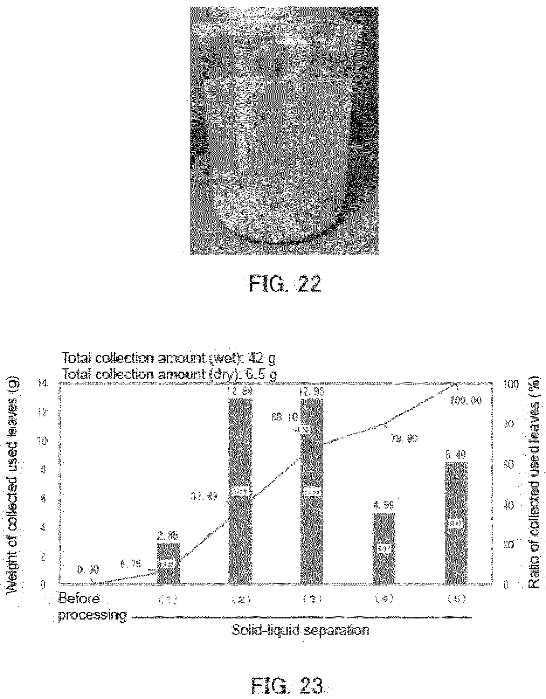

Common tea extraction was conducted using the tea leaves used in Example 4. Specifically, the tea leaves (25 g) were immersed in 500 ml of water (80°C) for 5 minutes. FIG. 22 shows the immersed tea leaves. Note that the protein amount, the protein content rate, the catechin amount, and the caffeine amount in the tea leaves (439 g) were 0.15 g, 0.034%, 383.90 mg, and 145.83 mg, respectively.

(Results and Discussion)

FIG. 23 shows the results of the weights of the wet tea-leaf slurries obtained in Example 4. In FIG. 23, the horizontal axis indicates the solid-liquid separation, and the vertical axes indicate the weight of the wet tea-leaf slurry obtained in the solid-liquid separation (weight of collected used leaves, bar graph, left axis) and the ratio thereof to the total weight (ratio of collected used leaves, line graph, right axis). As shown in FIG. 23, the weight of the wet tea-leaf slurry obtained through the solid-liquid separation (1) was 2.85 g. Meanwhile, the total weight of the wet tea-leaf slurries obtained through all the solid-liquid separation (1) to (5) was 42 g. Accordingly, the ratio of the weight of the wet tea-leaf slurry obtained through the solid-liquid separation (1) to the total weight was 6.75%.

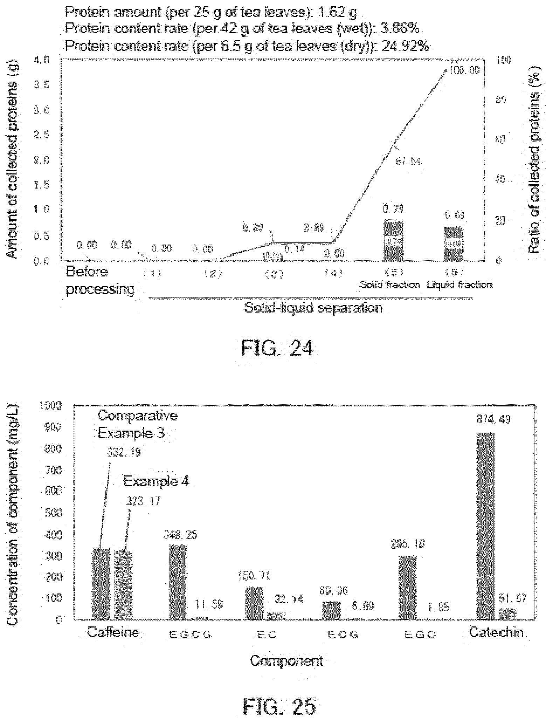

FIG. 24 shows the results of the protein amounts obtained in Example 4. In FIG. 24, the horizontal axis indicates the solid-liquid separation, and the vertical axes indicate the protein amount obtained in the solid-liquid separation (bar graph) and the ratio thereof to the total protein amount (line graph). As shown in FIG. 24, the protein amount obtained through the solid-liquid separation (3) was 0.14 g. Meanwhile, the total protein amount obtained through all the solid-liquid separation (1) to (5) was 1.62 g. Accordingly, the ratio of the protein amount obtained through the solid-liquid separation (3) to the total protein amount was 8.89%.

The protein amount in the tea-leaf slurry obtained through the solid-liquid separation (5) was 0.79 g. Meanwhile, the protein amount in the tea leaves (25 g) used in this example was 1.62 g. Accordingly, relative to the protein amount in the tea leaves (25 g) used in this example, the protein amount in the tea-leaf slurry obtained through the solid-liquid separation (5) was 48.77%.

The protein amount in the tea-leaf extract solution obtained through the solid-liquid separation (5) was 0.69 g. Accordingly, relative to the protein amount in the tea leaves (25 g) used in this example, the protein amount in the tea-leaf extract solution obtained through the solid-liquid separation (5) was 42.59%. Relative to the protein amount in the tea leaves (25 g) used in this example, the total protein amount in the tea-leaf slurry and the tea-leaf extract solution obtained through the solid-liquid separation (5) was 91.36%.

The protein content rate in the dry tea-leaf slurry obtained through the solid-liquid separation (5) was 60.25%. Therefore, it was found that a dry solid in which proteins are concentrated can be obtained through the solid-liquid separation as described above.

FIG. 25 shows the results of the concentrations of the components obtained in Example 4 and Comparative Example 3. In FIG. 25, the horizontal axis indicates the components, and the vertical axis indicates the concentrations of the components (component concentrations). In FIG. 25, the bars on the left indicate the results from Comparative Example 3, and the bars on the right indicate the results from Example 4. EGCG, EC, ECG, and EGC in the diagram represent epigallocatechin gallate, epicatechin, epicatechin gallate, and epigallocatechin, respectively. The catechin amount and the caffeine amount obtained in Example 4 were 23.61 mg and 147.69 mg, respectively. The amount of the tea-leaf extract solution obtained in Example 4 was 457.75 g, and therefore, the concentrations of the catechin amount and the caffeine amount obtained in Example 4 were 51.67 mg/L and 323.17 mg/L, respectively.

Meanwhile, the catechin amount and the caffeine amount obtained in Comparative Example 3 were 383.90 mg and 145.83 mg, respectively. The amount of the tea obtained in Comparative Example 3 was 439 g, and therefore, the concentrations of the catechin amount and the caffeine amount obtained in Comparative Example 3 were 874.49 mg/L and 332.19 mg/L, respectively.

Accordingly, it was found that the concentration of the caffeine obtained in Example 4 was similar to that in Comparative Example 3. Meanwhile, the concentration of the catechin obtained in Example 4 was less than 1/10 of the concentration of the catechin obtained in Comparative Example 3, and it was thus found that compared with caffeine, catechin did not move to the solutions and remained in the solid (tea-leaf slurries) during the solid-liquid separation processes in Example 4. Catechin could be moved to the solution by immersing the tea leaves in water at 80°C in Comparative Example 3, and therefore, it is considered that in Example 4, catechin could be moved to the solution by immersing the solid (tea-leaf slurry) in water at 80°C, whereas catechin could be allowed to remain in the solid fraction by setting the temperature to be lower than or equal to a certain temperature (e.g., a temperature lower than a temperature of 50°C or lower (30°C in this example)).

It was found from the results above that fibers derived from tea leaves, dietary fibers, proteins derived from tea leaves, a solid containing components derived from tea leaves, an extract solution, and the like can be efficiently extracted from tea leaves (raw leaves) different from the tea leaves used in Example 1 by subjecting the tea leaves to the decomposition and the separation in the same manner as in Example 1. It was also found that the rates of extraction of catechin to a solid, an extract solution, and the like could be adjusted by adjusting the temperature during the processing. Also, similarly to Example 3, a tea beverage containing proteins at a high concentration can be produced by processing the extract solution obtained through the solid-liquid separation (5) directly into a tea beverage in subsequent processes. Also, active components such as proteins and catechin can be separated and extracted from the extract solution in subsequent processes using techniques such as filtration, concentration-separation, freeze-concentration spray drying, and evaporation treatment.

[Example 5]

(Decomposition and Separation of Coffee Beans)

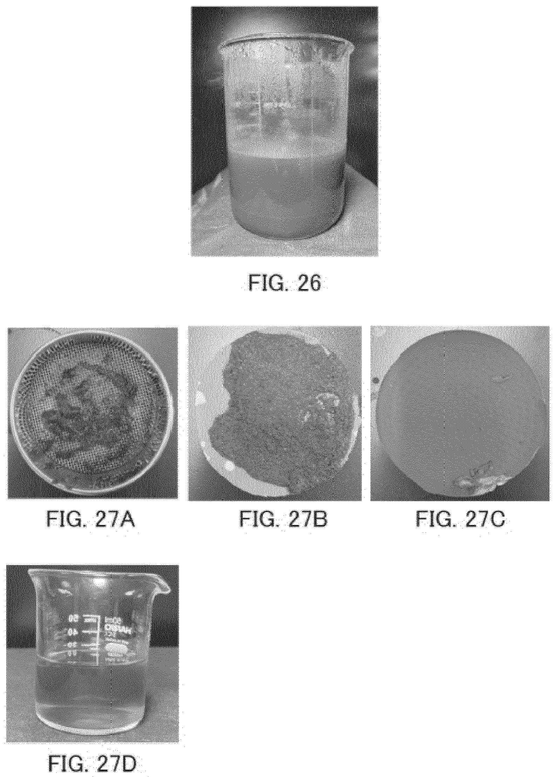

Coffee beans (Brazilian Arabica city roast) were subjected to the decomposition and the separation in the same manner as in Example 1. This example was different from Example 1 in that the coffee beans (dry weight: 36 g) were immersed in 500 ml of water (50°C, pH 4.0) containing an enzyme (cellulase, 3 g) for 24 hours, and were then homogenized under agitation at 4,000 rpm for 1 hour using the biological tissue processing apparatus 1A shown in FIGs. 1A-1B. FIG. 26 shows the immersed coffee beans after being homogenized under agitation. Note that the protein amount in the coffee beans (36 g) was 4.04 g, and the protein content rate in the coffee beans (36 g) was 11.22%. Note that the coffee beans above were pulverized (for espresso) through mill-grinding using a coffee mill (FUJI ROYAL, Model Number: DX R-220) and were then subjected to the processing above.

(Solid-Liquid Separation (1))

The processed product of the coffee beans was poured onto a commercially available sieving net (mesh: 1.00 mm square). A coffee-bean slurry remained on the net (FIG. 27A), and a coffee-bean extract solution passed through the net and fell into a receiving container. Thus, the coffee-bean slurry (wet) and the coffee-bean extract solution were separated and obtained. The weight of the coffee-bean slurry (wet) was 55.73 g, and was 17.17 g in terms of the weight of the coffee-bean slurry (dry). The protein amount in the coffee-bean slurry was 0 g. The protein content rates in the wet coffee-bean slurry and the dry coffee-bean slurry were both 0%.

(Solid-Liquid Separation (2))

The coffee-bean extract solution obtained through the solid-liquid separation (1) was poured onto a commercially available filter (aperture: 100 μm). A coffee-bean slurry remained on the filter (FIG. 27B), and a coffee-bean extract solution passed through the filter and fell into a receiving container. Thus, the coffee-bean slurry (wet) and the coffee-bean extract solution were separated and obtained. The weight of the coffee-bean slurry (wet) was 55.57 g, and was 17.12 g in terms of the weight of the coffee-bean slurry (dry). The protein amount in the coffee-bean slurry was 1.71 g. The protein content rates in the wet coffee-bean slurry and the dry coffee-bean slurry were 3.08% and 10.01%, respectively.

(Solid-Liquid Separation (3))

The coffee-bean extract solution obtained through the solid-liquid separation (2) was poured onto commercially available filter paper (aperture: 1.00 μm). A coffee-bean slurry remained on the filter paper (FIG. 27C), and a coffee-bean extract solution passed through the filter paper and fell into a receiving container (FIG. 27D). Thus, the coffee-bean slurry and the coffee-bean extract solution were separated and obtained. The weight of the coffee-bean slurry (wet) was 5.57 g, and was 1.72 g in terms of the weight of the coffee-bean slurry (dry). The protein amount in the coffee-bean slurry was 0.86 g. The protein content rates in the wet coffee-bean slurry and the dry coffee-bean slurry were 15.45% and 50.16%, respectively. The weight of the coffee-bean extract solution obtained after the solid-liquid separation (3) was 383.14 g. The protein amount in the coffee-bean extract solution was 1.46 g. The protein content rate in the coffee-bean extract solution was 0.38%. The caffeine amount in the coffee-bean extract solution was 289.54 mg.

[Comparative Example 4]

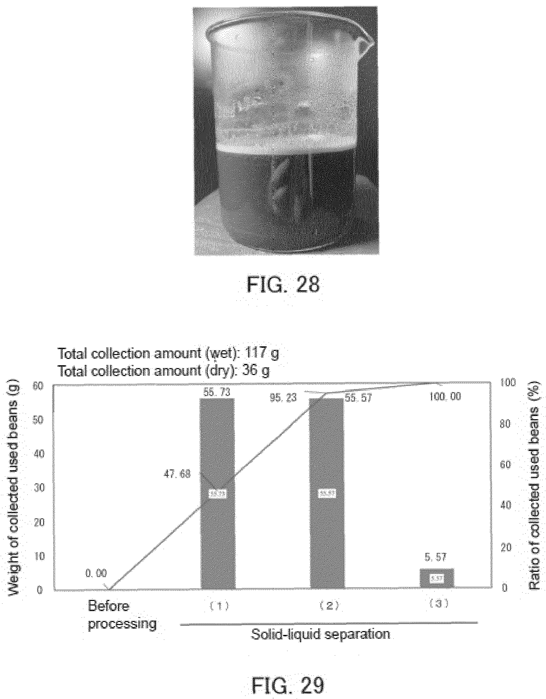

Common coffee extraction was conducted using the coffee beans used in Example 5. Specifically, the coffee beans (dry weight: 36 g) were immersed in 500 ml of water (80°C) for 5 minutes. FIG. 28 shows the immersed coffee beans. Note that the protein amount, the protein content rate, and the caffeine amount in the coffee beans (366 g) were 1.39 g, 0.38%, and 629.90 mg, respectively.

(Results and Discussion)

FIG. 29 shows the results of the weights of the wet coffee-bean slurries obtained in Example 5. In FIG. 29, the horizontal axis indicates the solid-liquid separation, and the vertical axes indicate the weight of the wet coffee-bean slurry obtained in the solid-liquid separation (weight of collected used beans, bar graph, left axis) and the ratio thereof to the total weight (ratio of collected used beans, line graph, right axis). As shown in FIG. 29, the weight of the wet coffee-bean slurry obtained through the solid-liquid separation (1) was 55.73 g. Meanwhile, the total weight of the wet coffee-bean slurries obtained through all the solid-liquid separation (1) to (3) was 117 g. Accordingly, the ratio of the weight of the wet coffee-bean slurry obtained through the solid-liquid separation (1) to the total weight was 47.68%.

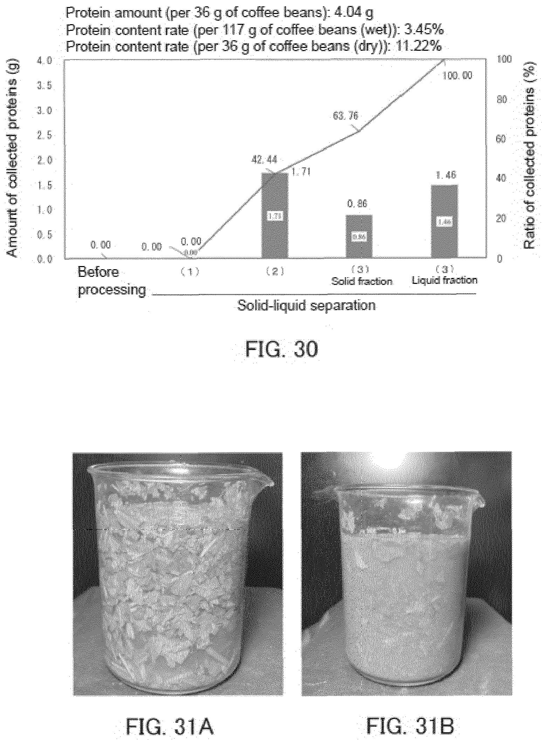

FIG. 30 shows the results of the protein amounts obtained in Example 5. In FIG. 30, the horizontal axis indicates the solid-liquid separation, and the vertical axes indicate the protein amount obtained in the solid-liquid separation (weight of collected used beans, bar graph, left axis) and the ratio thereof to the total protein amount (ratio of collected used beans, line graph, right axis). As shown in FIG. 30, the protein amount obtained through the solid-liquid separation (2) was 1.71 g. Meanwhile, the total protein amount obtained through all the solid-liquid separation (1) to (3) was 4.04 g. Accordingly, the ratio of the protein amount obtained through the solid-liquid separation (2) to the total protein amount was 42.44%.

The protein amount in the coffee-bean slurry obtained through the solid-liquid separation (3) was 0.86 g. Meanwhile, the protein amount in the coffee beans (36 g) used in this example was 4.04 g. Accordingly, relative to the protein amount in the coffee beans (36 g) used in this example, the protein amount in the coffee-bean slurry obtained through the solid-liquid separation (3) was 21.29%.

The protein amount in the coffee-bean extract solution obtained through the solid-liquid separation (3) was 1.46 g. Accordingly, relative to the protein amount in the coffee beans (36 g) used in this example, the protein amount in the coffee-bean extract solution obtained through the solid-liquid separation (3) was 36.14%. Relative to the protein amount in the coffee beans (36 g) used in this example, the total protein amount in the coffee-bean slurry and the coffee-bean extract solution obtained through the solid-liquid separation (3) was 57.43%.

The protein content rate in the dry coffee-bean slurry obtained through the solid-liquid separation (3) was 50.16%. A dry powder solid in which proteins are concentrated is obtained by drying the dry coffee-bean slurry shown in FIG. 27C using a hot-air dryer. Therefore, it was found that a dry solid in which proteins are concentrated can be obtained through the solid-liquid separation as described above.

It was found from the results above that fibers derived from coffee, proteins derived from coffee, a solution containing components derived from coffee, and the like can be efficiently extracted from coffee beans by subjecting the coffee beans to the decomposition and the separation in the same manner as in Example 1. Also, the extract solution obtained through the solid-liquid separation (3) contained proteins at a higher concentration than Comparative Example 4, and it was thus found that a coffee beverage containing proteins at a high concentration can be produced by processing the extract solution directly into a coffee beverage in subsequent processes.

Example 5 is an example in which coffee beans pulverized through mill-grinding are used, and a considerable amount of active components such as proteins remain in solids collected using a sieving net, a filter paper, a filter, and the like in the solid-liquid separation (1), (2), and (3) and used coffee beans (which are commonly discarded) after the coffee extraction in stores such as restaurants and convenience stores. Accordingly, the remaining active components such as proteins can be further extracted by subjecting these solids, used coffee beans, and the like to the processing of Example 5 again. An extract solution obtained at that time has a low concentration, and therefore, it is also possible to produce a coffee beverage by concentrating the extract solution or mixing the extract solution with a solution with a high concentration to adjust the concentration.

[Example 6]

(Decomposition and Separation of Strawberry Leaves)

Strawberry leaves were subjected to the decomposition and the separation in the same manner as in Example 1. This example was different from Example 1 in that the strawberry leaves (dry weight: 25 g) were immersed in 500 ml of water (50°C, pH 4.0) containing an enzyme (cellulase, 3 g) for 24 hours, and were then homogenized under agitation at 4,000 rpm for 1 hour using the biological tissue processing apparatus 1A shown in FIGs. 1A-1B. FIGS. 31A and 31B show the immersed strawberry leaves before and after being homogenized under agitation, respectively. Note that the protein amount in the strawberry leaves (25 g) was 1.85 g, and the protein content rate in the strawberry leaves (25 g) was 7.41%. Note that raw leaves, stem portions, and the like left after plucking strawberries were used as the strawberry leaves and were subjected to the processing above. These raw leaves, stem portions, and the like are usually discarded.

(Solid-Liquid Separation (1))

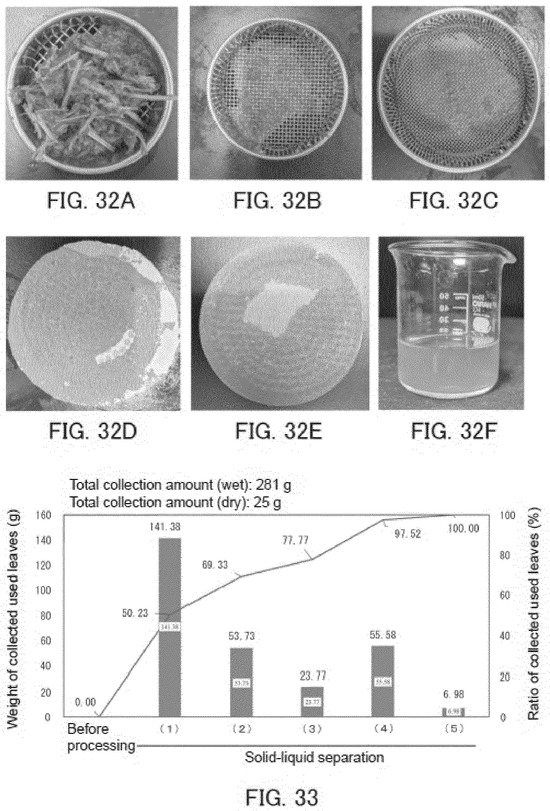

The processed product of the strawberry leaves was poured onto a commercially available sieving net (mesh: 3.35 mm square). A strawberry-leaf slurry remained on the net (FIG. 32A), and a strawberry-leaf extract solution passed through the net and fell into a receiving container. Thus, the strawberry-leaf slurry (wet) and the strawberry-leaf extract solution were separated and obtained. The weight of the strawberry-leaf slurry (wet) was 141.38 g, and was 12.56 g in terms of the weight of the strawberry-leaf slurry (dry). The protein amount in the strawberry-leaf slurry was 1.08 g. The protein content rates in the wet strawberry-leaf slurry and the dry strawberry-leaf slurry were 0.76% and 8.56%, respectively.

(Solid-Liquid Separation (2))

The strawberry-leaf extract solution obtained through the solid-liquid separation (1) was poured onto a commercially available sieving net (mesh: 1.70 mm square). A strawberry-leaf slurry remained on the net (FIG. 32B), and a strawberry-leaf extract solution passed through the net and fell into a receiving container. Thus, the strawberry-leaf slurry and the strawberry-leaf extract solution were separated and obtained. The weight of the strawberry-leaf slurry (wet) was 53.73 g, and was 4.77 g in terms of the weight of the strawberry-leaf slurry (dry). The protein amount in the strawberry-leaf slurry was 0.15 g. The protein content rates in the wet strawberry-leaf slurry and the dry strawberry-leaf slurry were 0.28% and 3.20%, respectively. Also, the strawberry-leaf slurry was rich in strawberry-leaf fibers, dietary fibers, and the like.

(Solid-Liquid Separation (3))

The strawberry-leaf extract solution obtained through the solid-liquid separation (2) was poured onto a commercially available sieving net (mesh: 1.00 mm square). A strawberry-leaf slurry remained on the net (FIG. 32C), and a strawberry-leaf extract solution passed through the net and fell into a receiving container. Thus, the strawberry-leaf slurry (wet) and the strawberry-leaf extract solution were separated and obtained. The weight of the strawberry-leaf slurry (wet) was 23.77, and was 2.11 g in terms of the weight of the strawberry-leaf slurry (dry). The protein amount in the strawberry-leaf slurry was 0.08 g. The protein content rates in the wet strawberry-leaf slurry and the dry strawberry-leaf slurry were 0.35% and 3.89%, respectively.

(Solid-Liquid Separation (4))

The strawberry-leaf extract solution obtained through the solid-liquid separation (3) was poured onto a commercially available filter (aperture: 100 $\mu$m). A strawberry-leaf slurry remained on the filter (FIG. 32D), and a strawberry-leaf extract solution passed through the filter and fell into a receiving container. Thus, the strawberry-leaf slurry (wet) and the strawberry-leaf extract solution were separated and obtained. The weight of the strawberry-leaf slurry (wet) was 55.58 g, and was 4.94 g in terms of the weight of the strawberry-leaf slurry (dry). The protein amount in the strawberry-leaf slurry was 0.17 g. The protein content rates in the wet strawberry-leaf slurry and the dry strawberry-leaf slurry were 0.30% and 3.39%, respectively.

(Solid-Liquid Separation (5))

The strawberry-leaf extract solution obtained through the solid-liquid separation (4) was poured onto commercially available filter paper (aperture: 1.00 $\mu$m). A strawberry-leaf slurry remained on the filter paper (FIG. 32E), and a strawberry-leaf extract solution passed through the filter paper and fell into a receiving container (FIG. 32F). Thus, the strawberry-leaf slurry (wet) and the strawberry-leaf extract solution were separated and obtained. The weight of the strawberry-leaf slurry (wet) was 6.98 g, and was 0.62 g in terms of the weight of the strawberry-leaf slurry (dry). The protein amount in the strawberry-leaf slurry was 0.21 g. The protein content rates in the wet strawberry-leaf slurry and the dry strawberry-leaf slurry were 3.02% and 33.95%, respectively. The weight of the strawberry-leaf extract solution obtained after the solid-liquid separation (5) was 218.57 g. The protein amount in the strawberry-leaf extract solution was 0.16 g. The protein content rate in the strawberry-leaf extract solution was 0.07%.

(Results and Discussion)

FIG. 33 shows the results of the weights of the wet strawberry-leaf slurries obtained in Example 6. In FIG. 33, the horizontal axis indicates the solid-liquid separation, and the vertical axes indicate the weight of the wet strawberry-leaf slurry obtained in the solid-liquid separation (weight of collected used leaves, bar graph, left axis) and the ratio thereof to the total weight (ratio of collected used leaves, line graph, right axis). As shown in FIG. 33, the weight of the wet strawberry-leaf slurry obtained through the solid-liquid separation (1) was 141.38 g. Meanwhile, the total weight of the wet tea-leaf slurries obtained through all the solid-liquid separation (1) to (5) was 281 g. Accordingly, the ratio of the weight of the wet strawberry-leaf slurry obtained through the solid-liquid separation (1) to the total weight was 50.23%. Also, the strawberry-leaf slurry was rich in strawberry-leaf fibers, dietary fibers, and the like.

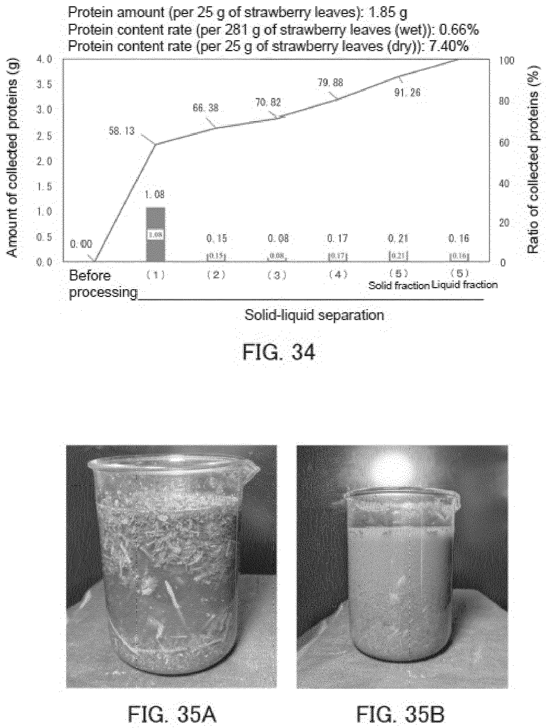

FIG. 34 shows the results of the protein amounts obtained in Example 6. As shown in FIG. 34, the horizontal axis indicates the solid-liquid separation, and the vertical axes indicate the protein amount obtained in the solid-liquid separation (weight of collected used leaves, bar graph, left axis) and the ratio thereof to the total protein amount (ratio of collected used leaves, line graph, right axis). As shown in FIG. 34, the protein amount obtained through the solid-liquid separation (1) was 1.08 g. Meanwhile, the total protein amount obtained through all the solid-liquid separation (1) to (5) was 1.85 g. Accordingly, the ratio of the protein amount obtained through the solid-liquid separation (1) to the total protein amount was 58.13%.

The protein amount in the strawberry-leaf slurry obtained through the solid-liquid separation (5) was 0.21 g. Meanwhile, the protein amount in the strawberry leaves (25 g) used in this example was 1.85 g. Accordingly, relative to the protein amount in the strawberry leaves (25 g) used in this example, the protein amount in the strawberry-leaf slurry obtained through the solid-liquid separation (5) was 11.35%.

The protein amount in the strawberry-leaf extract solution obtained through the solid-liquid separation (5) was 0.16 g. Accordingly, relative to the protein amount in the strawberry leaves (25 g) used in this example, the protein amount in the strawberry-leaf extract solution obtained through the solid-liquid separation (5) was 8.65%. Relative to the protein amount in the strawberry leaves (25 g) used in this example, the total protein amount in the strawberry-leaf slurry and the strawberry-leaf extract solution obtained through the solid-liquid separation (5) was 20%.

The protein content rate in the dry strawberry-leaf slurry obtained through the solid-liquid separation (5) was 33.95%. A dry powder solid in which proteins are concentrated is obtained by drying the dry strawberry-leaf slurry using a hot-air dryer. Therefore, it was found that a dry solid in which proteins are concentrated can be obtained through the solid-liquid separation as described above.

It was found from the results above that fibers derived from strawberry leaves, dietary fibers, proteins derived from strawberry leaves, a solid containing components derived from strawberry leaves, an extract solution, and the like can be efficiently extracted from strawberry leaves and the like, which are usually discarded, by subjecting the strawberry leaves and the like to the decomposition and the separation in the same manner as in Example 1. Also, active components such as proteins can be separated and extracted from the extract solution obtained through the solid-liquid separation (5) in subsequent processes using techniques such as filtration, concentration-separation, freeze-concentration spray drying, and evaporation treatment.

[Example 7]

(Decomposition and Separation of Tomato Leaves)

Tomato leaves were subjected to the decomposition and the separation in the same manner as in Example 1. This example was different from Example 1 in that the tomato leaves (dry weight: 25 g) were immersed in 500 ml of water (50°C, pH 4.0) containing an enzyme (cellulase, 3 g) for 24 hours, and were then homogenized under agitation at 4,000 rpm for 1 hour using the biological tissue processing apparatus 1A shown in FIGs. 1A-1B. FIGS. 35A and 35B show the immersed tomato leaves before and after being homogenized under agitation. Note that the protein amount in the tomato leaves (25 g) was 5.53 g, and the protein content rate in the tomato leaves (25 g) was 22.12%. Note that raw leaves, stem portions, and the like left after plucking tomatoes were used as the tomato leaves and were subjected to the processing above. These raw leaves, stem portions, and the like are usually discarded.

(Solid-Liquid Separation (1))

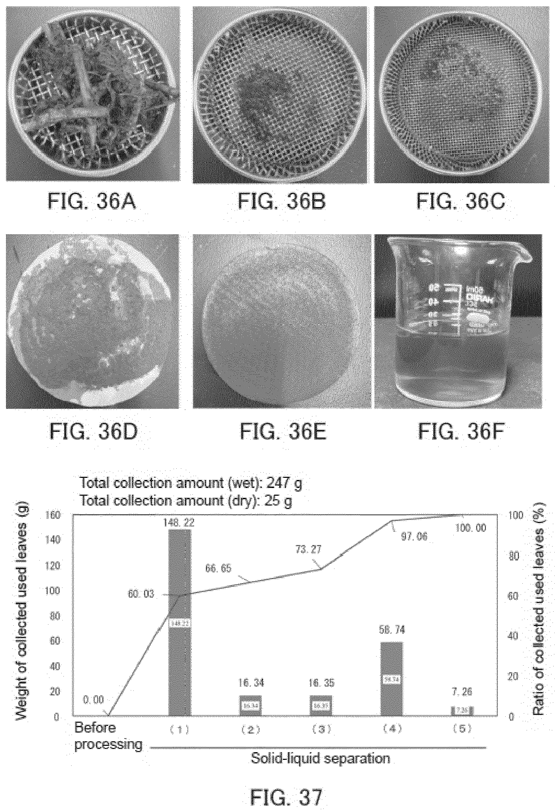

A processed product of the tomato leaves was poured onto a commercially available sieving net (mesh: 3.35 mm square). A tomato-leaf slurry remained on the net (FIG. 36A), and a tomato-leaf extract solution passed through the net and fell into a receiving container. Thus, the tomato-leaf slurry (wet) and the tomato-leaf extract solution were separated and obtained. The weight of the tomato-leaf slurry (wet) was 148.22 g, and was 15.01 g in terms of the weight of the tomato-leaf slurry (dry). The protein amount in the tomato-leaf slurry was 2.96 g. The protein content rates in the wet tomato-leaf slurry and the dry tomato-leaf slurry were 2.00% and 19.71%, respectively.

(Solid-Liquid Separation (2))

The tomato-leaf extract solution obtained through the solid-liquid separation (1) was poured onto a commercially available sieving net (mesh: 1.70 mm square). A tomato-leaf slurry remained on the net (FIG. 36B), and a tomato-leaf extract solution passed through the net and fell into a receiving container. Thus, the tomato-leaf slurry (wet) and the tomato-leaf extract solution were separated and obtained. The weight of the tomato-leaf slurry (wet) was 16.34 g, and was 1.65 g in terms of the weight of the tomato-leaf slurry (dry). The protein amount in the tomato-leaf slurry was 0.42 g. The protein content rates in the wet tomato-leaf slurry and the dry tomato-leaf slurry were 2.58% and 25.47%, respectively.

(Solid-Liquid Separation (3))

The tomato-leaf extract solution obtained through the solid-liquid separation (2) was poured onto a commercially available sieving net (mesh: 1.00 mm square). A tomato-leaf slurry remained on the net (FIG. 36C), and a tomato-leaf extract solution passed through the net and fell into a receiving container. Thus, the tomato-leaf slurry (wet) and the tomato-leaf extract solution were separated and obtained. The weight of the tomato-leaf slurry (wet) was 16.35 g, and was 1.66 g in terms of the weight of the tomato-leaf slurry (dry). The protein amount in the tomato-leaf slurry was 0.06 g. The protein content rates in the wet tomato-leaf slurry and the dry tomato-leaf slurry were 0.35% and 3.44%, respectively.

(Solid-Liquid Separation (4))

The tomato-leaf extract solution obtained through the solid-liquid separation (3) was poured onto a commercially available filter (aperture: 100 μm). A tomato-leaf slurry remained on the filter (FIG. 36D), and a tomato-leaf extract solution passed through the filter and fell into a receiving container. Thus, the tomato-leaf slurry (wet) and the tomato-leaf extract solution were separated and obtained. The weight of the tomato-leaf slurry (wet) was 58.74 g, and was 5.95 g in terms of the weight of the tomato-leaf slurry (dry). The protein amount in the tomato-leaf slurry was 0.62 g. The protein content rates in the wet tomato-leaf slurry and the dry tomato-leaf slurry were 1.06% and 10.51%, respectively.

(Solid-Liquid Separation (5))

The tomato-leaf extract solution obtained through the solid-liquid separation (4) was poured onto commercially available filter paper (aperture: 1.00 μm). A tomato-leaf slurry remained on the filter paper (FIG. 36E), and a tomato-leaf extract solution passed through the filter paper and fell into a receiving container (FIG. 36F). Thus, the tomato-leaf slurry (wet) and the tomato-leaf extract solution were separated and obtained. The weight of the tomato-leaf slurry (wet) was 7.26 g, and was 0.74 g in terms of the weight of the tomato-leaf slurry (dry). The protein amount in the tomato-leaf slurry was 0.48 g. The protein content rates in the wet tomato-leaf slurry and the dry tomato-leaf slurry were 6.59% and 65.10%, respectively. The weight of the tomato-leaf extract solution obtained after the solid-liquid separation (5) was 253.09 g. The protein amount in the tomato-leaf extract solution was 0.99 g. The protein content rate in the tomato-leaf extract solution was 0.39%.

(Results and Discussion)

FIG. 37 shows the results of the weights of the wet tomato-leaf slurries obtained in Example 7. In FIG. 37, the horizontal axis indicates the solid-liquid separation, and the vertical axes indicate the weight of the wet tomato-leaf slurry obtained in the solid-liquid separation (weight of collected used leaves, bar graph, left axis) and the ratio thereof to the total weight (ratio of collected used leaves, line graph, right axis). As shown in FIG. 37, the weight of the wet tomato-leaf slurry obtained through the solid-liquid separation (1) was 148.22 g. Meanwhile, the total weight of the wet tomato-leaf slurries obtained through all the solid-liquid separation (1) to (5) was 247 g. Accordingly, the ratio of the weight of the wet tomato-leaf slurry obtained through the solid-liquid separation (1) to the total weight was 60.03%.

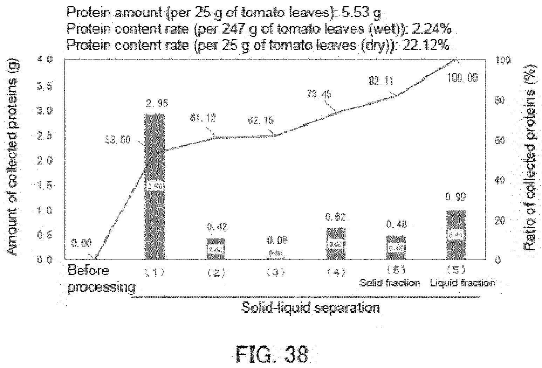

FIG. 38 shows the results of the protein amounts obtained in Example 7. In FIG. 38, the horizontal axis indicates the solid-liquid separation, and the vertical axes indicate the protein amount obtained in the solid-liquid separation (weight of collected used leaves, bar graph, left axis) and the ratio thereof to the total protein amount (ratio of collected used leaves, line graph, right axis). As shown in FIG. 38, the protein amount obtained through the solid-liquid separation (1) was 2.96 g. Meanwhile, the total protein amount obtained through all the solid-liquid separation (1) to (5) was 5.53 g. Accordingly, the ratio of the protein amount obtained through the solid-liquid separation (1) to the total protein amount was 53.50%.

The protein amount in the tomato-leaf slurry obtained through the solid-liquid separation (5) was 0.48 g. Meanwhile, the protein amount in the tomato leaves (25 g) used in this example was 5.53 g. Accordingly, relative to the protein amount in the tomato leaves (25 g) used in this example, the protein amount in the tomato-leaf slurry obtained through the solid-liquid separation (5) was 8.68%.

The protein amount in the tomato-leaf extract solution obtained through the solid-liquid separation (5) was 0.99 g. Accordingly, relative to the protein amount in the tomato leaves (25 g) used in this example, the protein amount in the tomato-leaf extract solution obtained through the solid-liquid separation (5) was 17.90%. Relative to the protein amount in the tomato leaves (25 g) used in this example, the total protein amount in the tomato-leaf slurry and the tomato-leaf extract solution obtained through the solid-liquid separation (5) was 26.58%.

The protein content rate in the dry tomato-leaf slurry obtained through the solid-liquid separation (5) was 65.10%. A dry powder solid in which proteins are concentrated is obtained by drying the dry tomato-leaf slurry using a hot-air dryer. Therefore, it was found that a dry solid in which proteins are concentrated can be obtained through the solid-liquid separation as described above.

It was found from the results above that fibers derived from tomato leaves, dietary fibers, proteins derived from tomato leaves, an extract solution containing components derived from tomato leaves, and the like can be efficiently extracted from tomato leaves and the like, which are usually discarded, by subjecting the tomato leaves and the like to the decomposition and the separation in the same manner as in Example 1. Also, active components such as proteins can be separated and extracted from the extract solution obtained through the solid-liquid separation (5) in subsequent processes using techniques such as filtration, concentration-separation, freeze-concentration spray drying, and evaporation treatment.

Various processing conditions (e.g., the solution amount, the water temperature, the immersing time, the agitation, the homogenization time, the rotation rate during the homogenization under agitation, the type of enzyme, the enzyme amount, and the like) in the examples above can be determined as appropriate in accordance with a processing target, an extract of interest, and the like.

The aperture sizes of the sieving net, the filter, the filter paper, etc., the frequency of the solid-liquid separation, and the like can also be determined as appropriate in accordance with a processing target, an extract of interest, and the like. For example, it is also possible to reduce the frequency of the solid-liquid separation from five to two or three, or increase it from five to six or seven. For example, when the proteins in Example 3 are taken as an example, it is also possible to conduct the solid-liquid separation twice using a filter, filter paper, and the like with an aperture of 100 $\mu$m and 1.00 $\mu$m in consideration of the protein sizes.

In Examples 4 and 5, the raw leaves and the coffee beans were subjected to the pretreatment (the former was pulverized using a food mill, and the latter was pulverized through mill-grinding), and were then subjected to the decomposition and the separation of the present disclosure. Such pretreatment is also effective for the strawberry leaves in Example 6 and the tomato leaves in Example 7. In the present disclosure, it is possible to increase the rate of extraction of an active component and the like by selecting favorable pretreatment in accordance with the type of leaf and conducting the decomposition and the separation of the present disclosure after subjecting leaves to the pretreatment. Such pretreatment is widely effective for biological tissues of plants and the like in addition to the examples above. In the present disclosure, it is possible to increase the rate of extraction of an active component and the like by selecting favorable pretreatment in accordance with the type of biological tissue and conducting the decomposition and the separation of the present disclosure after subjecting a biological tissue to the pretreatment.

Any pretreatment may be employed as long as it applies a physical stimulus on (e.g., crumples, crushes, or scratches) a biological tissue in advance. The pretreatment is conducted to, for example, apply a physical stimulus on cell walls in leaves, stems, and the like, and examples thereof include food-mill pulverization, mill-grinding pulverization, homogenization, shearing, heat treatment, rolling (crumpling process), wringing, compressing or pressing, squeezing, crushing, friction welding, and the like.

[Example 8]

(Decomposition of Tea Leaves)

100 L of water was placed in a container with a capacity of 200 L, and the water was heated to a temperature of 35°C using a liquid heating heater (Model Number: WPS-110, Kashima Co., Ltd.). 600 ml (corresponding to 0.6% of 100 L, the volume of the water) of cellulase was added to and mixed with the water. Next, 5 kg of the tea leaves were added to the water and were immersed therein. Sampling was conducted on the prepared mixed solution at timing shown in Table 3 below, and the pH of the mixed solution was measured. In the container, the mixed solution was agitated (4,000 rpm) using a homogenizer (Auto Mixer Model 40, PRIMIX Corporation) at timing shown in Table 3 below. The agitation was conducted in a rotational direction opposite to the rotational direction causing homogenization under agitation so as not to homogenize the tea leaves under agitation (i.e., so as to only agitate the mixed solution). The mixed solution was agitated using the homogenizer at a timing shown in Table 3 below, and the amount of electric current used by the homogenizer was measured. The immersion (decomposition) was conducted for about 4 hours. The tea leaves used were prepared as follows: raw leaves just after being plucked from a tea tree were subjected to enzyme deactivation for 60 seconds by supplying steam at 100°C thereto under agitation.

(Results and Discussion)

Table 3 shows the measurement results of the pH of the mixed solution and the amount of electric current used by the homogenizer. As shown in Table 3, the pH of the mixed solution was 4.0 at the start of the immersion, but was substantially constant around 4.8 about 2 to 3 hours after the start of the immersion. Also, as shown in Table 3, the amount of electric current used by the homogenizer at the start of the immersion was 1.9 A, but was substantially constant around 3.0 A about 2 to 3 hours after the start of the immersion.

It can be considered from the results above that the state of the mixed solution became constant about 2 to 3 hours after the start of the immersion, and the immersion (decomposition) was sufficiently conducted. Accordingly, it is considered that starting the separation by the homogenizer at timing about 2 to 3 hours after the start of the immersion makes it possible to efficiently process a biological tissue without continuing the immersion for an unnecessarily long period of time.

The pH of the mixed solution exceeded 5 until about 1 hour elapsed since the immersion was started, and it is considered that the reason for this is that the tea leaves were decomposed and thus the components thereof started to mix into the mixed solution. Also, it is considered that the reason why the amount of electric current used by the homogenizer had a tendency to increase since the immersion was started and became substantially constant about 2 to 3 hours after the start of the immersion is that as the immersion (decomposition) progressed, the viscosity of the mixed solution increased, the viscosity became substantially constant by sufficiently conducting the immersion, and along with this, the resistance applied to the homogenizer due to the viscosity of the mixed solution became substantially constant.

[Table 3]

| Table 3. Measurement results of pH of mixed solution and amount of electric current used by homogenizer | | | |
|---|---|---|---|
| Time (hh:mm) | pH of mixed solution | Amount of electric current used by homogenizer (A) | Agitation by homogenizer (no homogenization under agitation) |
| 00:00 | 4.0 | 1.9 | Start |
| 00:10 | 5.43 | | End |
| 00:55 | 5.07 | 2.5 | Start, and end after sampling |
| 01:54 | 4.89 | 2.6 | Start, and end after sampling |
| 02:55 | 4.79 | 3.4 | Start, and end after sampling |
| 03:54 | 4.73 | 3.2 | Start, and end after sampling |

[Example 9]

(Separation of Tea Leaves)

The decomposition product of the tea leaves obtained in Example 8 was homogenized under agitation at 4,000 rpm for about 1 hour using a homogenizer (Auto Mixer Model 40, PRIMIX Corporation) in the container (separation). After 5 minutes elapsed since the homogenization under agitation was started, the processed product obtained through the homogenization under agitation was circulated via a circulation pipe attached at two portions of the container, namely the upper and lower portions thereof, in the order of the container, the circulation pipe, and the container. A pump was disposed in the circulation pipe. The flow rate of the processed product in the circulation pipe was measured at timing shown in Table 4 below. Also, the amount of electric current used by the homogenizer was measured at a timing shown in Table 4 below.

(Results and Discussion)

Table 4 shows the measurement results of the flow rate of the processed product in the circulation pipe and the amount of electric current used by the homogenizer. As shown in Table 4, the flow rate of the processed product in the circulation pipe was 0 L/minute at the start of the immersion, but was substantially constant around 55 L/minute about 45 minutes after the start of the immersion. Also, as shown in Table 4, the amount of electric current used by the homogenizer at the start of the immersion was 3.7 A, but was substantially constant around 2.5 A about 45 minutes after the start of the immersion.

It can be considered from the results above that the state of the processed product became constant about 45 minutes after the start of the homogenization under agitation, and the homogenization under agitation (separation) was sufficiently conducted. Accordingly, it is considered that finishing the separation by the homogenizer at timing about 45 minutes after the start of the homogenization under agitation makes it possible to efficiently process a biological tissue without continuing the homogenization under agitation for an unnecessarily long period of time.

The flow rate of the processed product in the circulation pipe increased over time after the homogenization under agitation, and it is considered that the reason for this is that the particle size of the processed product in the circulation pipe, which had been large at the start of the homogenization under agitation, decreased over time after the homogenization under agitation, and thus the circulation was less likely to be inhibited. Also the amount of electric current used by the homogenizer decreased over time after the homogenization under agitation, and it is similarly considered that the reason for this is also that the particle size of the processed product in the circulation pipe, which had been large at the start of the homogenization under agitation, decreased over time after the homogenization under agitation, and thus the resistance applied to the homogenizer due to the processed product decreased.

The flow rate of the processed product in the circulation pipe once increased at the start of the circulation (5 minutes after the homogenization under agitation), and it is considered that the reason for this is that the supernatant solution in the container preferentially passed through the circulation pipe and then the precipitation in the container passed through the circulation pipe.

[Table 4]

| Table 4. Measurement results of flow rate of processed product in circulation pipe and amount of electric current used by homogenizer | | |
|---|---|---|
| Time (hh:mm) | Flow rate of processed product in circulation pipe (L/min) | Amount of electric current used by homogenizer (A) |
| 00:00 | 0 | 3.7 |
| 00:05 | 46 | 3.1 |
| 00:15 | 38 | 2.9 |
| 00:30 | 36 | 2.7 |
| 00:45 | 51 | 2.5 |
| 01:00 | 56 | 2.5 |

[Example 10]

(Solid-Liquid Separation of Processed Product)

The processed product of the tea leaves obtained in Example 9 was subjected to solid-liquid separation using a screw press (44.59 Hz). The screw press was disposed in the circulation pipe. A solid fraction obtained through the solid-liquid separation was collected in a container different from the container above. The rotation number of the pump disposed in the circulation pipe was set to 7.85 Hz or 4.22 Hz, and the state of the solid fraction collected at each rotation rate was visually confirmed.

(Results and Discussion)

When the rotation rate of the pump disposed in the circulation pipe was set to 7.85 Hz, the solid fraction contained a liquid in a large amount. Meanwhile, when the rotation rate of the pump disposed in the circulation pipe was set to 4.22 Hz, the solid fraction contained a liquid in a small amount.

It is considered from the results above that the amount of a liquid contained in the solid fraction is reduced by reducing the rotation rate of the pump, thus making it possible to efficiently collect the solid fraction.

It is considered that when the rotation rate of the screw press is excessively large, the screw press processes the processed product in an excessive amount, and thus the amount of a liquid contained in the solid fraction increases. Also, it is considered that when the rotation rate of the pump is excessively large, the pump supplies the processed product in an excessive amount to the screw press, and thus the amount of a liquid contained in the solid fraction increases. Accordingly, it is considered that it is important to adjust the rotation rate of the screw press and/or the rotation rate of the pump within a suitable range in order to reduce the amount of a liquid contained in the solid fraction and efficiently collect the solid fraction.

Various features of the present disclosure described in the present disclosure can be combined in various ways, and all aspects obtained by such combinations including combinations that are not specifically described in the present disclosure fall within the scope of the present disclosure. Those skilled in the art understand that many various modifications can be made without departing from the spirit of the present disclosure, and equivalents that include such modifications fall within the scope of the present disclosure. Therefore, the aspects described in the present disclosure are for illustrative purposes only, and are not construed as limiting the scope of the present disclosure.

The present application claims the benefit of priority from Japanese Patent Application No. 2023-19517 filed on February 10, 2023, and Japanese Patent Application No. 2023-152830 filed on September 20, 2023, the entire disclosure of which is incorporated herein.

The contents of the patents, patent applications, and references cited herein are incorporated herein by reference in their entireties as if specifically set forth herein.

<Supplementary Notes>

Some or all of the embodiments and the examples given above can be described as in the following supplementary notes. However, the scope of the present invention is not limited thereto.

(Supplementary Note 1)

A method for producing an extract of a biological tissue, including:

a step of decomposing extracellular matrix of the biological tissue with an enzyme; and
a step of separating cells of the biological tissue and/or an extract of the biological tissue from the extracellular matrix by subjecting the biological tissue to separation.

(Supplementary Note 2)

The production method according to Supplementary Note 1, wherein the separation includes a processing operation for mechanically shearing the extracellular matrix of the biological tissue.

(Supplementary Note 3)

The production method according to Supplementary Note 1 or 2, wherein the separation includes a processing operation for mechanically shearing the extracellular matrix of the biological tissue by bringing the biological tissue into contact with an agitation blade having a shearing blade.

(Supplementary Note 4)

The production method according to any one of Supplementary Notes 1 to 3, wherein the extracellular matrix includes a cell wall, collagen, and/or proteoglycan.

(Supplementary Note 5)

The production method according to any one of Supplementary Notes 1 to 4, wherein the extract includes a cellular component of the biological tissue.

(Supplementary Note 6)

The production method according to any one of Supplementary Notes 1 to 5, wherein the cellular component is a low-molecular-weight compound, a nucleic acid, a peptide, a protein, and/or extracellular matrix.

(Supplementary Note 7)

The production method according to any one of Supplementary Notes 1 to 6, wherein the extract is a liquid or a solid.

(Supplementary Note 8)

The production method according to any one of Supplementary Notes 1 to 7, wherein the separation includes a processing operation for separating a liquid and a solid of the biological tissue from each other.

(Supplementary Note 9)

The production method according to any one of Supplementary Notes 1 to 8, wherein at least portions of the decomposition and the separation are simultaneously conducted.

(Supplementary Note 10)

The production method according to any one of Supplementary Notes 1 to 9, wherein the decomposition and/or the separation is conducted a plurality of times.

(Supplementary Note 11)

The production method according to any one of Supplementary Notes 1 to 10, wherein the decomposition includes a processing operation for decomposing the extracellular matrix of the biological tissue with a microbial culture solution containing the enzyme.

(Supplementary Note 12)

The production method according to any one of Supplementary Notes 1 to 11, wherein the enzyme is a protease, a cellulase, a hemicellulase, and/or a pectinase.

(Supplementary Note 13)

The production method according to any one of Supplementary Notes 1 to 12, wherein the biological tissue is a plant tissue.

(Supplementary Note 14)

The production method according to Supplementary Note 13, wherein the plant is a tea plant (tea), a coffee plant (coffee), a moringa, a Brussels sprout, a cabbage, a tomato, and/or a bamboo.

(Supplementary Note 15)

The production method according to Supplementary Note 13 or 14, wherein the plant tissue is a leaf, a stem, a flower, a seed, a root, a trunk, a skin (e.g., a bark, an epidermis, and a bast), sap, and/or a fruit, or a processed product thereof.

(Supplementary Note 16)

The production method according to any one of Supplementary Notes 13 to 15, wherein the plant tissue is a tissue of a tea leaf or a used tea leaf.

(Supplementary Note 17)

An extract of a biological tissue produced using the production method according to any one of Supplementary Notes 1 to 16.

(Supplementary Note 18)

The extract according to Supplementary Note 17, including a cellulose fiber.

(Supplementary Note 19)

A food composition including the extract according to Supplementary Note 17 or 18.

(Supplementary Note 20)

A method for extracting an extract of a biological tissue, including:

a step of decomposing the extracellular matrix of the biological tissue with an enzyme; and
a step of separating cells of the biological tissue and/or an extract of the biological tissue from the extracellular matrix by subjecting the biological tissue to separation.

(Supplementary Note 21)

A biological tissue processing apparatus for processing a mixed solution obtained by mixing a biological tissue and a solution containing an enzyme, including:

a housing container in which the mixed solution is housed and decomposition is conducted for decomposing the extracellular matrix of the biological tissue in the mixed solution with the enzyme; and
a separation device for separating cells of the biological tissue and/or an extract of the biological tissue from the extracellular matrix by applying a force to the biological tissue in the mixed solution.

(Supplementary Note 22)

The biological tissue processing apparatus according to Supplementary Note 21,

wherein the separation device includes an agitation blade that is disposed in the housing container and is configured to agitate the mixed solution in the housing container, and
the agitation blade includes a shearing blade that comes into contact with the biological tissue subjected to the decomposition and applies a shearing force to the extracellular matrix of the biological tissue and/or the cells of the biological tissue.

(Supplementary Note 23)

The biological tissue processing apparatus according to Supplementary Note 21, further including a separation tank provided with the separation device,

wherein the separation tank includes:

a supply channel to which the mixed solution subjected to the decomposition is supplied from the housing container; and
a return channel through which the mixed solution subjected to the decomposition in the decomposition tank is returned to the housing container,

the separation device includes an agitation blade that is disposed in the separation tank and is configured to agitate the mixed solution in the separation tank, and
the agitation blade includes a shearing blade that comes into contact with the biological tissue subjected to the decomposition in the housing container and applies a shearing force to the extracellular matrix of the biological tissue and/or the cells of the biological tissue.(Supplementary Note 24)

The cell processing apparatus according to Supplementary Note 23, including a plurality of the housing containers,

wherein between the housing containers and the separation tank, provided are
a plurality of the supply channels through which the housing containers and the separation tank are in communication,
a supply-side switching mechanism for switching any of the plurality of the supply channels to a channel for supplying the mixed solution to the separation tank,
a plurality of the return channels through which the housing containers and the separation tank are in communication, and
a return-side switching mechanism for switching any of the plurality of the return channels to a channel for returning the mixed solution from the separation tank.

(Supplementary Note 25)

The cell processing apparatus according to any one of Supplementary Notes 22 to 24, further including:

a driving source for driving the agitation blade;
a sensor for detecting a state of the mixed solution in the housing container and/or a state of the mixed solution in the separation tank; and
a control unit for controlling an operation of the driving source based on a signal from the sensor.

(Supplementary Note 26)

The biological tissue processing apparatus according to Supplementary Note 21, further including a circulation channel that is disposed outside the housing container and through which the mixed solution subjected to the decomposition in the housing container is circulated,

wherein the separation device is provided at an intermediate portion of the circulation channel, and
the separation device includes a shearing blade that comes into contact with the biological tissue flowing in the circulation channel and applies a shearing force to the extracellular matrix of the biological tissue and/or the cells of the biological tissue.

(Supplementary Note 27)

The biological tissue processing apparatus according to Supplementary Note 26, including a plurality of the housing containers,
wherein the circulation channel includes:

a plurality of supply channels through which the housing containers and the separation tank are in communication,
a supply-side switching mechanism for switching any of the plurality of supply channels to a channel for supplying the mixed solution to the separation device,
a plurality of return channels through which the separation device and the housing containers are in communication, and
a return-side switching mechanism for switching any of the plurality of return channels to a channel that is in communication with the separation device.

(Supplementary Note 28)

The biological tissue processing apparatus according to Supplementary Note 26 or 27,

wherein the circulation channel includes:

first and second return channels for returning the mixed solution passing through the separation device to the housing container; and
a switching mechanism that is provided between the separation device and the first and second return channels and is configured to switch a channel for supplying the mixed solution from the separation device between the first and second return channels,

the first return channel includes an enzyme addition unit for adding an enzyme to the mixed solution, and
the second return channel includes a solid-liquid separation device for separating a solid from the mixed solution and/or a component collection portion for collecting a component contained in the cells of the biological tissue from the mixed solution.

(Supplementary Note 29)

The biological tissue processing apparatus according to any one of Supplementary Notes 21 to 28, further including:

an enzyme supply unit for supplying a solution containing an enzyme to a solution in the housing container; and
an electrolyzed water supply unit for supplying electrolyzed water to the solution in the housing container,
wherein the electrolyzed water supply unit includes:

an electrolyzed water production device for generating the electrolyzed water; and
an adjusting unit to which the electrolyzed water is supplied from the electrolyzed water production device and that is configured to adjust a state of the electrolyzed water.

(Supplementary Note 30)

The biological tissue processing apparatus according to any one of Supplementary Notes 21 to 29, further including an adsorption portion for adsorbing a component contained in the cells of the biological tissue contained in the solution in the housing container.

(Supplementary Note 31)

The biological tissue processing apparatus according to any one of Supplementary Notes 21 to 30, further including a biological tissue supply unit for supplying the biological tissue to the solution in the housing container.

(Supplementary Note 32)

A biological tissue processing apparatus including:

a first housing container that is capable of housing a mixed solution obtained by mixing a biological tissue and a solution containing an enzyme and is configured to be capable of decomposing the extracellular matrix of the biological tissue in the mixed solution with the enzyme;
a separation device that is configured to be capable of applying a force to the biological tissue in the mixed solution and separating cells of the biological tissue and/or an extract of the biological tissue from the extracellular matrix;
a first measurement device that is configured to be capable of measuring a state of the mixed solution over time; and
a first control device that is configured to be capable of controlling separation of a decomposition product of the biological tissue by the separation device based on the state of the mixed solution obtained in the measurement.

(Supplementary Note 33)

The processing apparatus according to Supplementary Note 32, wherein the state of the mixed solution is a viscosity, pH, temperature, turbidity, color, and/or air bubble density of the mixed solution.

(Supplementary Note 34)

The processing apparatus according to Supplementary Note 32 or 33, wherein the first control device is capable of controlling the separation of the decomposition product of the biological tissue by the separation device based on a change in the state of the mixed solution over time.

(Supplementary Note 35)

The processing apparatus according to any one of Supplementary Notes 32 to 34, wherein the separation device includes:

an agitation blade that is configured to be capable of agitating the mixed solution in the first housing container; and
a driving unit that is configured to be capable of driving the agitation blade.

(Supplementary Note 36)

The processing apparatus according to Supplementary Note 35, wherein the agitation blade includes a shearing blade that is configured to come into contact with the biological tissue in the mixed solution and be capable of applying a shearing force to the extracellular matrix of the biological tissue and/or the cells of the biological tissue.

(Supplementary Note 37)

The processing apparatus according to Supplementary Note 35 or 36, wherein the first control device is capable of controlling the separation of the decomposition product of the biological tissue by the separation device by controlling a rotation rate and/or rotational direction of the agitation blade.

(Supplementary Note 38)

The processing apparatus according to any one of Supplementary Notes 32 to 37, further including:

a second measurement device that is configured to be capable of measuring a state of the separation device over time; and
a second control device that is configured to be capable of controlling the separation of the decomposition product of the biological tissue by the separation device based on the state of the separation device obtained in the measurement.

(Supplementary Note 39)

The processing apparatus according to Supplementary Note 38, wherein the second control device is capable of controlling the separation of the decomposition product of the biological tissue by the separation device based on a change in the state of the separation device over time.

(Supplementary Note 40)

The processing apparatus according to Supplementary Note 38 or 39,
wherein the separation device includes:

an agitation blade that is configured to be capable of agitating the mixed solution in the first housing container; and
a driving unit that is configured to be capable of driving the agitation blade.

(Supplementary Note 41)

The processing apparatus according to Supplementary Note 40, wherein the state of the separation device is an amount of electric current used by the driving unit and/or an amount of power used by the driving unit.

(Supplementary Note 42)

The processing apparatus according to Supplementary Note 40 or 41, wherein the second control device is capable of controlling the separation of the decomposition product of the biological tissue by the separation device by controlling a rotation rate, rotational torque, rotational direction, and/or rotational pattern of the agitation blade.

(Supplementary Note 43)

The processing apparatus according to any one of Supplementary Notes 32 to 42, further including a channel that is configured to allow the mixed solution in the first housing container to be circulated therethrough.

(Supplementary Note 44)

The processing apparatus according to Supplementary Note 43, further including:

a liquid feeding device that is configured to be capable of feeding the mixed solution in the first housing container via the channel;
a third measurement device that is configured to be capable of measuring a state of the mixed solution in the channel over time; and
a third control device that is configured to be capable of controlling liquid feeding by the liquid feeding device based on the state of the mixed solution in the channel obtained in the measurement.

(Supplementary Note 45)

The processing apparatus according to Supplementary Note 44, wherein the liquid feeding device is disposed in the channel.

(Supplementary Note 46)

The processing apparatus according to Supplementary Note 44 or 45, wherein the state of the mixed solution in the channel is a flow rate, flow speed, viscosity, and/or turbidity of the mixed solution.

(Supplementary Note 47)

The processing apparatus according to any one of Supplementary Notes 44 to 46, wherein the third control device is capable of controlling the liquid feeding by the liquid feeding device based on a change in the state of the mixed solution in the channel over time.

(Supplementary Note 48)

The processing apparatus according to any one of Supplementary Notes 44 to 47, wherein the third control device is capable of controlling the liquid feeding by the liquid feeding device by controlling a liquid feeding amount (ejection amount), rotation rate, and/or rotational torque of the liquid feeding device.

(Supplementary Note 49)

The processing apparatus according to any one of Supplementary Notes 32 to 48, further including a solid-liquid separation device that is configured to be capable of conducting solid-liquid separation of the extract.

(Supplementary Note 50)

The processing apparatus according to Supplementary Note 49, further including a second housing container that is configured to be capable of housing a solid fraction obtained through the solid-liquid separation of the extract.

(Supplementary Note 51)

The processing apparatus according to Supplementary Note 50, further including:

a fourth measurement device that is configured to be capable of measuring a state of the solid fraction in the second housing container over time; and
a fourth control device that is configured to be capable of controlling the solid-liquid separation of the extract by the solid-liquid separation device based on the state of the solid fraction obtained in the measurement.

(Supplementary Note 52)

The processing apparatus according to Supplementary Note 51, wherein the state of the solid fraction in the second housing container is an amount of a liquid contained in the solid fraction, an amount of a solid contained in the solid fraction, and/or a ratio between the liquid amount and the solid amount.

(Supplementary Note 53)

The processing apparatus according to Supplementary Note 51 or 52, wherein the fourth control device is capable of controlling the solid-liquid separation of the extract by the solid-liquid separation device based on a change in the state of the solid fraction over time.

(Supplementary Note 54)

The processing apparatus according to any one of Supplementary Notes 51 to 53, wherein the solid-liquid separation device includes a screw press, a screw conveyor, a roller press, a belt screen, and/or a cyclone.

(Supplementary Note 55)

The processing apparatus according to Supplementary Note 54, wherein the fourth control device is capable of controlling the solid-liquid separation of the extract by the solid-liquid separation device by controlling a rotation rate of the screw press, the screw conveyor, the roller press, the belt screen, and/or the cyclone.

(Supplementary Note 56)

The processing apparatus according to any one of Supplementary Notes 32 to 55, further including:

a solid-liquid separation device that is configured to be capable of conducting solid-liquid separation of the extract;
a second housing container that is configured to be capable of housing a solid fraction obtained through the solid-liquid separation of the extract;
a channel that is configured to allow the extract to be circulated therethrough via the solid-liquid separation device; and
a liquid feeding device that is configured to be capable of feeding the extract via the channel.

(Supplementary Note 57)

The processing apparatus according to Supplementary Note 56, further including:

a fourth measurement device that is configured to be capable of measuring a state of the solid fraction in the second housing container over time; and
a fifth control device that is configured to be capable of controlling supply of the extract to the solid-liquid separation device via the channel based on the state of the solid fraction obtained in the measurement.

(Supplementary Note 58)

The processing apparatus according to Supplementary Note 57, wherein the state of the solid fraction in the second housing container is an amount of a liquid contained in the solid fraction, an amount of a solid contained in the solid fraction, and/or a ratio between the liquid amount and the solid amount.

(Supplementary Note 59)

The processing apparatus according to Supplementary Note 57 or 58, wherein the fifth control device is capable of controlling the supply of the extract to the solid-liquid separation device via the channel based on a change in the state of the solid fraction over time.

(Supplementary Note 60)

The processing apparatus according to any one of Supplementary Notes 57 to 59, wherein the fifth control device is capable of controlling the supply of the extract to the solid-liquid separation device by controlling a liquid feeding amount (ejection amount), rotation rate, and/or rotational torque of the liquid feeding device.

(Supplementary Note 61)

A biological tissue processing apparatus including:

a first housing container that is capable of housing a mixed solution obtained by mixing a biological tissue and a solution containing an enzyme and is configured to be capable of decomposing the extracellular matrix of the biological tissue in the mixed solution with the enzyme;
a separation device that is configured to be capable of applying a force to the biological tissue in the mixed solution and separating cells of the biological tissue and/or an extract of the biological tissue from the extracellular matrix;
a second measurement device that is configured to be capable of measuring a state of the separation device over time; and
a second control device that is configured to be capable of controlling separation of a decomposition product of the biological tissue by the separation device based on the state of the separation device obtained in the measurement.

(Supplementary Note 62)

The processing apparatus according to Supplementary Note 61, wherein the second control device is capable of controlling the separation of the decomposition product of the biological tissue by the separation device based on a change in the state of the separation device over time.

(Supplementary Note 63)

The processing apparatus according to Supplementary Note 61 or 62,
wherein the separation device includes:

an agitation blade that is configured to be capable of agitating the mixed solution in the first housing container; and
a driving unit that is configured to be capable of driving the agitation blade.

(Supplementary Note 64)

The processing apparatus according to Supplementary Note 63, wherein the state of the separation device is an amount of electric current used by the driving unit and/or an amount of power used by the driving unit.

(Supplementary Note 65)

The processing apparatus according to Supplementary Note 63 or 64, wherein the second control device is capable of controlling the separation of the decomposition product of the biological tissue by the separation device by controlling a rotation rate, rotational torque, rotational direction, and/or rotational pattern of the agitation blade.

(Supplementary Note 66)

The processing apparatus according to any one of Supplementary Notes 61 to 65, further including a channel that is configured to allow the mixed solution in the first housing container to be circulated therethrough.

(Supplementary Note 67)

The processing apparatus according to Supplementary Note 66, further including:

a liquid feeding device that is configured to be capable of feeding the mixed solution in the first housing container via the channel;
a third measurement device that is configured to be capable of measuring a state of the mixed solution in the channel over time; and
a third control device that is configured to be capable of controlling liquid feeding by the liquid feeding device based on the state of the mixed solution in the channel obtained in the measurement.

(Supplementary Note 68)

The processing apparatus according to Supplementary Note 67, wherein the liquid feeding device is disposed in the channel.

(Supplementary Note 69)

The processing apparatus according to Supplementary Note 67 or 68, wherein the state of the mixed solution in the channel is a flow rate, flow speed, viscosity, and/or turbidity of the mixed solution.

(Supplementary Note 70)

The processing apparatus according to any one of Supplementary Notes 67 to 69, wherein the third control device is capable of controlling the liquid feeding by the liquid feeding device based on a change in the state of the mixed solution in the channel over time.

(Supplementary Note 71)

The processing apparatus according to any one of Supplementary Notes 67 to 70, wherein the third control device is capable of controlling the liquid feeding by the liquid feeding device by controlling a liquid feeding amount (ejection amount), rotation rate, and/or rotational torque of the liquid feeding device.

(Supplementary Note 72)

The processing apparatus according to any one of Supplementary Notes 61 to 71, further including a solid-liquid separation device that is configured to be capable of conducting solid-liquid separation of the extract.

(Supplementary Note 73)

The processing apparatus according to Supplementary Note 72, further including a second housing container that is configured to be capable of housing a solid fraction obtained through the solid-liquid separation of the extract.

(Supplementary Note 74)

The processing apparatus according to Supplementary Note 73, further including:

a fourth measurement device that is configured to be capable of measuring a state of the solid fraction in the second housing container over time; and
a fourth control device that is configured to be capable of controlling the solid-liquid separation of the extract by the solid-liquid separation device based on the state of the solid fraction obtained in the measurement.

(Supplementary Note 75)

The processing apparatus according to Supplementary Note 74, wherein the state of the solid fraction in the second housing container is an amount of a liquid contained in the solid fraction, an amount of a solid contained in the solid fraction, and/or a ratio between the liquid amount and the solid amount.

(Supplementary Note 76)

The processing apparatus according to Supplementary Note 74 or 75, wherein the fourth control device is capable of controlling the solid-liquid separation of the extract by the solid-liquid separation device based on a change in the state of the solid fraction over time.

(Supplementary Note 77)

The processing apparatus according to any one of Supplementary Notes 74 to 76, wherein the solid-liquid separation device includes a screw press, a screw conveyor, a roller press, a belt screen, and/or a cyclone.

(Supplementary Note 78)

The processing apparatus according to Supplementary Note 77, wherein the fourth control device is capable of controlling the solid-liquid separation of the extract by the solid-liquid separation device by controlling a rotation rate of the screw press, the screw conveyor, the roller press, the belt screen, and/or the cyclone.

(Supplementary Note 79)

The processing apparatus according to any one of Supplementary Notes 61 to 78, further including:

a solid-liquid separation device that is configured to be capable of conducting solid-liquid separation of the extract;
a second housing container that is configured to be capable of housing a solid fraction obtained through the solid-liquid separation of the extract;
a channel that is configured to allow the extract to be circulated therethrough via the solid-liquid separation device; and
a liquid feeding device that is configured to be capable of feeding the extract via the channel.

(Supplementary Note 80)

The processing apparatus according to Supplementary Note 79, further including:

a fourth measurement device that is configured to be capable of measuring a state of the solid fraction in the second housing container over time; and

a fifth control device that is configured to be capable of controlling supply of the extract to the solid-liquid separation device via the channel based on the state of the solid fraction obtained in the measurement.

(Supplementary Note 81)

The processing apparatus according to Supplementary Note 80, wherein the state of the solid fraction in the second housing container is an amount of a liquid contained in the solid fraction, an amount of a solid contained in the solid fraction, and/or a ratio between the liquid amount and the solid amount.

(Supplementary Note 82)

The processing apparatus according to Supplementary Note 80 or 81, wherein the fifth control device is capable of controlling the supply of the extract to the solid-liquid separation device via the channel based on a change in the state of the solid fraction over time.

(Supplementary Note 83)

The processing apparatus according to any one of Supplementary Notes 80 to 82, wherein the fifth control device is capable of controlling the supply of the extract to the solid-liquid separation device by controlling a liquid feeding amount (ejection amount), rotation rate, and/or rotational torque of the liquid feeding device.

(Supplementary Note 84)

A biological tissue processing apparatus including:

a first housing container that is capable of housing a mixed solution obtained by mixing a biological tissue and a solution containing an enzyme and is configured to be capable of decomposing the extracellular matrix of the biological tissue in the mixed solution with the enzyme;
a separation device that is configured to be capable of applying a force to the biological tissue in the mixed solution and separating cells of the biological tissue and/or an extract of the biological tissue from the extracellular matrix;
a channel that is configured to allow the mixed solution in the first housing container to be circulated therethrough;
a liquid feeding device that is configured to be capable of feeding the mixed solution in the first housing container via the channel;
a third measurement device that is configured to be capable of measuring a state of the mixed solution in the channel over time; and
a third control device that is configured to be capable of controlling liquid feeding by the liquid feeding device based on the state of the mixed solution in the channel obtained in the measurement.

(Supplementary Note 85)

The processing apparatus according to Supplementary Note 84, wherein the liquid feeding device is disposed in the channel.

(Supplementary Note 86)

The processing apparatus according to Supplementary Note 84 or 85, wherein the state of the mixed solution in the channel is a flow rate, flow speed, viscosity, and/or turbidity of the mixed solution.

(Supplementary Note 87)

The processing apparatus according to any one of Supplementary Notes 84 to 86, wherein the third control device is capable of controlling the liquid feeding by the liquid feeding device based on a change in the state of the mixed solution in the channel over time.

(Supplementary Note 88)

The processing apparatus according to any one of Supplementary Notes 84 to 87, wherein the third control device is capable of controlling the liquid feeding by the liquid feeding device by controlling a liquid feeding amount (ejection amount), rotation rate, and/or rotational torque of the liquid feeding device.

(Supplementary Note 89)

The processing apparatus according to any one of Supplementary Notes 84 to 88, further including a solid-liquid separation device that is configured to be capable of conducting solid-liquid separation of the extract.

(Supplementary Note 90)

The processing apparatus according to Supplementary Note 89, further including a second housing container that is configured to be capable of housing a solid fraction obtained through the solid-liquid separation of the extract.

(Supplementary Note 91)

The processing apparatus according to Supplementary Note 90, further including:

a fourth measurement device that is configured to be capable of measuring a state of the solid fraction in the second housing container over time; and
a fourth control device that is configured to be capable of controlling the solid-liquid separation of the extract by the solid-liquid separation device based on the state of the solid fraction obtained in the measurement.

(Supplementary Note 92)

The processing apparatus according to Supplementary Note 91, wherein the state of the solid fraction in the second housing container is an amount of a liquid contained in the solid fraction, an amount of a solid contained in the solid fraction, and/or a ratio between the liquid amount and the solid amount.

(Supplementary Note 93)

The processing apparatus according to Supplementary Note 91 or 92, wherein the fourth control device is capable of controlling the solid-liquid separation of the extract by the solid-liquid separation device based on a change in the state of the solid fraction over time.

(Supplementary Note 94)

The processing apparatus according to any one of Supplementary Notes 91 to 93, wherein the solid-liquid separation device includes a screw press, a screw conveyor, a roller press, a belt screen, and/or a cyclone.

(Supplementary Note 95)

The processing apparatus according to Supplementary Note 94, wherein the fourth control device is capable of controlling the solid-liquid separation of the extract by the solid-liquid separation device by controlling a rotation rate of the screw press, the screw conveyor, the roller press, the belt screen, and/or the cyclone.

(Supplementary Note 96)

The processing apparatus according to any one of Supplementary Notes 84 to 95, further including:

a solid-liquid separation device that is configured to be capable of conducting solid-liquid separation of the extract;
a second housing container that is configured to be capable of housing a solid fraction obtained through the solid-liquid separation of the extract;
a channel that is configured to allow the extract to be circulated therethrough via the solid-liquid separation device; and
a liquid feeding device that is configured to be capable of feeding the extract via the channel.

(Supplementary Note 97)

The processing apparatus according to Supplementary Note 96, further including:

a fourth measurement device that is configured to be capable of measuring a state of the solid fraction in the second housing container over time; and

a fifth control device that is configured to be capable of controlling supply of the extract to the solid-liquid separation device via the channel based on the state of the solid fraction obtained in the measurement.

(Supplementary Note 98)

The processing apparatus according to Supplementary Note 97, wherein the state of the solid fraction in the second housing container is an amount of a liquid contained in the solid fraction, an amount of a solid contained in the solid fraction, and/or a ratio between the liquid amount and the solid amount.

(Supplementary Note 99)

The processing apparatus according to Supplementary Note 97 or 98, wherein the fifth control device is capable of controlling the supply of the extract to the solid-liquid separation device via the channel based on a change in the state of the solid fraction over time.

(Supplementary Note 100)

The processing apparatus according to Supplementary Note 99, wherein the fifth control device is capable of controlling the supply of the extract to the solid-liquid separation device by controlling a liquid feeding amount (ejection amount), rotation rate, and/or rotational torque of the liquid feeding device.

(Supplementary Note 101)

A biological tissue processing apparatus including:

a first housing container that is capable of housing a mixed solution obtained by mixing a biological tissue and a solution containing an enzyme and is configured to be capable of decomposing the extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a solid-liquid separation device that is configured to be capable of conducting solid-liquid separation of the extract;

a second housing container that is configured to be capable of housing a solid fraction obtained through the solid-liquid separation of the extract;

a fourth measurement device that is configured to be capable of measuring a state of the solid fraction in the second housing container over time; and

a fourth control device that is configured to be capable of controlling the solid-liquid separation of the extract by the solid-liquid separation device based on the state of the solid fraction obtained in the measurement.

(Supplementary Note 102)

The processing apparatus according to Supplementary Note 101, wherein the state of the solid fraction in the second housing container is an amount of a liquid contained in the solid fraction, an amount of a solid contained in the solid fraction, and/or a ratio between the liquid amount and the solid amount.

(Supplementary Note 103)

The processing apparatus according to Supplementary Note 101 or 102, wherein the fourth control device is capable of controlling the solid-liquid separation of the extract by the solid-liquid separation device based on a change in the state of the solid fraction over time.

(Supplementary Note 104)

The processing apparatus according to any one of Supplementary Notes 101 to 103, wherein the solid-liquid separation device includes a screw press, a screw conveyor, a roller press, a belt screen, and/or a cyclone.

(Supplementary Note 105)

The processing apparatus according to Supplementary Note 104, wherein the fourth control device is capable of controlling the solid-liquid separation of the extract by the solid-liquid separation device by controlling a rotation rate of the screw press, the screw conveyor, the roller press, the belt screen, and/or the cyclone.

(Supplementary Note 106)

A biological tissue processing apparatus including:

a first housing container that is capable of housing a mixed solution obtained by mixing a biological tissue and a solution containing an enzyme and is configured to be capable of decomposing the extracellular matrix of the biological tissue in the mixed solution with the enzyme;
a separation device that is configured to be capable of applying a force to the biological tissue in the mixed solution and separating cells of the biological tissue and/or an extract of the biological tissue from the extracellular matrix;
a solid-liquid separation device that is configured to be capable of conducting solid-liquid separation of the extract;
a second housing container that is configured to be capable of housing a solid fraction obtained through the solid-liquid separation of the extract;
a channel that is configured to allow the extract to be circulated therethrough via the solid-liquid separation device;
a liquid feeding device that is configured to be capable of feeding the extract via the channel;
a fourth measurement device that is configured to be capable of measuring a state of the solid fraction in the second housing container over time; and
a fifth control device that is configured to be capable of controlling supply of the extract to the solid-liquid separation device via the channel based on the state of the solid fraction obtained in the measurement.

(Supplementary Note 107)

The processing apparatus according to Supplementary Note 106, wherein the state of the solid fraction in the second housing container is an amount of a liquid contained in the solid fraction, an amount of a solid contained in the solid fraction, and/or a ratio between the liquid amount and the solid amount.

(Supplementary Note 108)

The processing apparatus according to Supplementary Note 106 or 107, wherein the fifth control device is capable of controlling the supply of the extract to the solid-liquid separation device via the channel based on a change in the state of the solid fraction over time.

(Supplementary Note 109)

The processing apparatus according to any one of Supplementary Notes 106 to 108, wherein the fifth control device is capable of controlling the supply of the extract to the solid-liquid separation device by controlling a liquid feeding amount (ejection amount), rotation rate, and/or rotational torque of the liquid feeding device.

(Supplementary Note 110)

The processing apparatus according to any one of Supplementary Notes 32 to 109, wherein the enzyme is a protease, a cellulase, a hemicellulase, and/or a pectinase.

(Supplementary Note 111)

The processing apparatus according to any one of Supplementary Notes 32 to 110, wherein the biological tissue is a plant tissue.

(Supplementary Note 112)

The processing apparatus according to Supplementary Note 111, wherein the plant is a tea plant (tea), a coffee plant (coffee), a moringa, a Brussels sprout, a cabbage, a tomato, and/or a bamboo.

(Supplementary Note 113)

The processing apparatus according to Supplementary Note 111 or 112, wherein the plant tissue is a leaf, a stem, a flower, a seed, a root, a trunk, a skin (e.g., a bark, an epidermis, and a bast), sap, and/or a fruit, or a processed product thereof.

(Supplementary Note 114)

The processing apparatus according to any one of Supplementary Notes 111 to 113, wherein the plant tissue is a tissue of a tea leaf or a used tea leaf.

(Supplementary Note 115)

A method for processing a biological tissue, including:

a decomposition step of decomposing the extracellular matrix of a biological tissue in a mixed solution containing the biological tissue and an enzyme;
a measurement step of measuring a state of the mixed solution containing the biological tissue and the enzyme over time in the decomposition step; and
a separation step of separating cells of the biological tissue and/or an extract of the biological tissue from a decomposition product of the biological tissue,
wherein the separation of the decomposition product of the biological tissue is conducted based on the state of the mixed solution obtained in the measurement step.

(Supplementary Note 116)

The processing method according to Supplementary Note 115, wherein the separation of the decomposition product of the biological tissue is conducted based on a change in the state of the mixed solution over time obtained in the measurement step.

(Supplementary Note 117)

The processing method according to Supplementary Note 115 or 116, wherein the separation of the decomposition product of the biological tissue is conducted when a rate of change in a measurement value of the state of the mixed solution over time obtained in the measurement step satisfies a predetermined condition.

(Supplementary Note 118)

The processing method according to Supplementary Note 115 or 116, wherein the separation of the decomposition product of the biological tissue is conducted when a derivative value of a measurement value of the state of the mixed solution over time obtained in the measurement step satisfies a predetermined condition.

(Supplementary Note 119)

The processing method according to any one of Supplementary Notes 115 to 118, wherein the state of the mixed solution is a viscosity, pH, temperature, turbidity, color, and/or air bubble density of the mixed solution.

(Supplementary Note 120)

The processing method according to any one of Supplementary Notes 115 to 119, wherein the enzyme is a protease, a cellulase, a hemicellulase, and/or a pectinase.

(Supplementary Note 121)

The processing method according to any one of Supplementary Notes 115 to 120, wherein the biological tissue is a plant tissue.

(Supplementary Note 122)

The processing method according to Supplementary Note 121, wherein the plant is a tea plant (tea), a coffee plant (coffee), a moringa, a Brussels sprout, a cabbage, a tomato, and/or a bamboo.

(Supplementary Note 123)

The processing method according to Supplementary Note 121 or 122, wherein the plant tissue is a leaf, a stem, a flower, a seed, a root, a trunk, a skin (e.g., a bark, an epidermis, and a bast), sap, and/or a fruit, or a processed product thereof.

(Supplementary Note 124)

The processing method according to any one of Supplementary Notes 121 to 123, wherein the plant tissue is a tissue of a tea leaf or a used tea leaf.

Industrial Applicability

As described above, with the present disclosure, it is possible to provide a method for producing an extract of a biological tissue and an apparatus for processing a biological tissue, with improved extraction efficiency. Accordingly, the present disclosure is very useful in the fields of, for example, raw materials, materials, and the like.

List of Reference Numerals

1, 100 to 104: Biological tissue processing apparatus
2: Housing container
3: Separation device
4: Agitation device
4c: Agitation blade
e: Leading edge of agitation blade 4c
ef: Outer edge of agitation blade 4c
4T: Separation tank
6: Filtration member
7: Component collection unit
8: Solid-liquid separation device
10: Control unit
11: Biological tissue supply unit
12: Electrolytic solution supply device
12a: Electrolyzed water production device
12b: Reserve tank
13: Enzyme addition unit
20: Supply unit
21: Supply channel
21a: Processing container-side supply channel
21b: Separation tank-side supply channel
21d: Homogenization device-side supply channel
25: Supply-side switching mechanism
30: Return unit
31: Return channel
31a: Processing container-side return channel
31b: Separation tank-side return channel
31d: Homogenization device-side return channel
32: First return channel
33: Second return channel
35: Return-side switching mechanism
40: Homogenization device

L: Mixed solution
L1: Solution
T: Biological tissue
T1: Non-separate-type biological tissue
M: Extracellular matrix
C: Cell
E: Extract
50: First housing container
51: Separation device
51a: Agitation blade
51b: Motor
52: First measurement device
53: First control device
54: Second measurement device
55: Second control device
56: Channel
57: Liquid feeding device
57a: Pump
57b: Motor
58: Third measurement device
59: Third control device
60: Solid-liquid separation device
60a: Screw press
60b: Motor
61: Second housing container
62: Fourth measurement device
63: Fourth control device
64: Fifth control device

**Claims**

1. A method for producing an extract of a biological tissue, comprising:

   a step of decomposing extracellular matrix of the biological tissue with an enzyme; and
   a step of separating cells of the biological tissue and/or an extract of the biological tissue from the extracellular matrix by subjecting the biological tissue to separation.

2. The production method according to claim 1, wherein the separation includes a processing operation for mechanically shearing the extracellular matrix of the biological tissue.

3. The production method according to claim 1 or 2, wherein the separation includes a processing operation for mechanically shearing the extracellular matrix of the biological tissue by bringing the biological tissue into contact with an agitation blade having a shearing blade.

4. The production method according to any one of claims 1 to 3, wherein the extracellular matrix includes a cell wall, collagen, and/or proteoglycan.

5. The production method according to any one of claims 1 to 4, wherein the extract includes a cellular component of the biological tissue.

6. The production method according to any one of claims 1 to 5, wherein the cellular component is a low-molecular-weight compound, a nucleic acid, a peptide, a protein, and/or extracellular matrix.

7. The production method according to any one of claims 1 to 6, wherein the extract is a liquid or a solid.

8. The production method according to any one of claims 1 to 7, wherein the separation includes a processing operation for separating a liquid and a solid of the biological tissue from each other.

9. The production method according to any one of claims 1 to 8, wherein at least portions of the decomposition and the separation are simultaneously conducted.

10. The production method according to any one of claims 1 to 9, wherein the decomposition and/or the separation is conducted a plurality of times.

11. The production method according to any one of claims 1 to 10, wherein the decomposition includes a processing operation for decomposing the extracellular matrix of the biological tissue with a microbial culture solution containing the enzyme.

12. The production method according to any one of claims 1 to 11, wherein the enzyme is a protease, a cellulase, a hemicellulase, and/or a pectinase.

13. The production method according to any one of claims 1 to 12, wherein the biological tissue is a plant tissue.

14. The production method according to claim 13, wherein the plant is a tea plant (tea), a coffee plant (coffee), a moringa, a Brussels sprout, and/or a cabbage.

15. The production method according to claim 13 or 14, wherein the plant tissue is a leaf, a stem, a flower, a seed, a root, a trunk, a skin (e.g., a bark, an epidermis, and a bast), sap, and/or a fruit, or a processed product thereof.

16. The production method according to any one of claims 13 to 15, wherein the plant tissue is a tissue of a tea leaf or a used tea leaf.

17. An extract of a biological tissue produced using the production method according to any one of claims 1 to 16.

18. The extract according to claim 17, comprising a cellulose fiber.

19. A food composition comprising the extract according to claim 17 or 18.

20. A method for extracting an extract of a biological tissue, comprising:

a step of decomposing extracellular matrix of the biological tissue with an enzyme; and
a step of separating cells of the biological tissue and/or an extract of the biological tissue from the extracellular matrix by subjecting the biological tissue to separation.

21. A biological tissue processing apparatus for processing a mixed solution obtained by mixing a biological tissue and a solution containing an enzyme, comprising:

a housing container in which the mixed solution is housed and decomposition is conducted for decomposing extracellular matrix of the biological tissue in the mixed solution with the enzyme; and
a separation device for separating cells of the biological tissue and/or an extract of the biological tissue from the extracellular matrix by applying a force to the biological tissue in the mixed solution.

22. The biological tissue processing apparatus according to claim 21,

wherein the separation device includes an agitation blade that is disposed in the housing container and is configured to agitate the mixed solution in the housing container, and
the agitation blade includes a shearing blade that comes into contact with the biological tissue subjected to the decomposition and applies a shearing force to the extracellular matrix of the biological tissue and/or the cells of the biological tissue.

23. The biological tissue processing apparatus according to claim 21, further comprising a separation tank provided with the separation device,

wherein the separation tank includes:

a supply channel to which the mixed solution subjected to the decomposition is supplied from the housing container; and
a return channel through which the mixed solution subjected to the decomposition in the decomposition tank is returned to the housing container,

the separation device includes an agitation blade that is disposed in the separation tank and is configured to agitate the mixed solution in the separation tank, and
the agitation blade includes a shearing blade that comes into contact with the biological tissue subjected to the decomposition in the housing container and applies a shearing force to the extracellular matrix of the biological tissue and/or the cells of the biological tissue.

24. The cell processing apparatus according to claim 23, comprising a plurality of the housing containers,

wherein between the housing containers and the separation tank, provided are
a plurality of the supply channels through which the housing containers and the separation tank are in communication,
a supply-side switching mechanism for switching any of the plurality of the supply channels to a channel for supplying the mixed solution to the separation tank,
a plurality of the return channels through which the housing containers and the separation tank are in communication, and
a return-side switching mechanism for switching any of the plurality of the return channels to a channel for returning the mixed solution from the separation tank.

25. The cell processing apparatus according to any one of claims 22 to 24, further comprising:

a driving source for driving the agitation blade;
a sensor for detecting a state of the mixed solution in the housing container and/or a state of the mixed solution in the separation tank; and
a control unit for controlling an operation of the driving source based on a signal from the sensor.

26. The biological tissue processing apparatus according to claim 21, further including a circulation channel that is disposed outside the housing container and through which the mixed solution subjected to the decomposition in the housing container is circulated,

wherein the separation device is provided at an intermediate portion of the circulation channel, and
the separation device includes a shearing blade that comes into contact with the biological tissue flowing in the circulation channel and applies a shearing force to the extracellular matrix of the biological tissue and/or the cells of the biological tissue.

27. The biological tissue processing apparatus according to claim 26, comprising a plurality of the housing containers, wherein the circulation channel includes:

a plurality of supply channels through which the housing containers and the separation tank are in communication,
a supply-side switching mechanism for switching any of the plurality of supply channels to a channel for supplying the mixed solution to the separation device,
a plurality of return channels through which separation device and the housing containers are in communication, and
a return-side switching mechanism for switching any of the plurality of return channels to a channel that is in communication with the separation device.

28. The biological tissue processing apparatus according to claim 26 or 27,

wherein the circulation channel includes:

first and second return channels for returning the mixed solution passing through the separation device to the housing container; and
a switching mechanism that is provided between the separation device and the first and second return channels and is configured to switch a channel for supplying the mixed solution from the separation device between the first and second return channels,

the first return channel includes an enzyme addition unit for adding an enzyme to the mixed solution, and the second return channel includes a solid-liquid separation device for separating a solid from the mixed solution and/or a component collection portion for collecting a component contained in the cells of the biological tissue from the mixed solution.

29. The biological tissue processing apparatus according to any one of claims 21 to 28, further comprising:

an enzyme supply unit for supplying a solution containing an enzyme to a solution in the housing container; and an electrolyzed water supply unit for supplying electrolyzed water to the solution in the housing container, wherein the electrolyzed water supply unit includes:

an electrolyzed water production device for generating the electrolyzed water; and an adjusting unit to which the electrolyzed water is supplied from the electrolyzed water production device and that is configured to adjust a state of the electrolyzed water.

30. The biological tissue processing apparatus according to any one of claims 21 to 29, further comprising an adsorption portion for adsorbing a component contained in the cells of the biological tissue contained in the solution in the housing container.

31. The biological tissue processing apparatus according to any one of claims 21 to 30, further comprising a biological tissue supply unit for supplying the biological tissue to the solution in the housing container.

32. A biological tissue processing apparatus comprising:

a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a first measurement device that is configured to be capable of measuring a state of the mixed solution over time; and a first control device that is configured to be capable of controlling separation of a decomposition product of the biological tissue by the separation device based on the state of the mixed solution obtained in the measurement.

33. A biological tissue processing apparatus comprising:

a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a second measurement device that is configured to be capable of measuring a state of the separation device over time; and a second control device that is configured to be capable of controlling separation of a decomposition product of the biological tissue by the separation device based on the state of the separation device obtained in the measurement.

34. A biological tissue processing apparatus comprising:

a first housing container that is capable of housing a mixed solution obtained by mixing a biological tissue and a solution containing an enzyme and is configured to be capable of decomposing extracellular matrix of the biological tissue in the mixed solution with the enzyme; a separation device that is configured to be capable of applying a force to the biological tissue in the mixed solution and separating cells of the biological tissue and/or an extract of the biological tissue from the extracellular matrix; a channel that is configured to allow the mixed solution in the first housing container to be circulated therethrough; a liquid feeding device that is configured to be capable of feeding the mixed solution in the first housing container via the channel;

a third measurement device that is configured to be capable of measuring a state of the mixed solution in the channel over time; and

a third control device that is configured to be capable of controlling liquid feeding by the liquid feeding device based on the state of the mixed solution in the channel obtained in the measurement.

35. A biological tissue processing apparatus comprising:

a first housing container that is capable of housing a mixed solution obtained by mixing a biological tissue and a solution containing an enzyme and is configured to be capable of decomposing extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a solid-liquid separation device that is configured to be capable of conducting solid-liquid separation of the extract;

a second housing container that is configured to be capable of housing a solid fraction obtained through the solid-liquid separation of the extract;

a fourth measurement device that is configured to be capable of measuring a state of the solid fraction in the second housing container over time; and

a fourth control device that is configured to be capable of controlling the solid-liquid separation of the extract by the solid-liquid separation device based on the state of the solid fraction obtained in the measurement.

36. A biological tissue processing apparatus comprising:

a first housing container that is capable of housing a mixed solution obtained by mixing a biological tissue and a solution containing an enzyme and is configured to be capable of decomposing extracellular matrix of the biological tissue in the mixed solution with the enzyme;

a separation device that is configured to be capable of applying a force to the biological tissue in the mixed solution and separating cells of the biological tissue and/or an extract of the biological tissue from the extracellular matrix;

a solid-liquid separation device that is configured to be capable of conducting solid-liquid separation of the extract;

a second housing container that is configured to be capable of housing a solid fraction obtained through the solid-liquid separation of the extract;

a channel that is configured to allow the extract to be circulated therethrough via the solid-liquid separation device;

a liquid feeding device that is configured to be capable of feeding the extract via the channel;

a fourth measurement device that is configured to be capable of measuring a state of the solid fraction in the second housing container over time; and

a fifth control device that is configured to be capable of controlling supply of the extract to the solid-liquid separation device via the channel based on the state of the solid fraction obtained in the measurement.

37. A method for processing a biological tissue, comprising:

a decomposition step of decomposing extracellular matrix of a biological tissue in a mixed solution containing the biological tissue and an enzyme;

a measurement step of measuring a state of the mixed solution containing the biological tissue and the enzyme over time in the decomposition step; and

a separation step of separating cells of the biological tissue and/or an extract of the biological tissue from a decomposition product of the biological tissue,

wherein the separation of the decomposition product of the biological tissue is conducted based on the state of the mixed solution obtained in the measurement step.

FIG. 1A

FIG. 1B

FIG. 2

FIG. 3

FIG. 4A

FIG. 4B

EP 4 663 767 A1

FIG. 5A

FIG. 5B

81

FIG. 6A

FIG. 6B

FIG. 7

FIG. 8

FIG. 9

FIG. 10

FIG. 11

|  | 100 times | 200 times | 500 times |
|---|---|---|---|
| Tea-leaf slurry of Example 1 | | | |
| Used-tea-leaf slurry of Example 2 | | | |
| Tea leaves of Comparative Example 1 | | | |

FIG. 12

|  | 100 times | 200 times | 500 times |
|---|---|---|---|
| Venous cellulose fibers contained in tea-leaf slurry of Example 1 | | | |

FIG. 13

FIG. 14A          FIG. 14B

FIG. 15A          FIG. 15B          FIG. 15C

FIG. 15D          FIG. 15E          FIG. 15F

FIG. 16

FIG. 17

FIG. 18

FIG. 19

FIG. 20

FIG. 21A

FIG. 21B

FIG. 21C

FIG. 21D

FIG. 21E

FIG. 21F

FIG. 22

FIG. 23

Protein amount (per 25 g of tea leaves): 1.62 g
Protein content rate (per 42 g of tea leaves (wet)): 3.86%
Protein content rate (per 6.5 g of tea leaves (dry)): 24.92%

FIG. 24

FIG. 25

FIG. 26

FIG. 27A

FIG. 27B

FIG. 27C

FIG. 27D

FIG. 28

Total collection amount (wet): 117 g
Total collection amount (dry): 36 g

FIG. 29

Protein amount (per 36 g of coffee beans): 4.04 g
Protein content rate (per 117 g of coffee beans (wet)): 3.45%
Protein content rate (per 36 g of coffee beans (dry)): 11.22%

FIG. 30

FIG. 31A

FIG. 31B

FIG. 32A    FIG. 32B    FIG. 32C

FIG. 32D    FIG. 32E    FIG. 32F

Total collection amount (wet): 281 g
Total collection amount (dry): 25 g

FIG. 33

Protein amount (per 25 g of strawberry leaves): 1.85 g
Protein content rate (per 281 g of strawberry leaves (wet)): 0.66%
Protein content rate (per 25 g of strawberry leaves (dry)): 7.40%

FIG. 34

FIG. 35A

FIG. 35B

FIG. 36A  FIG. 36B  FIG. 36C

FIG. 36D  FIG. 36E  FIG. 36F

Total collection amount (wet): 247 g
Total collection amount (dry): 25 g

FIG. 37

FIG. 38

FIG. 39

START

DECOMPOSITION OF
BIOLOGICAL TISSUE ~ S1

MEASUREMENT OF STATE
OF MIXED SOLUTION ~ S2

DOES MEASUREMENT VALUE SATISFY
PREDETERMINED CONDITION? ~ S3   No

Yes

SEPARATION OF
BIOLOGICAL TISSUE ~ S4

END

FIG. 40

101

54    55

51

51b

51a

50

FIG. 41

START

↓ S5

DECOMPOSITION OF
BIOLOGICAL TISSUE

↓ S6

SEPARATION OF
BIOLOGICAL TISSUE

↓ S7

MEASUREMENT OF STATE
OF SEPARATION DEVICE

↓ S8

DOES MEASUREMENT VALUE SATISFY
PREDETERMINED CONDITION? — No

Yes
↓ S9

END OF SEPARATION OF
BIOLOGICAL TISSUE

END

FIG. 42

102

59

51

51b

51a

57

57b

57a

56

50

58

FIG. 43

START

S10
DECOMPOSITION OF
BIOLOGICAL TISSUE

S11
SEPARATION OF
BIOLOGICAL TISSUE

S12
MEASUREMENT OF STATE
OF MIXED SOLUTION IN CHANNEL

S13
DOES MEASUREMENT VALUE SATISFY
PREDETERMINED CONDITION? — No

Yes

S14
END OF LIQUID FEEDING BY
LIQUID FEEDING DEVICE

END

FIG. 44

FIG. 45

START

S15
DECOMPOSITION OF
BIOLOGICAL TISSUE

S16
SEPARATION OF
BIOLOGICAL TISSUE

S17
SOLID-LIQUID SEPARATION
OF EXTRACT

S18
MEASUREMENT OF STATE
OF SOLID FRACTION

S19
DOES MEASUREMENT VALUE SATISFY
PREDETERMINED CONDITION?

Yes

No
S20
CHANGE OF ROTATION RATE OF
SOLID-LIQUID SEPARATION DEVICE

END

FIG. 46

FIG. 47

FIG. 48

## INTERNATIONAL SEARCH REPORT

| International application No. |
|---|
| **PCT/JP2024/004689** |

**A. CLASSIFICATION OF SUBJECT MATTER**

*C12P 21/06*(2006.01)i; *A23F 3/16*(2006.01)i; *A23F 5/24*(2006.01)i; *A23J 1/00*(2006.01)i; *A23J 3/14*(2006.01)i; *A23L 5/00*(2016.01)i; *A23L 19/00*(2016.01)i; *C07K 1/34*(2006.01)i; *C12M 1/00*(2006.01)i; *C12P 1/00*(2006.01)i
FI:  C12P21/06; A23L5/00 K; A23J3/14; A23F3/16; A23F5/24; A23L5/00 J; A23L19/00 Z; A23J1/00 A; C07K1/34; C12M1/00 H; A23L5/00 M; C12P1/00 A

According to International Patent Classification (IPC) or to both national classification and IPC

**B. FIELDS SEARCHED**

Minimum documentation searched (classification system followed by classification symbols)

C12P21/06; A23F3/16; A23F5/24; A23J1/00; A23J3/14; A23L5/00; A23L19/00; C07K1/34; C12M1/00; C12P1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); MEDLINE (JDreamIII)

**C. DOCUMENTS CONSIDERED TO BE RELEVANT**

| Category* | Citation of document, with indication, where appropriate, of the relevant passages | Relevant to claim No. |
|---|---|---|
| P, X | JP 2023-110255 A (JRS CO., LTD.) 09 August 2023 (2023-08-09) claims 1-3 | 1-37 |
| X | JP 2015-100276 A (MITSUI NORIN CO., LTD.) 04 June 2015 (2015-06-04) claims 1-14, examples 7-10, paragraphs [0033]-[0040], [0042] | 1, 4-10, 12-21, 29-31 |
| Y | | 1-37 |
| X | JP 2006-014615 A (JAPAN TOBACCO INC.) 19 January 2006 (2006-01-19) claims 1-11, examples 2-3, 5-6, 8-9, paragraphs [0017]-[0018], [0042]-[0043], [0053]-[0055], [0058], table 1 | 1, 4-8, 11-21, 29, 31 |
| Y | | 1-37 |
| X | WO 2012/046349 A1 (T. HASEGAWA CO., LTD.) 12 April 2012 (2012-04-12) claims 1-6, examples 1-6, p. 4, lines 14-27, p. 10, line 22 to p. 11, line 6 | 1, 4-8, 12-21, 29, 31 |
| Y | | 1-37 |

✓ Further documents are listed in the continuation of Box C.      ✓ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **15 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

International application No.

**PCT/JP2024/004689**

### C. DOCUMENTS CONSIDERED TO BE RELEVANT

| Category* | Citation of document, with indication, where appropriate, of the relevant passages | Relevant to claim No. |
|---|---|---|
| X | JP 2007-217466 A (UCC UESHIMA COFFEE CO., LTD.) 30 August 2007 (2007-08-30) claims 1-5, examples 1-3, paragraphs [0030]-[0033], [0041]-[0045], [0049]-[0050], [0056], [0063], [0069], tables 1-3 | 1, 4-15, 17-21, 29-31, 37 |
| Y | | 1-37 |
| X | JP 2011-050271 A (MITSUBISHI-KAGAKU FOODS CORP.) 17 March 2011 (2011-03-17) claims 1-6, examples 1-4, paragraphs [0017], [0025]-[0043], [0048], [0054], [0061]-[0062] | 1, 4-9, 11-21, 29, 31 |
| Y | | 1-37 |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2023-110255 | A | 09 August 2023 | (Family: none) | | | |
| JP | 2015-100276 | A | 04 June 2015 | (Family: none) | | | |
| JP | 2006-014615 | A | 19 January 2006 | (Family: none) | | | |
| WO | 2012/046349 | A1 | 12 April 2012 | CN claims 1-6, examples 1-6, paragraphs [0025], [0040]-[0041] | 102573507 | A | |
| | | | | TW | 201215328 | A | |
| | | | | HK | 1168998 | A | |
| JP | 2007-217466 | A | 30 August 2007 | US claims 1-16, examples 1-3, paragraphs [0046]-[0069], tables 1-3 | 2010/0227366 | A1 | |
| | | | | WO | 2007/094360 | A1 | |
| | | | | EP | 1985635 | A1 | |
| JP | 2011-050271 | A | 17 March 2011 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5645994 B
- JP 5646035 B
- JP 2023019517 A
- JP 2023152830 A